# EUROPEAN PATENT APPLICATION

(11) **EP 1 166 781 A1**
(43) Date of publication of application: **02.01.2002**
(21) Application number: 00900834.3
(22) Date of filing: 20.01.2000
(51) Int. Cl.: A61K 31/215, A61K 31/27, A61K 31/337, A61K 31/497, A61K 31/501, A61K 31/506, C07C 271/22, C07C 237/10, C07C 327/22, C07C 327/28, C07D 305/12, C07D 405/12, A61P 43/00, A61P 35/00

(54) **PROTEASOME INHIBITORS**

(30) Priority: 20.01.1999 JP 1239199; 08.10.1999 JP 28853999
(71) Applicant: KYOWA HAKKO KOGYO CO., LTD., Chiyoda-ku, Tokyo 100-8185 (JP)
(72) Inventor: YAMAGUCHI, Hiroyuki, Kyowa Hakko Kogyo Co.,Ltd., Machida-shi, Tokyo 194-8533 (JP); ASAI, Akira, Kyowa Hakko Kogyo Co.,Ltd., Machida-shi, Tokyo 194-8533 (JP); MIZUKAMI, Tamio, Kyowa Hakko Kogyo Co., Ltd, Tokyo 100-8185 (JP); YAMASHITA, Yoshinori, Kyowa Hakko Kogyo Co.,Ltd., Mashida-shi, Tokyo 194-8533 (JP); AKINAGA, Shiro, Kyowa Hakko Kogyo Co.,Ltd., Sunto-gun, Shizuoka 411-8731 (JP); IKEDA, Shun-Ichi, Kyowa Hakko Kogyo Co.,Ltd., Sakai-shi, Osaka 590-0003 (JP); KANDA, Yutaka, Kyowa Hakko Kogyo Co., Ltd., Sunto-gun, Shizuoka 411-8731 (JP)
(74) Representative: VOSSIUS & PARTNER
(86) International application number: JP0000247
(87) International publication number: WO0043000

(57) **Abstract**

The present invention relates to proteasome inhibitors as the active ingredient carboxylic acid derivatives represented by general formula (I) or pharmaceutically acceptable salts thereof: 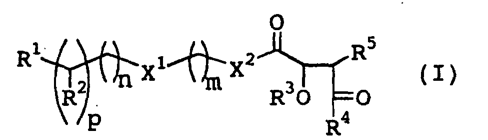 <wherein m and n are the same or different and represent an integer of 0 to 10; p is 0 or 1; R¹ represents a hydrogen atom, substituted or unsubstituted alkyl, substituted or unsubstituted alicyclic alkyl, or the like; and R² represents a hydrogen atom, COR¹³, or CH₂OR^{3a}, or R¹ and R² together represent the formula: 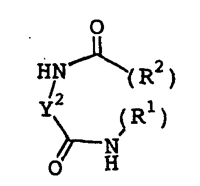 X¹ represents a bond, substituted or unsubstituted alkylene, substituted or unsubstituted cycloalkylene, or the like; X² represents an oxygen atom, a sulfur atom, or NR¹⁷; R³ is has the same significance as the above R^{3a}; R⁴ represents hydroxy, mercapto, substituted or unsubstituted alkoxy, or the like, or R¹ and R² together represent a bond; R⁵ represents a hydrogen atom, substituted or unsubstituted alkyl, substituted or unsubstituted alkenyl, or the like.

## Description

### Technical Field

The present invention relates to a proteasome inhibitor containing a carboxylic acid derivative as an active ingredient, which is useful for the treatment of malignant tumors such as leukemia, lung cancer, colon cancer, breast cancer or the like, for the treatment of diseases associated with autoimmune diseases, inflammation, neurodegeneration or the like such as rheumatoid chronic arthritis, asthma, Alzheimer disease or the like, or further for the reduction of rejection in organ transplantation.

### Background Art

Proteasome also called multicatalytic protease is a multicomponent complex. Its structure consists of 20S proteasome, which has cylindrical catalytic subunits and oxbow regulatory subunits. 26S proteasome in which both are associated is a macromolecular complex with a molecular weight of 200k having a dumbbell shaped structure, and plays a role in decomposing intracellularly ubiquitinated proteins depending on ATP [Tanaka K. et al., Molecular Biology Report, 24, 3-11 (1997); Baumeister W. et al., Cell, 92, 367-380 (1998)]. It has been previously reported that its expression levels are increased in leukemia cells. However in recent years, it has been demonstrated that this degradation mechanism by ubiquitin-proteasome is deeply involved in control of cell cycle and control of immune initiation. In many cancer cells, acceleration of degradation of a tumor suppressor gene product, p53 occurs regardless of its variant or wild type, and p53 does not function normally. Induction of the wild type of p53 causes G1 arrest or apoptosis of cells. The system bearing degradation of this wild type p53 is the ubiquitin-proteasome degradative system [Spataro V. et al., British J. Cancer, 77, 448-455 (1998)]. Recently, from the analysis using clinical specimens of breast cancer, colon cancer and lung cancer, it has been shown that proteasome-dependent degradation of an endogenous inhibitor of cyclin-dependent kinase, p27 is facilitated in these cancers, particularly in those with poor prognosis [Porter P. L. et al., Nature Medicine, 3, 222-225 (1997); Catzavelos C. et al., Nature Medicine, 3, 227-230 (1997); Esposito V. et al., Cancer Res., 57, 3381-3385 (1997)]. Thus, inhibition of proteasome can accumulate p53 or p27, degradation of which is facilitated in cancer cells, and thereby can induce growth arrest or apoptosis of cancer cells. Further, proteasome has been reported to function as a processing enzyme for endogenous antigens in immune initiation system [Rock K. L. et al., Cell, 78, 761-771 (1994)]. Additionally, it has been demonstrated that proteasome plays an important role in activation of NF-κB, which is a transcription regulatory factor for inflammatory cytokines such as TNF-α [Palombella, V. J., Cell, 78, 773-785 (1994)]. Thus, it is believed that a proteasome inhibitor is also useful for the reduction of rejection in organ transplantation, or for inflammatory diseases or the like.

Lactacystin produced by *Actinomycetes* [Fenteany G., J. Biol. Chem., 273, 8545-8548 (1998)] and a peptide aldehyde compound, MG132 [Palombella V. J., Cell, 78, 773-785 (1994)] have been reported as existing selective proteasome inhibitors. These inhibitors not only have an action of induction of growth arrest or apoptosis of cancer cells and that of inhibition of NF-KB activation but also are known as the compounds, which induce prong elongation in nerve cells [Omura S. et al., J. Antibiot., 40, 113-116 (1991)]. Thus, it is believed that a proteasome inhibitor is effective for diseases associated with neurodegeneration or the like.

On the other hand, UCK14 compounds are the compounds produced by a microorganism belonging to the genus *Streptomyces* and are disclosed in Japanese Published Unexamined Application No. 169796/97, which exhibit an antitumor action. Among them, UCK14A₁, UCK14A₂ and UCK14C have the following structures, but none of them are known to have an inhibitory activity for proteasome.

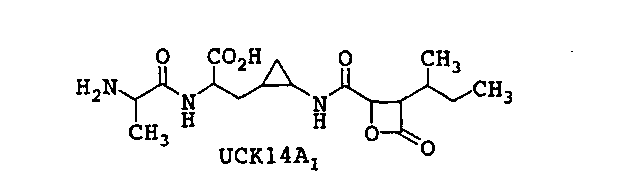

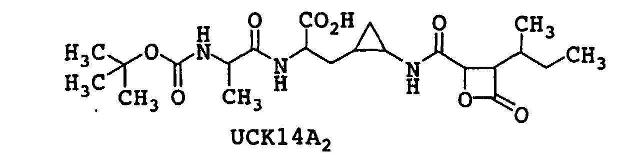

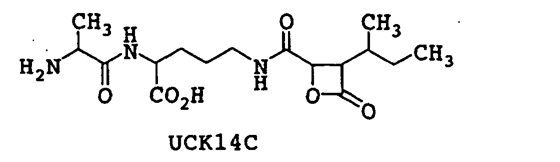

Besides, the following compounds (a) through (e) [Shih, Hsiencheng et al., Synthesis, 866-867 (1989); Georgy Liesen et al., J. Org. Chem., 52, 455-457 (1987); David J. Hart et al., J. Org. Chem., 50, 235-342 (1985); Japanese Published Unexamined Application No. 21661/92] are known, but none of them are known to have an inhibitory activity for proteasome.

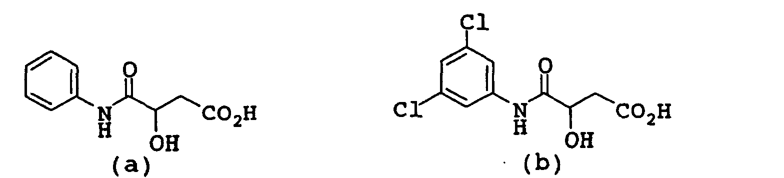

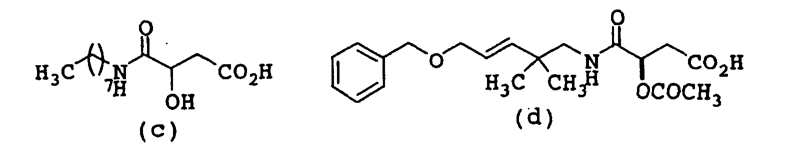

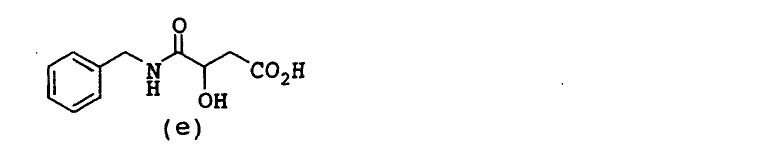

On the other hand, the following compound having a β-lactone structure has been reported [S. Cammas et al., Polymer Bulletin, 33, 149-158 (1994)], but its pharmacological activity is not known.

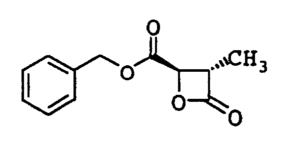

### Disclosure of the Invention

The object of the present invention is to provide a proteasome inhibitor. Also another object of the present invention is to provide a pharmaceutical composition comprising a substance having the above action as an active ingredient. Specifically, providing a pharmaceutical composition useful for the treatment of malignant tumors such as leukemia, lung cancer, colon cancer, breast cancer or the like, for the treatment of diseases associated with autoimmune diseases, inflammation, neurodegeneration or the like such as rheumatoid chronic arthritis, asthma, Alzheimer disease or the like, or further for the reduction of rejection in organ transplantation is the object of the present invention. Still another subject of the present invention is to provide a novel carboxylic acid derivative useful as an active ingredient of various medicines such as an antitumor agent or the like.

The inventors of the present invention have found an inhibitory activity for proteasome in UCK compounds and derivatives of UCK compounds. The present invention relates to a medical agent effective for the treatment of the diseases associated with malignant tumors, autoimmune diseases, inflammation, neurodegeneration or the like by inhibition of proteasome.

Thus, the present invention relates to proteasome inhibitors comprising, as an active ingredient, a carboxylic acid derivative represented by the formula (I) [hereinafter, the compound represented by the formula (I) is referred to as Compound (I). The other formula numbers are treated in a similar manner] or a pharmaceutically acceptable salt thereof: 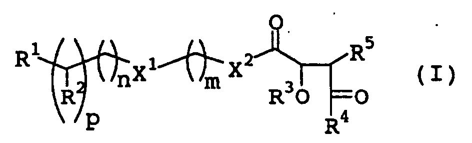 <wherein m and n are the same or different and represent an integer of 0 to 10; p represents an integer of 0 or 1; R¹ represents a hydrogen atom, substituted or unsubstituted alkyl, substituted or unsubstituted alicyclic alkyl, substituted or unsubstituted aralkyl, substituted or unsubstituted aryl, or NR⁶R⁷ {wherein R⁶ represents a hydrogen atom, substituted or unsubstituted alkyl, or substituted or unsubstituted aralkyl; and R⁷ represents a hydrogen atom, substituted or unsubstituted alkyl, substituted or unsubstituted aralkyl, CW¹R⁸ (wherein R⁸ represents a hydrogen atom, substituted or unsubstituted alkyl, substituted or unsubstituted alkylamino, substituted or unsubstituted alkoxy, substituted or unsubstituted aryl, a substituted or unsubstituted heterocyclic group, substituted or unsubstituted aralkyl, substituted or unsubstituted aralkylamino, or substituted or unsubstituted aralkyloxy; and W¹ represents an oxygen atom or a sulfur atom); or represents the formula: 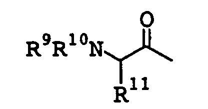 [wherein R⁹ represents a hydrogen atom, substituted or unsubstituted alkyl, or substituted or unsubstituted aralkyl; R¹⁰ represents a hydrogen atom, substituted or unsubstituted alkyl, substituted or unsubstituted aralkyl, CW²R^{8a} (wherein R^{8a} and W² have the same significances as the above R⁸ and W¹, respectively), substituted or unsubstituted alkylsulfonyl, substituted or unsubstituted arylsulfonyl, or PW³R¹²₂ (wherein R¹²'s are the same or different and represent substituted or unsubstituted alkyl, or substituted or unsubstituted aryl; and W³ has the same significance as the above W¹), or R⁹ and R¹⁰ together represent the formula: 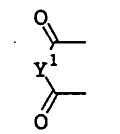 (wherein Y¹ represents substituted or unsubstituted alkylene or substituted or unsubstituted arylene); and R¹¹ represents a hydrogen atom, substituted or unsubstituted alkyl, or substituted or unsubstituted aralkyl]}; R² represents a hydrogen atom, COR¹³ [wherein R¹³ represents hydroxy, substituted or unsubstituted alkoxy, substituted or unsubstituted alkenyloxy, substituted or unsubstituted aralkyloxy, substituted or unsubstituted alicyclic alkylalkoxy, substituted or unsubstituted aroylalkoxy, or NR¹⁴R¹⁵ (wherein R¹⁴ represents a hydrogen atom, substituted or unsubstituted alkyl, or substituted or unsubstituted aryl; and R¹⁵ represents substituted or unsubstituted alkyl, substituted or unsubstituted aralkyl, substituted or unsubstituted alkoxycarbonylalkyl, amino, substituted or unsubstituted alkylamino, or substituted or unsubstituted arylamino, or R¹⁴ and R¹⁵ together with the adjacent N form a substituted or unsubstituted heterocyclic group)], or CH₂OR^{3a} [wherein R^{3a} represents a hydrogen atom, substituted or unsubstituted alkyl, substituted or unsubstituted aralkyl, substituted or unsubstituted alkanoyl, substituted or unsubstituted aroyl, or SiR¹⁶₃ (wherein R¹⁶'s are the same or different and represent substituted or unsubstituted alkyl, or substituted or unsubstituted aryl)], or R¹ and R² together represent the formula: 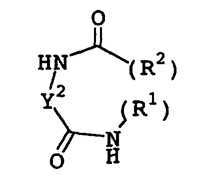 (wherein Y² represents substituted or unsubstituted alkylene); X¹ represents a bond, substituted or unsubstituted alkylene, substituted or unsubstituted alicyclic alkylene, substituted or unsubstituted alkenylene, or substituted or unsubstituted arylene; X² represents an oxygen atom, a sulfur atom or NR¹⁷ (wherein R¹⁷ represents a hydrogen atom, substituted or unsubstituted alkyl, or substituted or unsubstituted aralkyl); R³ has the same significance as the above R^{3a}; R⁴ represents hydroxy, mercapto, substituted or unsubstituted alkoxy, or substituted or unsubstituted alkylthio, or R³ and R⁴ together represent a bond; and R⁵ represents a hydrogen atom, substituted or unsubstituted alkyl, substituted or unsubstituted alkenyl, or substituted or unsubstituted aralkyl>.

Another embodiment includes proteasome inhibitors comprising a carboxylic acid derivative, which is Compound (I) wherein R³ and R⁴ together represent a bond, or a pharmaceutically acceptable salt thereof as an active ingredient.

In another respect, the present invention relates to carboxylic acid derivatives [hereinafter referred to as Compound (IA)], which are Compound (I) wherein R⁴ is hydroxy, or substituted or unsubstituted alkoxy; p is 1; and R¹ is a hydrogen atom or NR⁶R⁷ (wherein each of R⁶ and R⁷ has the same significance as defined above), or R¹ and R² together are the formula: 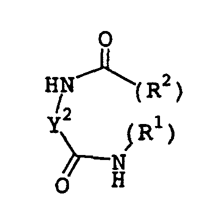 (wherein Y² has the same significance as defined above); X¹ is substituted or unsubstituted alicyclic alkylene, or substituted or unsubstituted arylene; and X² is NR¹⁷ (wherein R¹⁷ has the same significance as defined above), or pharmaceutically acceptable salts thereof, or relates to carboxylic acid derivatives [hereinafter referred to as Compound (IB)], which are Compound (I) wherein R⁴ is mercapto, or substituted or unsubstituted alkylthio, or R³ and R⁴ together are a bond; and X² is NR¹⁷ (wherein R¹⁷ has the same significance as defined above)[but, when m is 0; n and p are 1; R² is carboxy; R³ and R⁴ together are a bond; R⁵ is sec-butyl; and X¹ is cyclopropylene or ethylene, R¹ is neither NHC(=O)-C(CH₃)NH₂ nor NHC(=O)-C(CH₃)NHC(=O)O-C(CH₃)₃], or pharmaceutically acceptable salts thereof. Also another embodiment includes carboxylic acid derivatives, which are Compound (IA) wherein R¹ is a hydrogen atom or NR⁶R⁷ (wherein each of R⁶ and R⁷ has the same significance as defined above), or pharmaceutically acceptable salts thereof. In the above compounds, carboxylic acid derivatives or pharmaceutically acceptable salts thereof, wherein R¹ is NR⁶R⁷ (wherein each of R⁶ and R⁷ has the same significance as defined above); X¹ is cyclopropylene or alkylene; and X² is NH, are preferred. Also another embodiment includes carboxylic acid derivatives, which are Compound (IB) wherein R⁴ is mercapto, or substituted or unsubstituted alkylthio; R¹ is NR⁶R⁷ (wherein each of R⁶ and R⁷ has the same significance as defined above); and X¹ is cyclopropylene or alkylene, or pharmaceutically acceptable salts thereof. Further, another embodiment includes carboxylic acid derivatives, which are a compound wherein R³ and R⁴ together represent a bond in the formula (IB), or pharmaceutically acceptable salts thereof. In the above compounds, carboxylic acid derivatives or pharmaceutically acceptable salts thereof, wherein m is 0; n and p are 1; R¹ is NR⁶R⁷ (wherein each of R⁶ and R⁷ has the same significance as defined above); R² is COR^{13a} (wherein R^{13a} is alkylamino, aralkyloxy or aralkylamino); R⁵ is alkyl; X¹ is cyclopropylene, alkylene, or substituted or unsubstituted phenylene; and X² is NH, are preferred.

The above novel carboxylic acid derivatives or pharmaceutically acceptable salts thereof are useful, for example, as an active ingredient of pharmaceutical compositions or as an active ingredient for antitumor agents, antiinflammatory agents, proteasome inhibitors or the like.

Also, the present invention relates to a process for producing the above-mentioned carboxylic acid derivatives wherein R³ and R⁴ together represent a bond and X² is NR¹⁷, characterized in that a carboxylic acid represented by the formula (II): 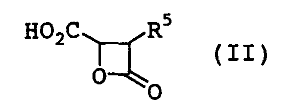 (wherein R⁵ has the same significance as defined above) is reacted with an amine represented by the formula (III): 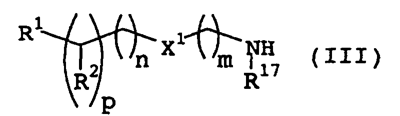 (wherein each of m, n, p, R¹, R², R¹⁷ and X¹ has the same significance as defined above). Further, the present invention relates to carboxylic acids [hereinafter referred to as Compound (IIa)] which are Compound (II) wherein R⁵ is substituted or unsubstituted alkyl, substituted or unsubstituted alkenyl, or substituted or unsubstituted aralkyl, or salts thereof. Also, the present invention relates to amines [hereinafter referred to as Compound (IIIa)] or salts thereof which are Compound (III) wherein m is 0; n and p are 1; R¹ is NR⁶R⁷ (wherein each of R⁶ and R⁷ has the same significance as defined above); R² is COR¹³ (wherein R¹³ has the same significance as defined above) or CH₂OR^{3a} (wherein R^{3a} has the same significance as defined above), or R¹ and R² together are the formula: 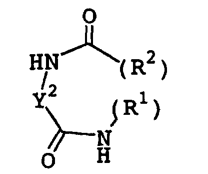 (wherein Y² has the same significance as defined above); and X¹ is cyclopropylene. Among Compound (IIIa), the amines [hereinafter referred to as Compound (IIIb)] or salts thereof, wherein R¹ is amino and R¹⁷ is a hydrogen atom are preferred, and amines [hereinafter referred to as Compound (IIIc)] or salts thereof, wherein R¹ is amino; R² is carboxy; and R¹⁷ is a hydrogen atom are more preferred. In another respect, the present invention relates to the compounds or salts thereof, wherein Compound (IIIa) through Compound (IIIc) are protected with protecting groups.

In another respect, the present invention relates to medicines comprising Compound (I) or a pharmaceutically acceptable salt thereof as an active ingredient, used for the treatment of the diseases curable by proteasome inhibition. These diseases may include, for example, malignant tumors such as leukemia, lung cancer, colon cancer, breast cancer or the like, diseases associated with autoimmune diseases, inflammation, neurodegeneration or the like such as rheumatoid chronic arthritis, asthma, Alzheimer disease and the like, and further the diseases such as the reduction of rejection in organ transplantation and the like.

The above-mentioned inhibitor or the above-mentioned medicine is preferably provided in the form of a pharmaceutical composition comprising Compound (I) or a pharmaceutically acceptable salt thereof and additives for the formulation. Also, the present invention relates to the use of Compound (I) or a pharmaceutically acceptable salt thereof for producing the above-mentioned proteasome inhibitor or the above-mentioned medicine, and to a method to inhibit proteasome comprising a process to administer an effective amount of Compound (I) or a pharmaceutically acceptable salt thereof to a mammal including human. Also, Compound (II) or salts thereof may be used as a useful intermediate in producing Compound (I) or a pharmaceutically acceptable salt thereof. Also, Compound (III) or salts thereof may be used as a useful intermediate in producing Compound (I) or a pharmaceutically acceptable salt thereof. Besides, Compound (IIIc) or salts thereof may be used as an active ingredient in medicines or agricultural chemicals, a synthetic starting material or synthetic intermediate of medicines or agricultural chemicals, a chemical seasoning, a food additive, a feed additive, an active ingredient of cosmetics, a building block in organic synthesis, an industrial material (for example, a starting material for producing a polymer), or the like, in addition to being used as a useful intermediate in producing Compound (I) or a pharmaceutically acceptable salt thereof.

In the definition of each group in the formula (I), formula (II) and formula (III), the alkyl moieties of the alkyl, alkoxy, alkylamino, alkanoyl, alkoxycarbonylalkyl, alkylthio and alkylsulfonyl include straight-chain or branched alkyl having 1 to 20 carbon atoms, such as methyl, ethyl, propyl, isopropyl, butyl, isobutyl, sec-butyl, tert-butyl, pentyl, isopentyl, hexyl, heptyl, octyl, nonyl, decyl, undecyl, dodecyl, pentadecyl, eicosyl, and preferably they have 1 to 8 carbon atoms. The alkylene moieties of the alkylene, alicyclic alkylalkoxy, aroylalkoxy and alkoxycarbonylalkyl are those in which one hydrogen atom is eliminated from the above alkyl moieties. The alicyclic alkyl moieties of the alicyclic alkyl and alicyclic alkylalkoxy include alicyclic alkyl having 3 to 8 carbon atoms, such as cyclopropyl, cyclobutyl, cyclopentyl, cyclohexyl, cycloheptyl, and cyclooctyl. The alicyclic alkylene is the one in which one hydrogen atom is eliminated from the above alicyclic alkyl. The alkenyl moieties of the alkenyl and alkenyloxy include, straight-chain or branched alkenyl having 2 to 6 carbon atoms and 1 to 3 double bonds, such as vinyl, allyl, crotyl, prenyl, butenyl, pentenyl, hexenyl, pentadienyl, and hexadienyl. The alkenylene is the one in which one hydrogen atom is eliminated from the above alkenyl.

The aryl moieties of the aryl, arylamino, arylsulfonyl, aroylalkoxy and aroyl include phenyl, naphthyl, anthryl and phenanthryl. The aralkyl moieties of the aralkyl, aralkyloxy and aralkylamino include those having 7 to 15 carbon atoms, such as benzyl, phenethyl, benzhydryl, naphthylmethyl, and anthrylmethyl. The arylene is the one in which one hydrogen atom is eliminated from the above aryl.

The heterocyclic group includes monocyclic or polycyclic 3- to 8-membered heterocyclic groups containing at least one or more heteroatoms such as oxygen, sulfur and nitrogen atoms. Preferably, 5- or 6-membered aromatic heterocyclic groups containing nitrogen such as imidazolyl, pyridyl, indolyl, quinolyl, isoquinolyl, quinoxalinyl, quinazolinyl, pyridazinyl, pyrimidinyl and pyrazinyl are included. And preferably, 5- or 6-membered alicyclic heterocyclic groups containing nitrogen such as pyrrolidinyl, oxopyrrolidinyl, piperidinyl, piperidino, piperazinyl, morpholino, thiomorpholino, homopiperidinyl, homopiperazinyl and tetrahydropyridinyl are included. Further, 1,3-dioxolan-4-yl, 1,3-dioxan-4-yl and 1,3-dioxan-5-yl are also suitable as the alicyclic heterocyclic group containing oxygen. The heterocyclic group formed together with adjacent N can be any of the above heterocyclic groups as long as they contain N.

The substituents of the substituted alkylamino, substituted alkylsulfonyl, substituted alkanoyl, substituted alkoxy, substituted alkylene, substituted aralkyl, substituted aralkylamino, substituted alicyclic alkyl, substituted alicyclic alkylalkoxy, substituted alicyclic alkylene, substituted aralkyloxy, substituted alkenyl, substituted alkenyloxy, substituted alkenylene, substituted aryl, substituted arylamino, substituted aroyl, substituted aroylalkoxy, substituted alkylthio, substituted arylsulfonyl, substituted heterocyclic group formed together with adjacent N and substituted arylene, are the same or different, and include halogen, nitro, hydroxy, amino, carboxy, alkanoyl, alkanoyloxy, alkyl, alkoxy, aroyl, aralkyl, aroyloxy, aroylalkoxy, alkoxycarbonyl, alkenyloxycarbonyl, alkoxycarbonylamino, alkoxycarbonyloxy, dialkylcarbamoyloxy, aralkyloxy, alkoxyaralkyloxycarbonyl, aralkyloxycarbonyl, alicyclic alkylalkoxycarbonyl, OPO(OH)₂, OSO₃H, OSiR^{16a}₃ (wherein R^{16a}'s are the same or different and represent alkyl or aryl each of which has the same significance as defined above, respectively), and SiR^{16b}₃ (wherein R^{16b} has the same significance as the above R^{16a}). The substituents of the substituted heterocyclic group include, in addition to the above substituents of the substituted alkylamino and the like, CW⁴R^{8d} [wherein R^{8d} represents a hydrogen atom, substituted or unsubstituted alkyl, substituted or unsubstituted alkylamino, substituted or unsubstituted alkoxy, substituted or unsubstituted aryl, a heterocyclic group, substituted or unsubstituted aralkyl, substituted or unsubstituted aralkylamino, or substituted or unsubstituted aralkyloxy. Herein, each of the alkyl, alkylamino, alkoxy, aryl, heterocyclic group, aralkyl, aralkylamino and aralkyloxy has the same significance as defined above, respectively; the substituents of the substituted alkyl, substituted alkylamino, substituted alkoxy, substituted aryl, substituted aralkyl, substituted aralkylamino and substituted aralkyloxy have the same significance as defined for the above substituents of the substituted alkylamino and the like; and W ⁴ has the same significance as the above W¹]. The substituents of the substituted alkyl include substituted alkenyloxycarbonyl, substituted aralkyloxycarbonyl, substituted alicyclic alkylalkoxycarbonyl and substituted alkoxycarbonyl (herein, the alkenyl moiety of the alkenyloxycarbonyl has the same significance as the above alkenyl; the aralkyl moiety of the aralkyloxycarbonyl has the same significance as the above aralkyl; the alkyl moieties of the alicyclic alkylalkoxycarbonyl and alkoxycarbonyl have the same significance as the above alkyl; the alicyclic alkyl moiety of the alicyclic alkylalkoxycarbonyl has the same significance as the above alicyclic alkyl; and the substituents of the substituted alkenyloxycarbonyl, substituted aralkyloxycarbonyl, substituted alicyclic alkylalkoxycarbonyl and substituted alkoxycarbonyl have the same significance as the above substituents of the substituted alkylamino and the like), including the above substituents of the substituted alkylamino and the like. The substituents of the substituted alkoxycarbonylalkyl include substituted aroyl (herein the aroyl has the same significance as defined above, and the substituents of the substituted aroyl have the same significance as the above substituents of the substituted alkylamino and the like) including the above substituents of the substituted alkylamino and the like.

In the definitions of the above-mentioned substituents, the halogen represents a fluorine, chlorine, bromine or iodine atom. The alkyl moieties of the alkanoyl, alkanoyloxy, alkyl, alkoxy, alkoxycarbonyl, alkoxycarbonylamino, alkoxycarbonyloxy, dialkylcarbamoyloxy, alkoxyaralkyloxycarbonyl and aroylalkoxy have the same significance as the above alkyl. The aryl moieties of the aroyl, aroyloxy and aroylalkoxy have the same significance as the above aryl. The alkenyl moiety of the alkenyloxycarbonyl has the same significance as the above alkenyl. The aralkyl moieties of the aralkyloxy, aralkyloxycarbonyl and alkoxyaralkyloxycarbonyl have the same significance as the aralkyl.

The protecting groups of Compound (IIIa) through Compound (IIIc) include any of those, which can be used as a protecting group. The examples of the protecting group for an amino group include substituted or unsubstituted alkoxycarbonyl, substituted or unsubstituted aryloxycarbonyl, substituted or unsubstituted alkanoyl and substituted or unsubstituted aroyl. The examples of the protecting group for a carboxyl group include substituted or unsubstituted alkoxy, substituted or unsubstituted aryloxy and the like [See "Basis and Experiment for Peptide Synthesis" Izumiya et al., Ed., Maruzen (1985)].

The alkyl moieties of the above alkoxycarbonyl, alkanoyl and alkoxy have the same significance as the above alkyl. The aryl moieties of the aryloxycarbonyl, aroyl and aryloxy have the same significance as the above aryl. The substituents of the substituted alkoxycarbonyl, substituted aryloxycarbonyl, substituted alkanoyl, substituted aroyl, substituted alkoxy and substituted aryloxy have the same significance as the above substituents of the substituted alkylamino and the like.

Pharmaceutically acceptable salts of Compound (I) include pharmaceutically acceptable acid addition salts, metal salts, ammonium salts, organic amine addition salts, and amino acid addition salts. The acid addition salts include inorganic salts such as hydrochloride, hydrobromide, sulfate and phosphate, and organic salts such as formate, acetate, trifluoroacetate, benzoate, maleate, fumarate, succinate, tartrate, citrate, oxalate, methanesulfonate and p-toluene sulfonate. The metal salts include alkali metal salts such as lithium salts, sodium salts and potassium salts, alkaline earth metal salts such as magnesium salts and calcium salts, aluminium salts and zinc salts. The ammonium salts include ammonium and tetramethyl ammonium. The organic amine addition salts include addition salts of morpholine and piperidine, and the amino acid addition salts include addition salts of an amino acid such as glycine, phenylalanine, aspartic acid, glutamic acid and lysine.

The salts of Compound (II) and Compound (III) include various salts that are available as the salts of Compound (II) and Compound (III) comprising pharmaceutically acceptable salts described above.

The processes for the preparation of Compound (I), Compound (II) and Compound (III) are described below.

In the processes described below, when the compounds used as starting materials or one or two or more functional groups of the products are changed under the conditions of the practical method, or are not appropriate in performing the method, the objective compounds can be obtained using standard methods used in synthetic organic chemistry, for example, protection and deprotection of functional groups [for example, see "Protective Groups in Organic Synthesis" Greene, T. W. Ed., John Wiley & Sons, Inc. (1981)], oxidation, reduction, hydrolysis or the like. Also, the order of the reaction steps such as introduction of the substituents can be altered, if necessary.

### Process 1

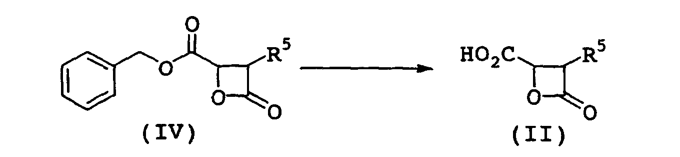

(wherein R⁵ has the same significance as defined above.)

Compound (II) can be obtained by hydrogenating Compound (IV), which can be produced in a manner similar to that described in the references [Corey, E.J. et al., J. Am. Chem. Soc., 120, 2330-2336 (1998); S. Cammas et al., Polymer Bulletin, 33, 149-158 (1994)] in an inert solvent in the presence of a catalyst. The solvents used for the reaction include methanol, ethanol, water and the like. The catalysts include, for example, palladium carbon, platinum oxide or the like, and are usually used in an amount of 0.1 equivalent or more, preferably 1 to 200 equivalents based on Compound (IV). The reaction usually terminates in the range of 0 to 50°C within 10 minutes to 24 hours. Also, Compound (II) can be produced in a manner similar to that described in Bajwa, J. S. et al., J. Org. Chem., 48, 1114-1116 (1983).

### Process 2

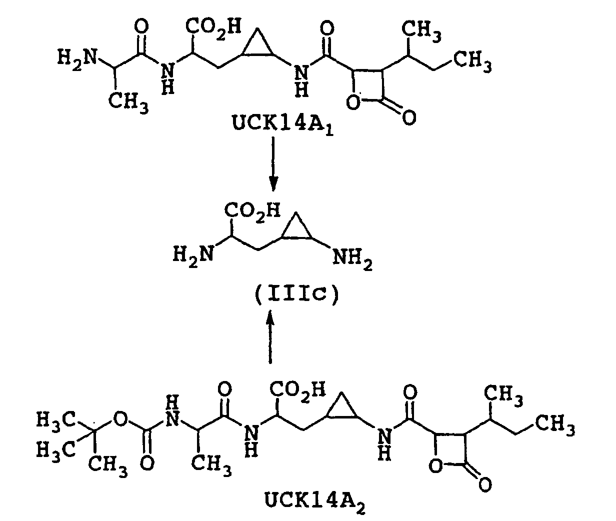

Compound (IIIc) can be obtained by treating UCK14A₁ or UCK14A₂ in an inert solvent in the presence of an acid.

The solvent used in the reaction may be any of water, dimethylformamide, acetonitrile or the like or the reaction may be conducted without solvents. The acid may be any of inorganic acids such as hydrochloric acid, sulfuric acid or the like, but hydrochloric acid is preferably used usually in an amount of 1 equivalent or more, preferably 1 to 200 equivalents based on UCK14A₁ or UCK14A₂. The reaction usually terminates in the range of 0 to 150°C within 1 to 24 hours.

### Process 3

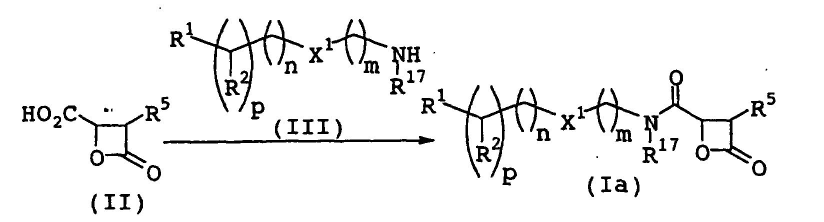

(wherein each of m, n, p, R¹, R², R⁵, R¹⁷ and X¹ has the same significance as defined above)

Compound (Ia), i.e., Compound (I) wherein R³ and R⁴ together represent a bond and X² is N-R¹⁷ (wherein R¹⁷ has the same significance as defined above) can be obtained by reacting Compound (II) with Compound (III) obtained by synthesis from an amino acid such as lysine, ornithine, Compound (IIIc), or the like [See "Basis and Experiment for Peptide Synthesis" Izumiya et al., Ed., Maruzen (1985)], in an inert solvent in the presence of a condensing agent. The solvent used in the reaction may be any of inert solvents for the reaction such as chloroform, dichloromethane, ether, tetrahydrofuran, acetone, dimethylformamide, acetonitrile, and the like, and these may be used alone or in combination. The condensing agent may be any of those conventionally used for condensation between a carboxylic acid and an amine, and for example, dicyclohexylcarbodiimide, 1-ethyl-3-(3-dimethylaminopropyl)carbodiimide hydrochloride or the like may be used. 1-Hydroxybenzotriazol, N-hydroxysuccinimide, or the like can be further added thereto in an amount of 1 to 10 equivalents. Compound (III) and the condensing agent are usually used in an amount of 1 equivalent or more, preferably 1 to 200 equivalents based on Compound (II). The reaction usually terminates in the range of 0 to 50°C within 5 minutes to 24 hours.

### Process 4

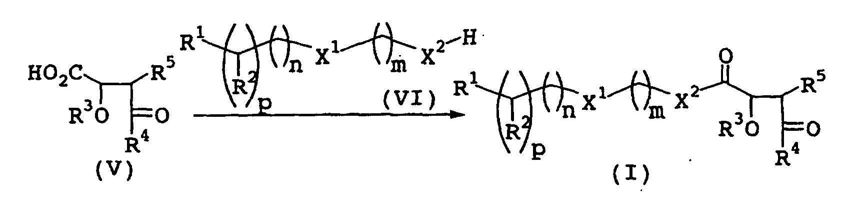

(wherein each of m, n, p, R¹, R², R³, R⁴, R⁵, X¹ and X² has the same significance as defined above)

Compound (I) can be obtained from Compound (V) [See Bajwa, J. S. et al., J. Org. Chem., 48, 1114-1116 (1983)] and Compound (VI) in a manner similar to that described in Process 3.

### Process 5

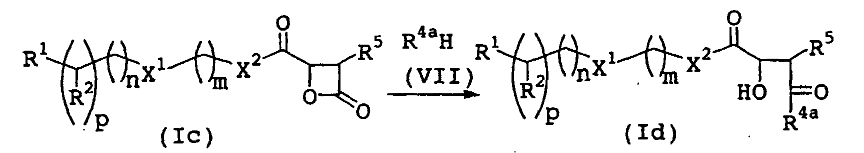

(wherein each of m, n, p, R¹, R², R⁵, X¹ and X² has the same significance as defined above, and R^{4a} represents a group defined for the above R⁴ except when R³ and R⁴ together represent a bond)

Compound (Id), i.e., Compound (I) wherein R³ is a hydrogen atom, can be synthesized by the above-mentioned Process 4, however, can also be obtained by reacting Compound (Ic), i.e., Compound (I) wherein R³ and R⁴ are bound together, with Compound (VII) in an inert solvent in the presence of a base. The solvent used in the reaction may be any of inert solvents for the reaction described in Process 3, and dimethyl sulfoxide is preferably used. Amines such as pyridine, imidazole, triethylamine, diisopropylethylamine, or the like, or carbonates, bicarbonates or phosphates of an alkali metal or an alkaline earth metal such as sodium carbonate, potassium carbonate, calcium carbonate, sodium hydrogencarbonate, or the like may be used as the base. Compound (VII) and the base are usually used in an amount of 1 equivalent or more, preferably 1 to 200 equivalents based on Compound (Ic). The reaction usually terminates in the range of -10 to 50°C within 10 minutes to 24 hours.

### Process 6

Compound (Ie), i.e., Compound (I) wherein R¹ is NHC(=O)-C(CH₃)NR⁹R¹⁰; R² is COR^{13a} (wherein R^{13a} represents a group defined for R¹³ except for hydroxy); m is 0; n and p are 1; R³ and R⁴ together represent a bond; R⁵ is sec-butyl; X¹ is cyclopropylene or ethylene; and X² is NH, can be synthesized by the above-mentioned Process 3, however, can also be produced, for example, by the following synthetic pathway using UCK14A₂ or Compound (VIII) as a starting material: 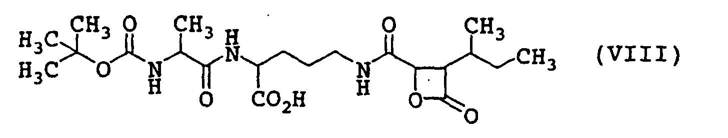 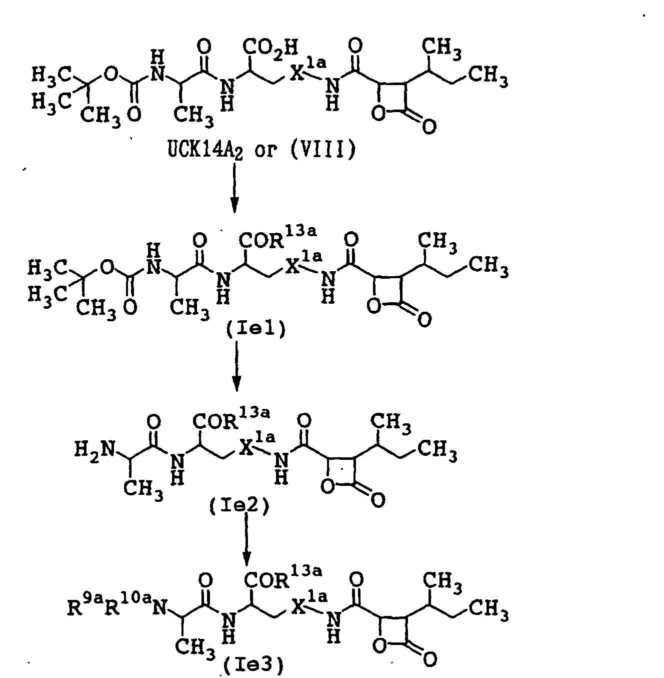 (wherein R^{13a} has the same significance as defined above; R^{9a} and R^{10a} represent groups defined for the above R^{9a} and R^{10a} except when R⁹ is a hydrogen atom and simultaneously R¹⁰ is a hydrogen atom or CO₂C(CH₃)₃; and X^{1a} represents cyclopropylene or ethylene).

Compound (Ie) can also be produced, for example, by the steps described below depending on types of R⁹, R¹⁰, R¹³ and X^{1a} and based on the above-mentioned synthetic pathway.

### (Step 1-1)

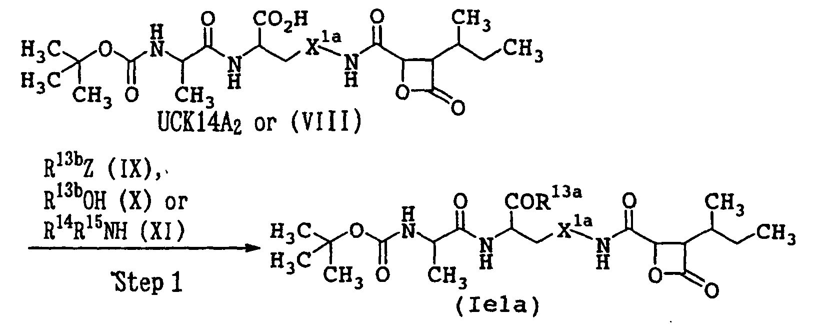

(wherein R^{13b} represents substituted or unsubstituted alkyl, substituted or unsubstituted alkenyl, substituted or unsubstituted aralkyl, substituted or unsubstituted alicyclic alkylalkyl, or substituted or unsubstituted aroylalkyl; each of R^{13a}, R¹⁴, R¹⁵ and X^{1a} has the same significance as defined above; and Z represents chlorine, bromine or iodine)

In the definition of R^{13b}, the alkyl moieties of the alicyclic alkylalkyl and aroylalkyl are as defined for the alkyl descrived above; the alicyclic alkyl moiety of the alicyclic alkylalkyl is as defined for the alicyclic alkyl descrived above; the aryl moiety of the aroylalkyl is as defined for the aryl descrived above; the substituents of the substituted alicyclic alkylalkyl and substituted aroylalkyl are as defined for the substituents of the substituted alkyl descrived above; and each of the substituted or unsubstituted alkyl, substituted or unsubstituted alkenyl, and substituted or unsubstituted aralkyl has the same significance as defined above.

Compound (Ie1), i.e., Compound (Ie) wherein R⁹ is a hydrogen atom and R¹⁰ is represented by CO₂C(CH₃)₃ can be obtained by reacting UCK14A₂ or Compound (VIII) with Compound (IX) in an inert solvent in the presence of a base, or with Compound (X) or Compound (XI) in an inert solvent in the presence of a condensing agent.

The solvent used in the reaction may be any of inert solvents for the reaction described in Process 3, and these solvents may be used alone or in combination. Amines such as pyridine, imidazole, triethylamine, diisopropylethylamine, or the like, or carbonates or bicarbonates of an alkal metal or an alkaline earth metal such as sodium carbonate, potassium carbonate, calcium carbonate, sodium hydrogencarbonate, or the like may be used as the base. Dimethylaminopyridine or the like may also be used as a catalyst. The condensing agent may be any of those conventionally used for condensation between a carboxylic acid and an amine as mentioned above. In the case of Compound (X), dimethylaminopyridine or the like may be further added thereto in an amount of 0.1 to 10 equivalents, and in the case of Compound (XI), 1-hydroxybenzotriazol or the like may be further added thereto in an amount of 1 to 10 equivalents. Compound (IX), Compound (X), Compound (XI), the base and the condensing agent are usually used in an amount of 1 equivalent or more, preferably 1 to 200 equivalents based on UCK14A₂ or Compound (VIII). The reaction usually terminates in the range of 0 to 50°C within 5 minutes to 24 hours.

### (Step 2)

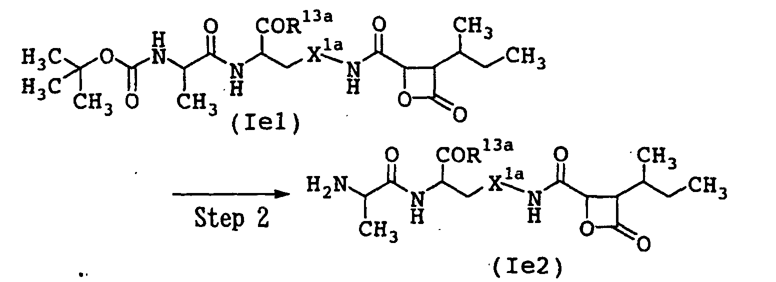

(wherein each of R^{13a} and X^{1a} has the same significance as defined above)

Compound (Ie2), i.e., Compound (Ie) wherein R⁹ and R¹⁰ are simultaneously represented by a hydrogen atom may be obtained by treating Compound (Ie1) in a solvent in the presence of an acid.

The solvent used in the reaction may be any of chloroform, dichloromethane, ether, tetrahydrofuran, acetone, dimethylformamide, acetonitrile, methanol, ethanol or the like, and preferably, chloroform, dichloromethane or the like is used. The acid can be any of organic acids such as p-toluenesulfonic acid, camphorsulfonic acid, pyridinium p-toluenesulfonate, trifluoroacetic acid, trifluoromethanesulfonic acid, and the like, inorganic acids such as hydrochloric acid, sulfuric acid, and the like, and Lewis acids such as titanium tetrachloride, boron trifluoride diethyl etherate, and the like, however, preferably trifluoroacetic acid is used usually in an amount of 1 equivalent or more, preferably 1 to 10 equivalents based on Compound (Ie1). The reaction usually terminates in the range of 0 to 50°C within 5 minutes to 24 hours.

### (Step 3-1)

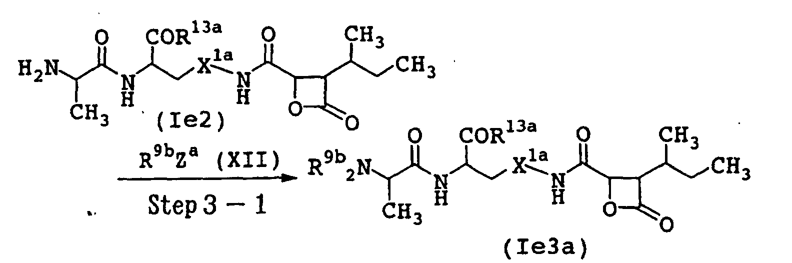

(wherein R^{9b} represents substituted or unsubstituted alkyl, or substituted or unsubstituted aralkyl; Z^{a} is as defined for the above Z; and each of R^{13a} and X^{1a} has the same significance as defined above)

In the definition of R^{9b}, each of substituted or unsubstituted alkyl and substituted or unsubstituted aralkyl has the same significance as defined above.

Compound (Ie3a), i.e., Compound (Ie) wherein R⁹ and R¹⁰ are identical substituted or unsubstituted alkyl, or substituted or unsubstituted aralkyl can also be obtained by reacting Compound (Ie2) with Compound (XII) in an inert solvent in the presence of a base.

The solvent used in the reaction may be any of inert solvents for the reaction described in Process 3, and these solvents may be used alone or in combination. Amines such as pyridine, imidazole, triethylamine, diisopropylethylamine, or the like, or carbonates or bicarbonates of an alkal metal or an alkaline earth metal such as sodium carbonate, potassium carbonate, calcium carbonate, sodium hydrogencarbonate, or the like may be used as the base, and dimethylaminopyridine or the like may also be used as a catalyst. Compound (XII) and the base are usually used in an amount of 1 equivalent or more, preferably 1 to 200 equivalents based on Compound (Ie2). The reaction usually terminates in the range of 0 to 50°C within 5 minutes to 24 hours.

### (Step 3-2)

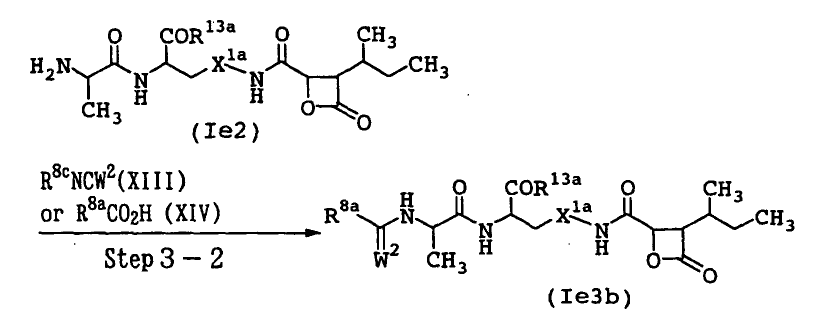

(wherein R^{8c} represents substituted or unsubstituted alkyl, or substituted or unsubstituted aralkyl; and each of R^{8a}, R^{13a}, W² and X^{1a} has the same significance as defined above)

Compound (Ie3b), i.e., Compound (Ie) wherein R⁹ is a hydrogen atom and R¹⁰ is CW²R^{8a} (wherein each of R^{8a} and W² has the same significance as defined above) can be obtained by reacting Compound (Ie2) with Compound (XIII) represented by R^{8c}NCW² (wherein each of R^{8c} and W² has the same significance as defined above) in an inert solvent in the presence of a base, or with Compound (XIV) represented by R^{8a}CO₂H (wherein R^{8a} has the same significance as defined above) in an inert solvent in the presence of a condensing agent.

The solvent used in the reaction may be any of inert solvents for the reaction described in Process 3, and these solvents may be used alone or in combination. Amines such as pyridine, imidazole, triethylamine, diisopropylethylamine, or the like may be used as the base, and dimethylaminopyridine or the like can also be used as a catalyst. The condensing agent may be any of those conventionally used for condensation between a carboxylic acid and an amine as mentioned above, and 1-hydroxybenzotriazol or the like may be further added thereto in an amount of 1 to 10 equivalents. Compound (XIII), Compound (XIV), the base and the condensing agent are usually used in an amount of 1 equivalent or more, preferably 1 to 200 equivalents based on Compound (Ie2). The reaction usually terminates in the range of 0 to 50°C within 5 minutes to 24 hours.

### (Step 3-3)

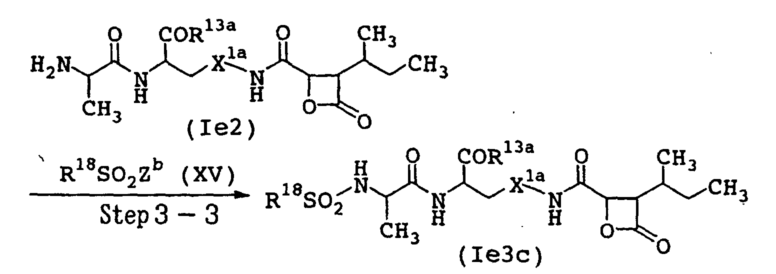

(wherein Z^{b} is as defined for the above Z; each of R^{13a} and X^{1a} has the same significance as defined above; and R¹⁸ represents substituted or unsubstituted alkyl, or substituted or unsubstituted aryl)

In the definition of R¹⁸, each of substituted or unsubstituted alkyl and substituted or unsubstituted aryl has the same significance as defined above.

Compound (Ie3c), i.e., Compound (Ie) wherein R⁹ is a hydrogen atom and R¹⁰ is SO₂R¹⁸ (wherein R¹⁸ has the same significance as defined above) can also be obtained by reacting Compound (Ie2) with Compound (XV) in an inert solvent in the presence of a base. The solvent used in the reaction may be any of inert solvents for the reaction described in Process 3, and these solvents may be used alone or in combination. Amines such as pyridine, imidazole, triethylamine, diisopropylethylamine, or the like may be used as the base, and dimethylaminopyridine or the like can also be used as a catalyst. Compound (XV) and the base are usually used in an amount of 1 equivalent or more, preferably 1 to 200 equivalents based on Compound (Ie2). The reaction usually terminates in the range of 0 to 50°C within 5 minutes to 24 hours.

### (Step 3-4)

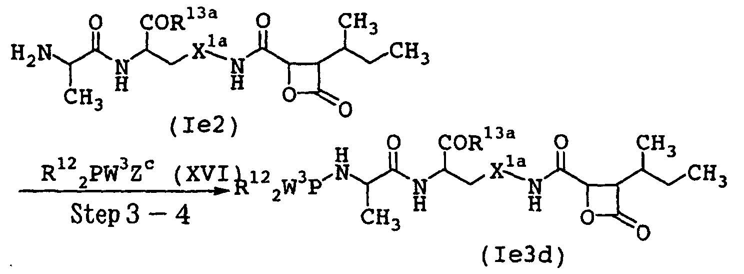

(wherein Z^{c} is as defined for the above Z; and each of R¹², R^{13a}, X^{1a} and W³ has the same significance as defined above)

Compound (Ie3d), i.e., Compound (Ie) wherein R⁹ is a hydrogen atom and R¹⁰ is PW³R¹²₂ (wherein each of R¹² and W³ has the same significance as defined above) can also be obtained by reacting Compound (Ie2) with Compound (XVI) in an inert solvent in the presence of a base. The solvent used in the reaction may be any of inert solvents for the reaction described in Process 3, and these solvents may be used alone or in combination. Amines such as pyridine, imidazole, triethylamine, diisopropylethylamine, or the like may be used as the base, and dimethylaminopyridine or the like may also be used as a catalyst. Compound (XVI) and the base are usually used in an amount of 1 equivalent or more, preferably 1 to 200 equivalents based on Compound (Ie2). The reaction usually terminates in the range of 0 to 50°C within 5 minutes to 24 hours.

In the production of Compound (I), Compound (II) and Compound (III), transformations of functional groups can also be conducted by the method known in the art [for example, Comprehensive Organic Transformations, Larock, R. C., Ed. (1989)], in addition to the above-mentioned method in each step.

The objective compounds in the above-mentioned processes can be isolated and purified by standard purification methods used in synthetic organic chemistry such as neutralization, filtration, extraction, washing, drying, concentration, recrystallization, various types of chromatography, or the like.

Stereoisomers such as diastereomers or the like may exist in some of Compound (I), Compound (II) and Compound (III), and the present invention covers all possible isomers including these and the mixture thereof.

Also, some of Compound (I), pharmaceutically acceptable salts thereof, Compound (II), Compound (III) and salts thereof may be in the form of adducts with water or various solvents, and these adducts are also included in the present invention.

Specific examples of Compound (I) obtained from the above-mentioned processes are shown in Table 1.

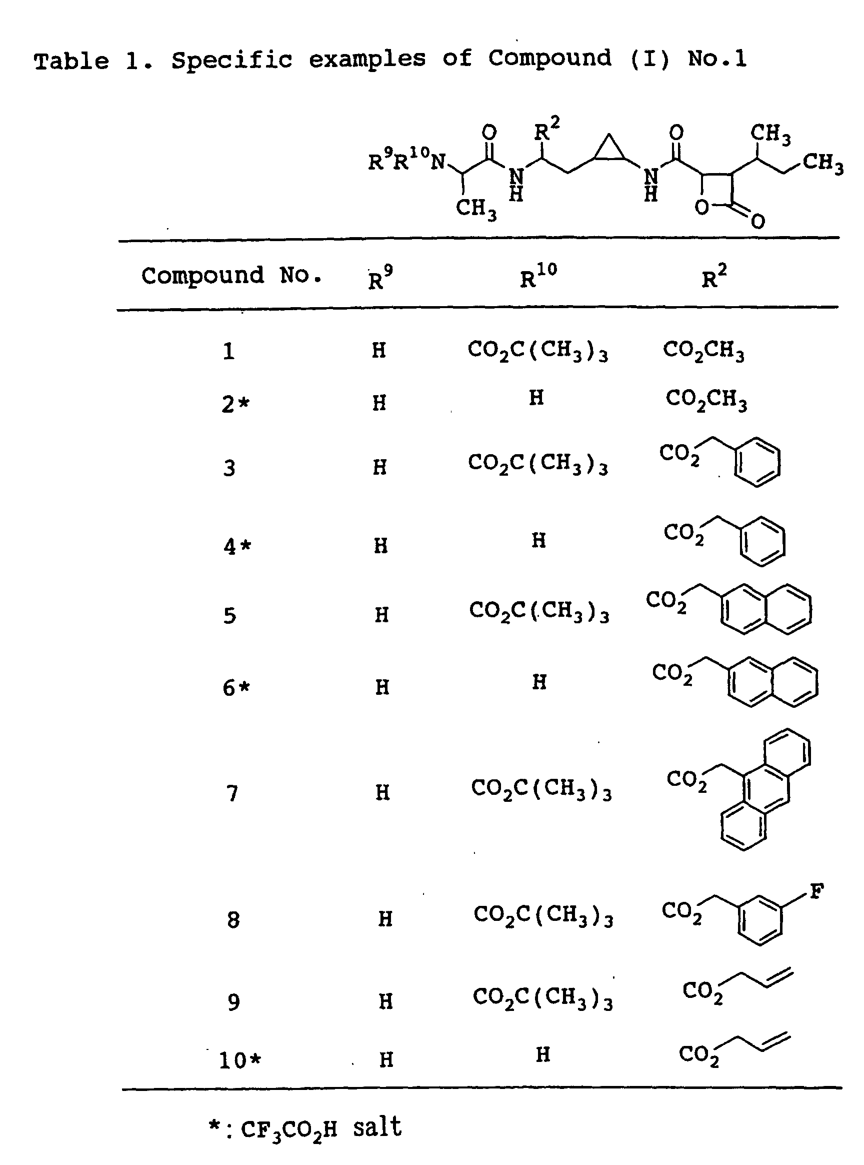

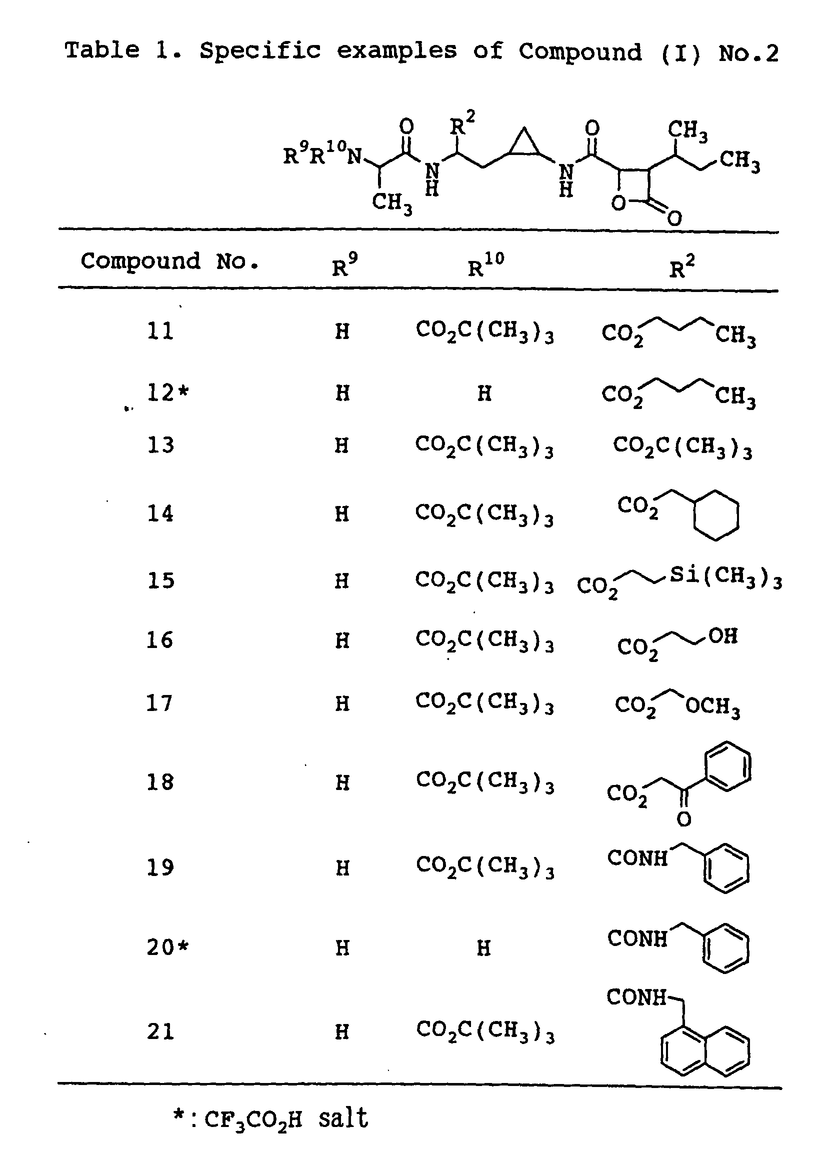

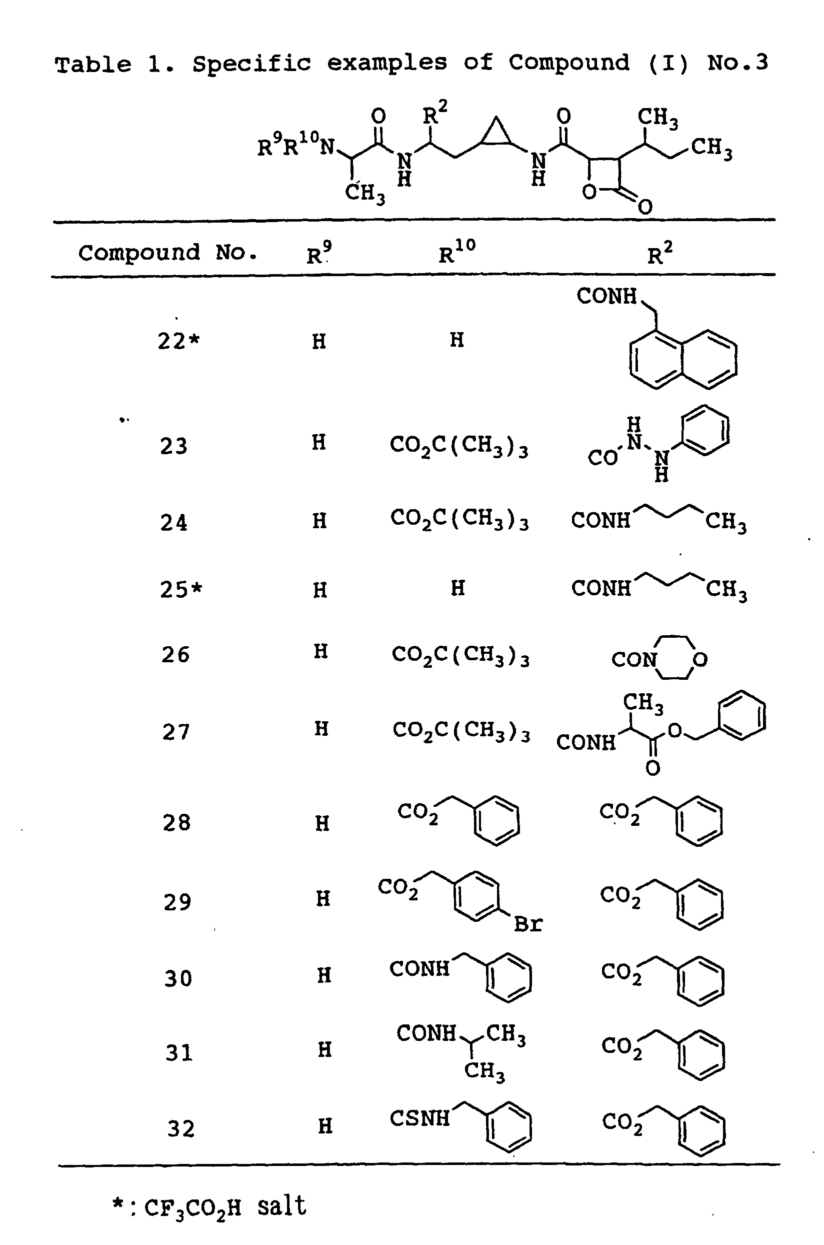

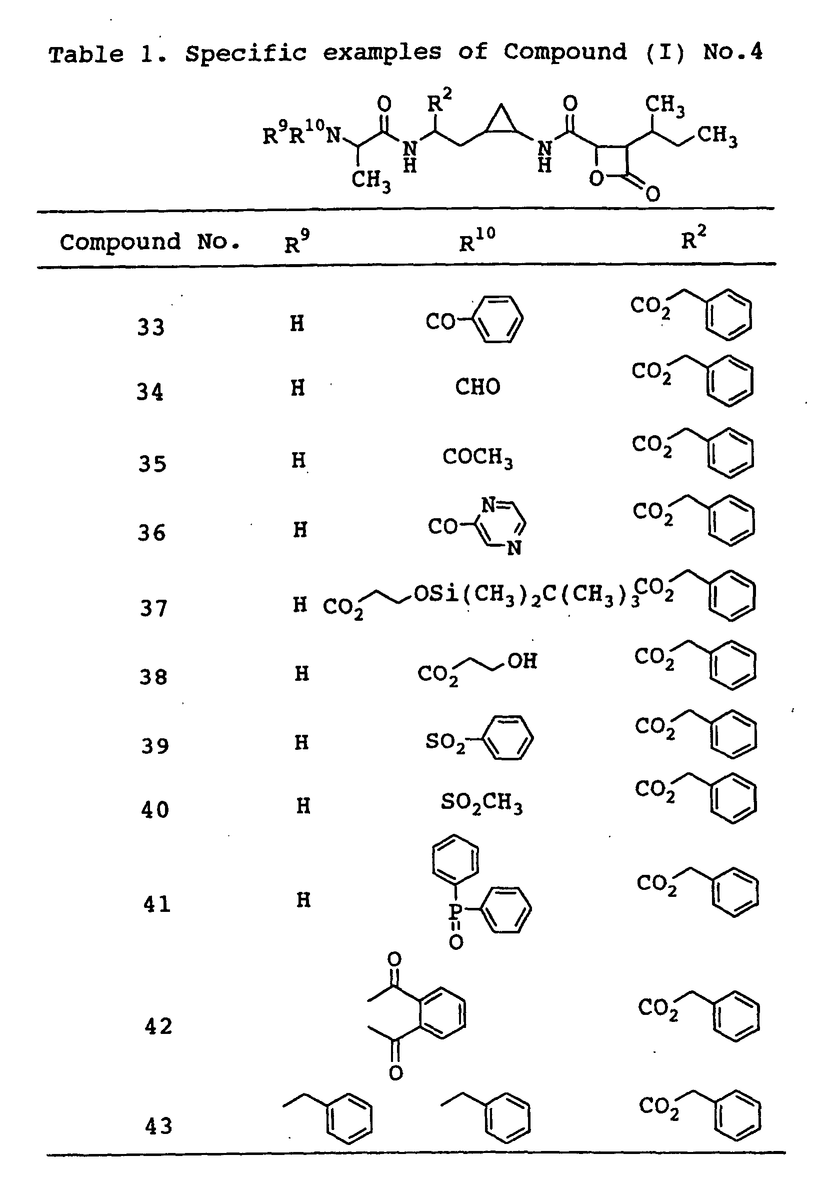

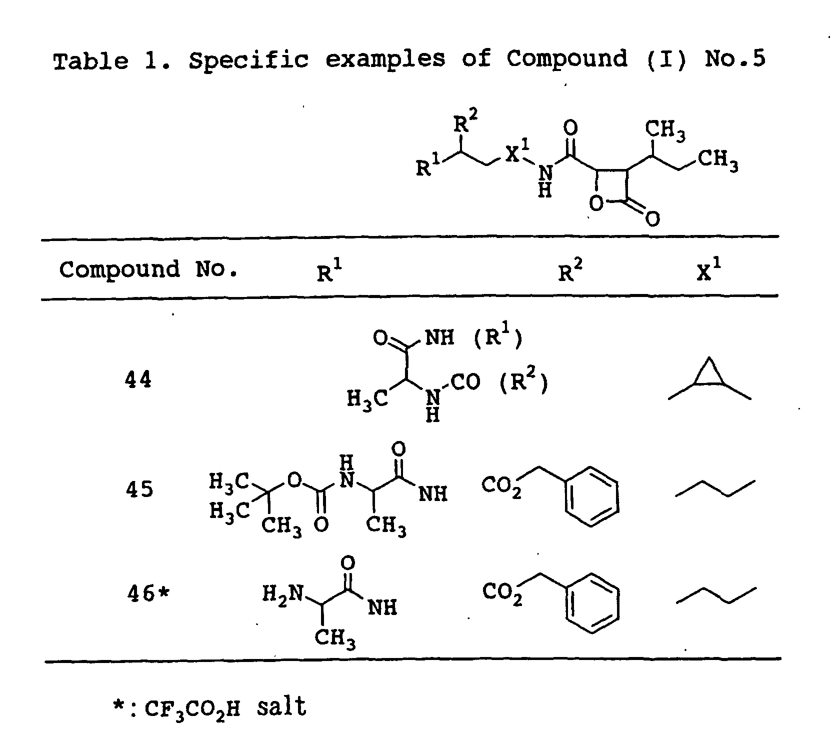

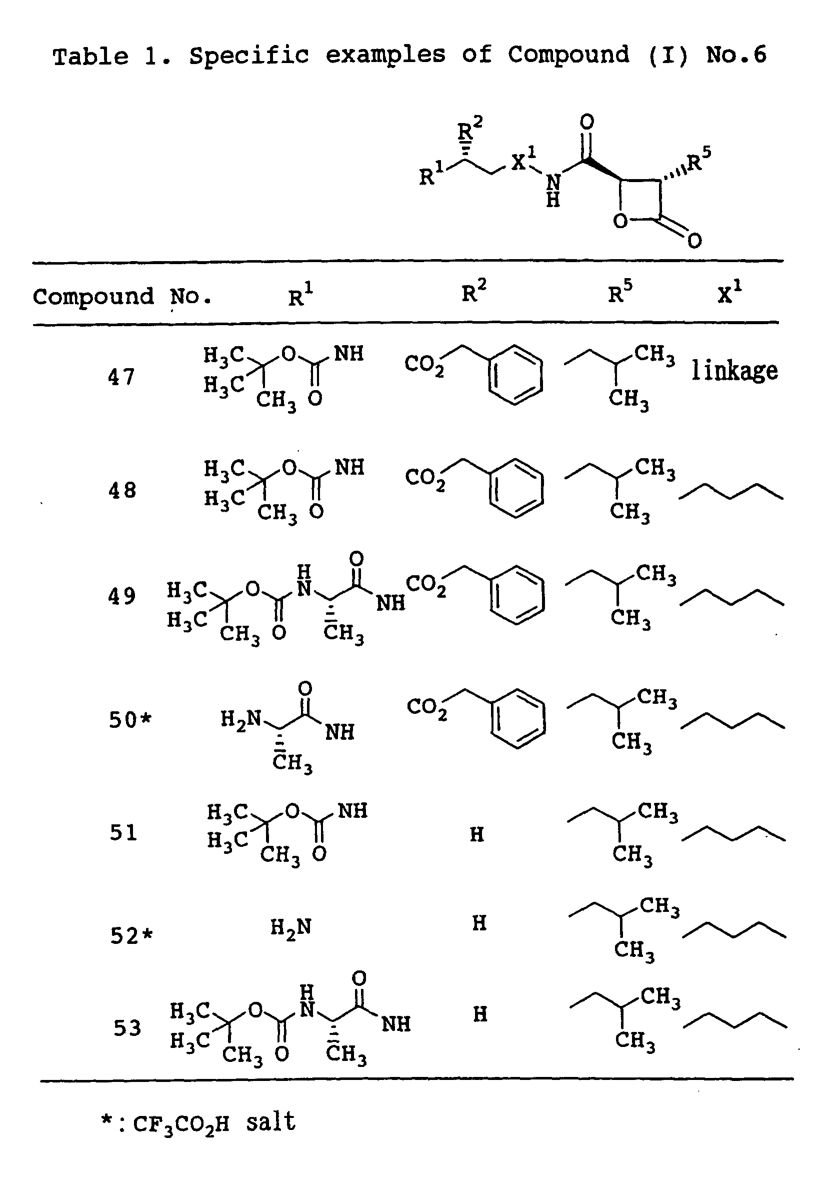

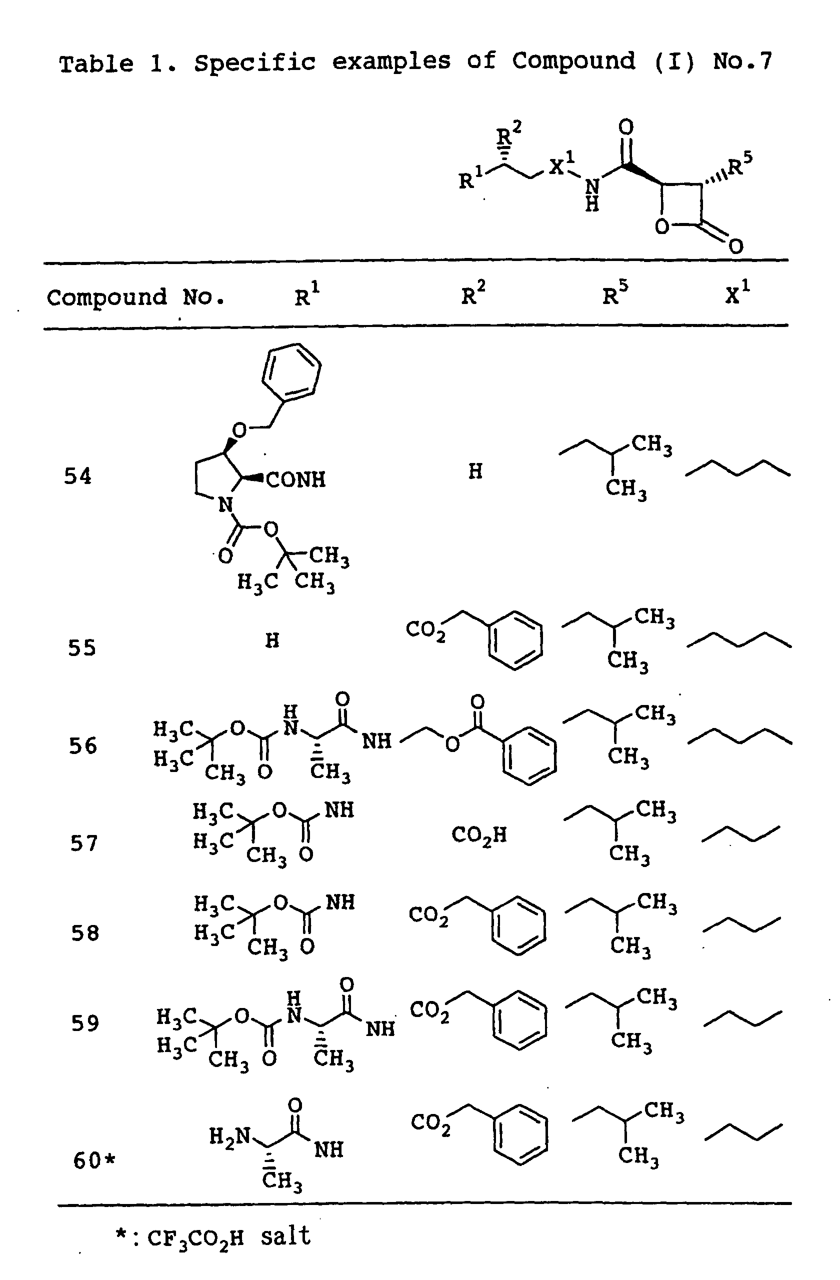

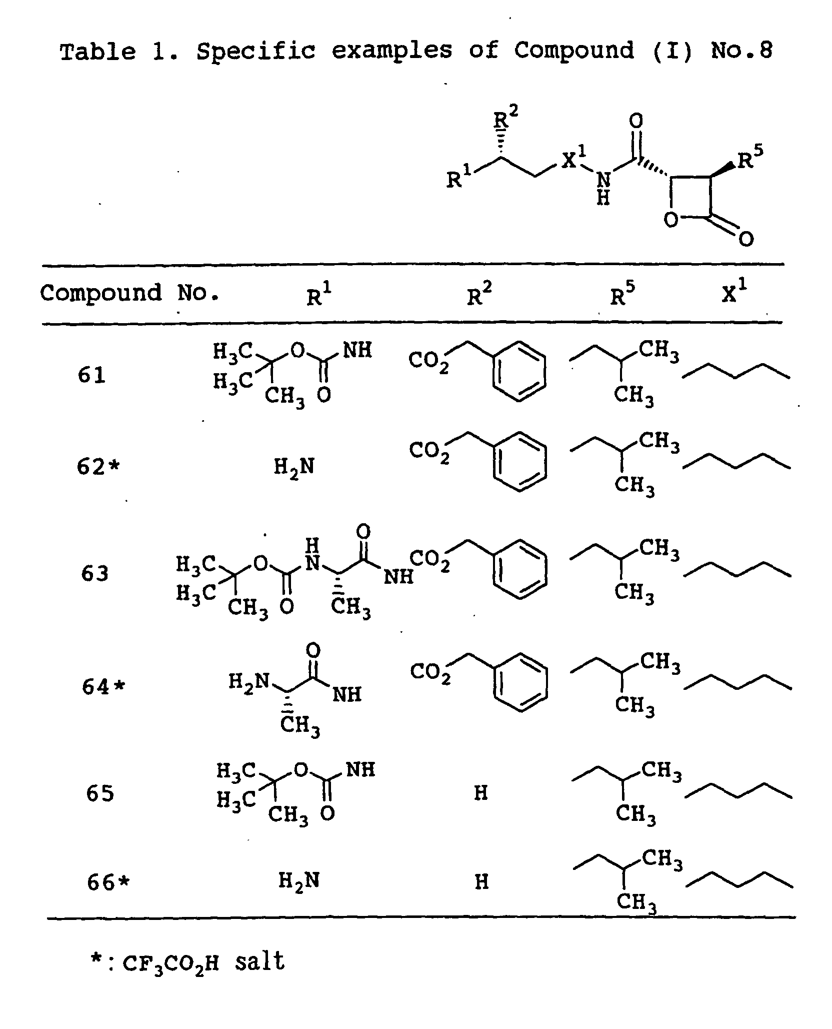

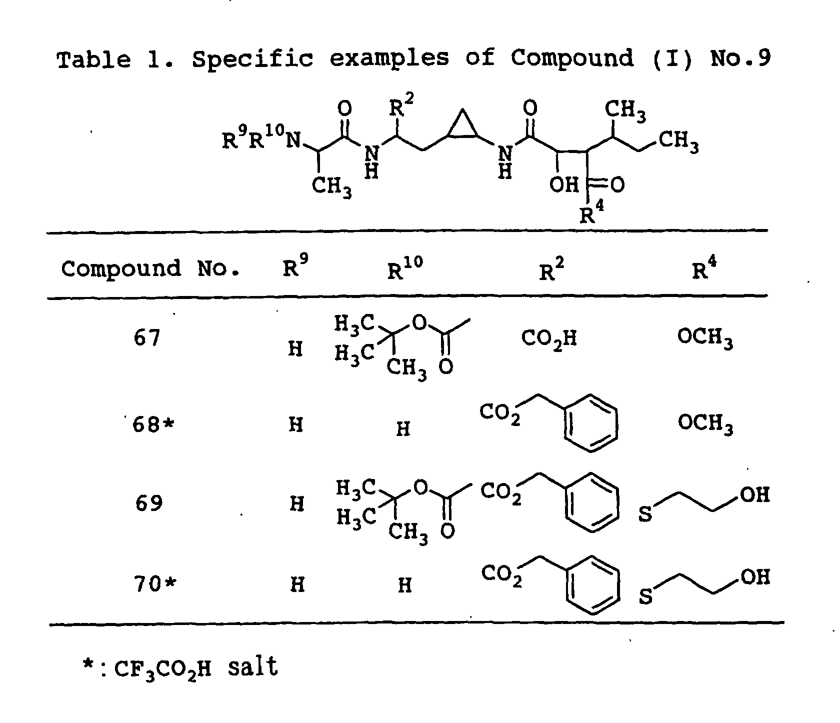

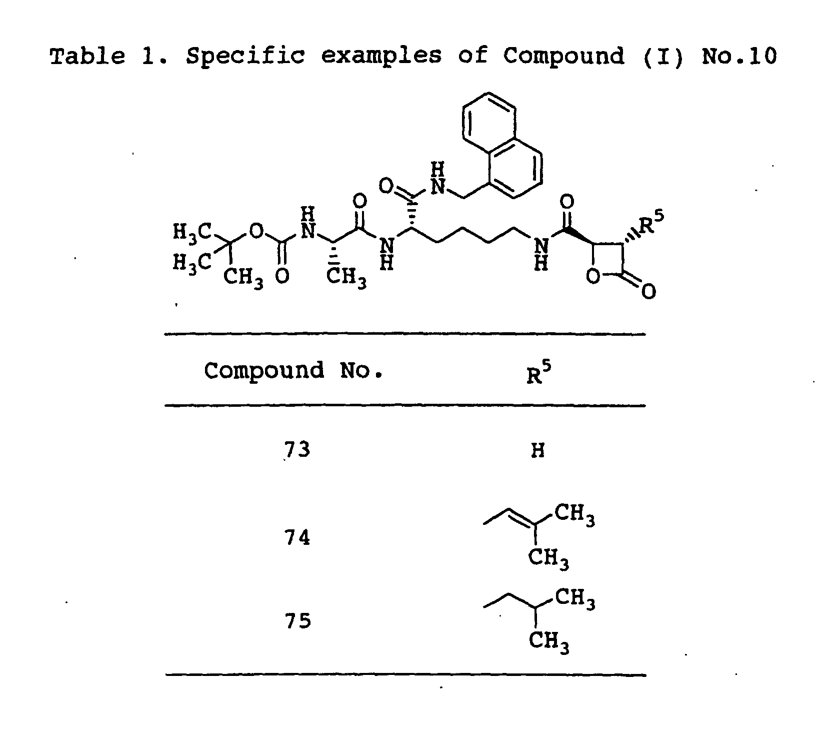

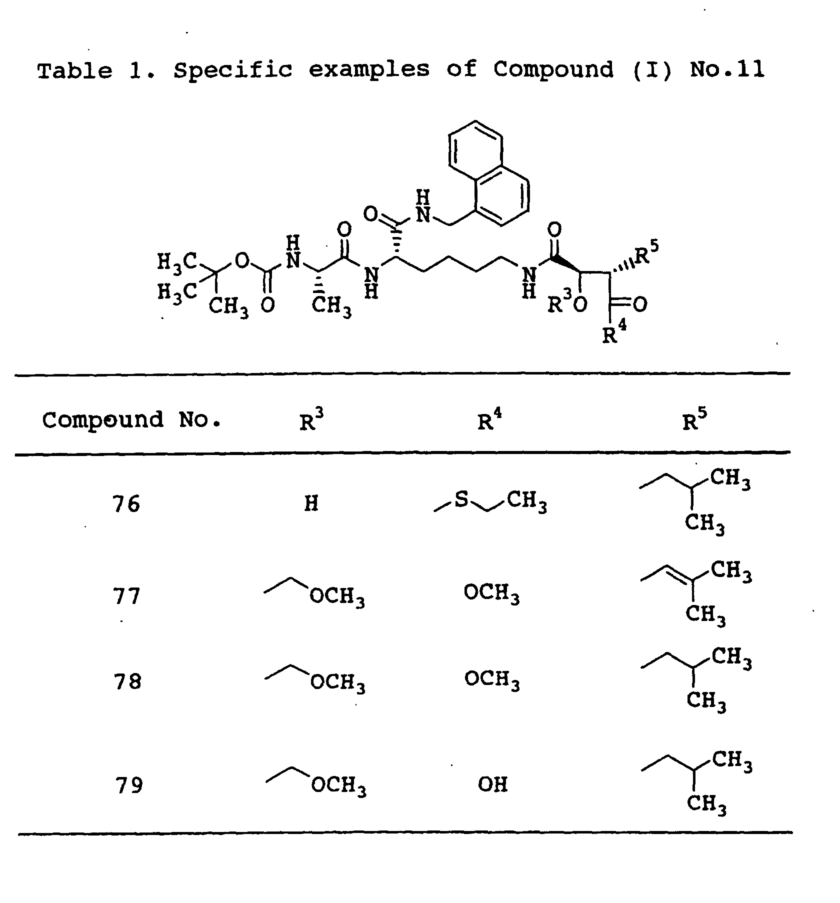

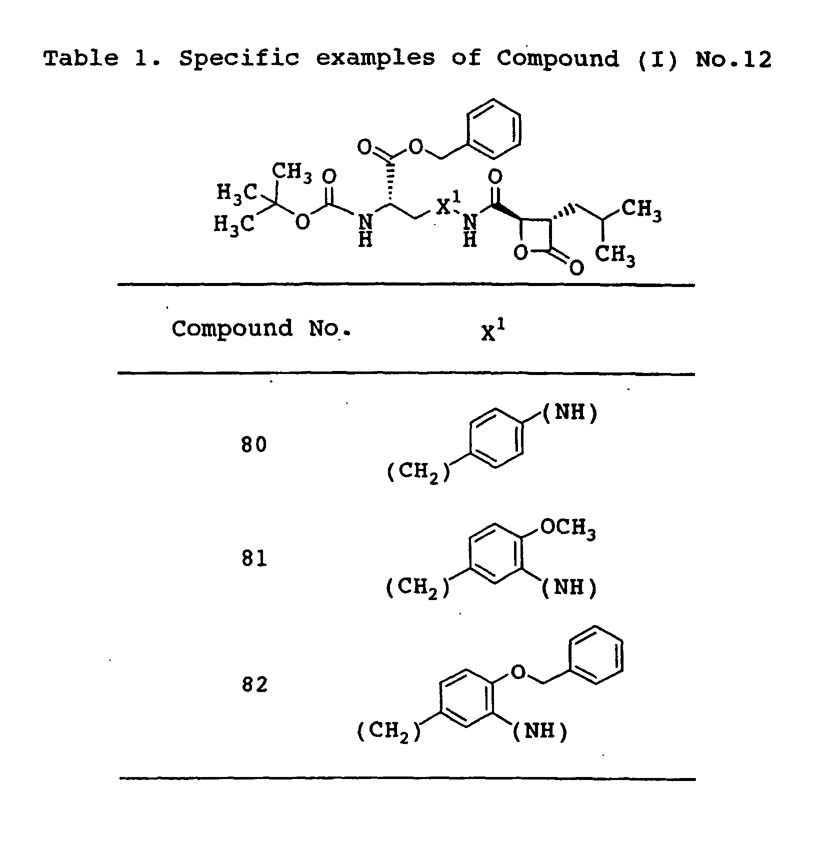

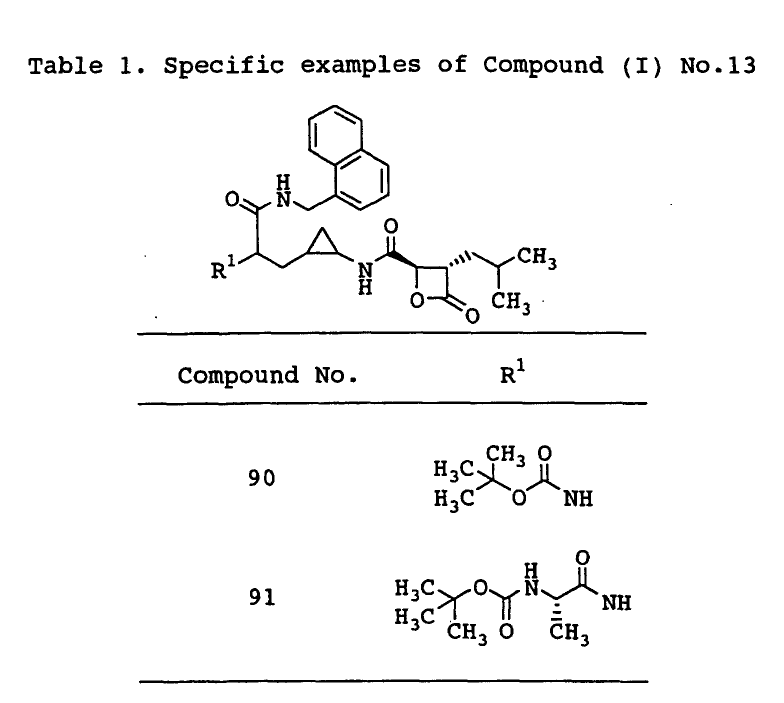

Then, Specific examples of Compound (II) obtained from the above-mentioned processes are shown in Table 2.

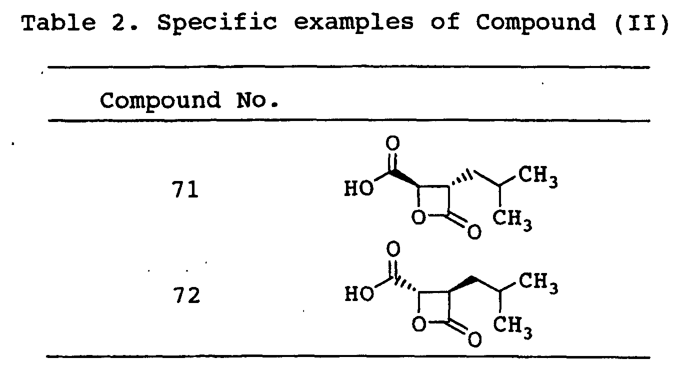

Then, Specific examples of Compound (III) obtained from the above-mentioned processes are shown in Table 3.

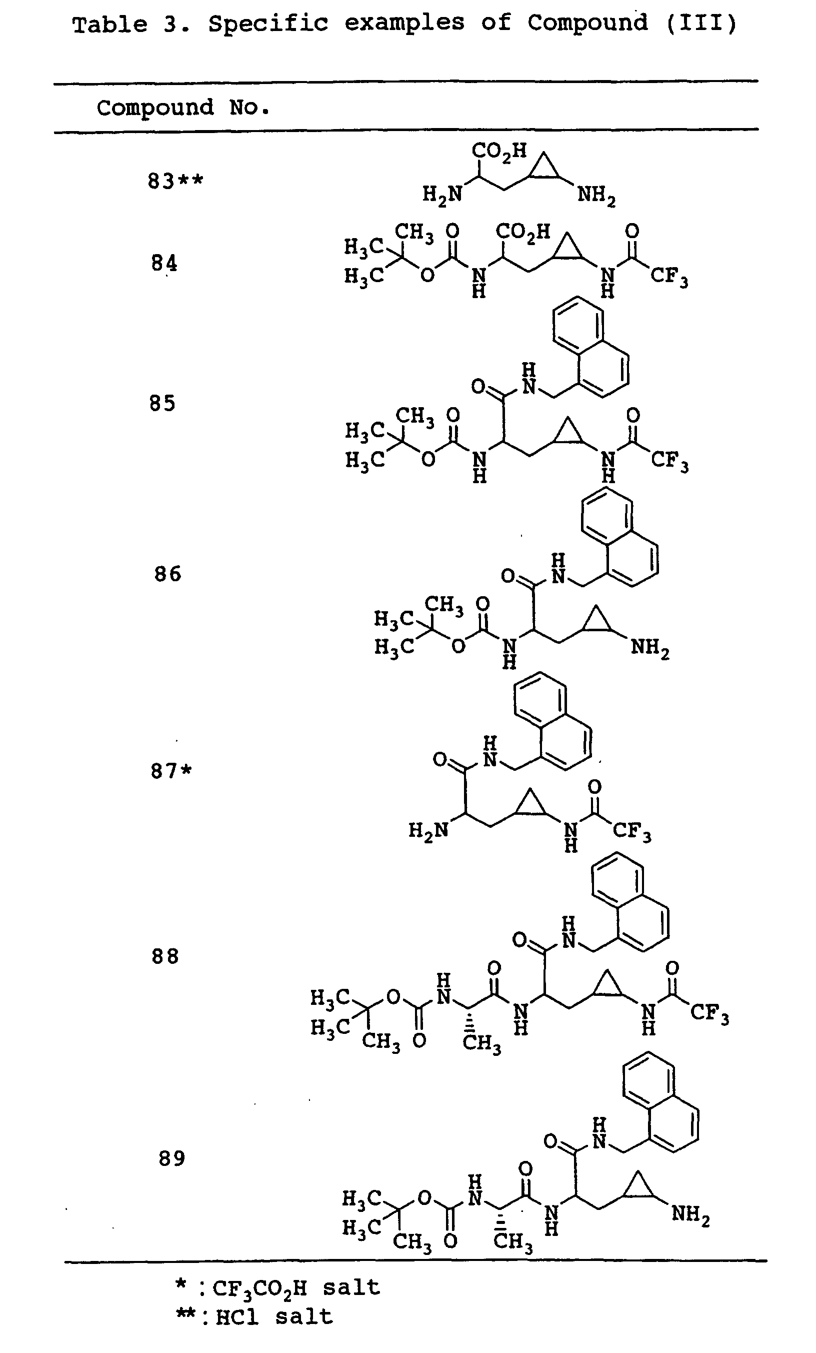

Then, proteasome inhibitory activity, WiDr antiproliferative activity and antitumor activity of representative Compound (I) are described by Test Examples.

### Test Example 1: Proteasome inhibitory activity

A crude enzyme solution fractionated by molecular weight from an extract solution of HeLa S3 cells in reference to the method known in the art was used as proteasome [Ugai S. et al., J. Biochem., 113, 754-768 (1993); Orino E. et al., FEBS, 284, 206-210 (1991)]. 1 x 10⁹ of HeLa S3 cells were dispersed in 5 ml of the buffer A [20 mmol/L of Tris (pH 7.5), 2 mmol/L of ATP, 5 mmol/L of MgCl₂, 1 mmol/L of dithiothreitol (DTT)], and then homogenized at 4°C. The supernatant centrifuged at 100,000G was concentrated by a Centricon-500 (supplied by Amicon) and diluted with 2.5 ml of the buffer B [25 mmol/L of Tris/HCl (pH 7.5), 2 mmol/L of ATP, 1 mmol/L of DTT, 20% of glycerol]. The obtained high molecular fraction (molecular weight >500K) was used as an enzyme solution for the detection of proteasome activity. Suc-Leu-Leu-Val-Tyr-AMC [Suc: Succinyl; AMC: Aminomethylcoumarin](supplied by Peptide Institute, Inc.) was used as a substrate for measuring inhibitory activity for proteasome (stored as a 2 mmol/L DMSO solution). The reaction was carried out by the following method using 96-well microtiter plates. To 96 µl of a reaction buffer [20 mmol/L of Tris/HCl (pH 8), 0.5 mmol/L of EDTA, 0.04% of sodium dodecylsulfate (SDS)] were added 1 µl of a drug solution (DMSO) and 2 µl of an enzyme solution followed by adding 1 µl of a substrate solution thereto, and then the mixture was incubated at 37°C for one hour. After adding 0.1ml of a quenching buffer [0.1 mol/L of Tris (pH 8.9), 0.5 % of SDS] to the reaction mixture, the fluorescent intensity of the released aminomethylcoumarin was measured at Ex 380 nm and Em 460 nm (1420 ARVO supplied by Wallac). The fluorescent intensity without a compound was made 100, and the amount of the compound at which 50% of proteasome activity was inhibited (IC₅₀) was calculated. The results are shown in Table 4.

**Table 4.**

| Inhibitory activity for proteasome (1) | |
|---|---|
| Compound No. | IC₅₀(µmol/L) |
| UCK14A₁ | 0.40 |
| 1 | 0.05 |
| 2 | 0.07 |
| 3 | 0.015 |
| 4 | 0.03 |
| 5 | 0.0062 |
| 6 | 0.038 |
| 7 | 0.017 |
| 8 | 0.014 |
| 9 | 0.026 |
| 10 | 0.046 |
| 11 | 0.019 |
| 12 | 0.051 |
| 13 | 0.03 |
| 14 | 0.018 |
| 15 | 0.018 |
| 16 | 0.052 |
| 17 | 0.019 |
| 18 | 0.012 |
| 19 | 0.0062 |
| 20 | 0.011 |
| 21 | 0.004 |
| 22 | 0.012 |
| 23 | 0.017 |
| 24 | 0.015 |
| 25 | 0.024 |
| 26 | 0.013 |
| 27 | 0.003 |
| 28 | 0.005 |

**Table 4.**

| Inhibitory activity for proteasome (2) | |
|---|---|
| Compound No. | IC₅₀(µmol/L) |
| 29 | 0.005 |
| 30 | 0.004 |
| 31 | 0.011 |
| 32 | 0.005 |
| 33 | 0.015 |
| 34 | 0.038 |
| 35 | 0.027 |
| 36 | 0.021 |
| 37 | 0.006 |
| 38 | 0.041 |
| 39 | 0.008 |
| 40 | 0.024 |
| 41 | 0.012 |
| 42 | 0.022 |
| 43 | 0.014 |
| 44 | 0.047 |
| 45 | 0.006 |
| 46 | 0.006 |
| 47 | 0.15 |
| 48 | 0.050 |
| 49 | 0.030 |
| 50 | 0.030 |
| 53 | 0.25 |
| 54 | 0.047 |
| 55 | 0.077 |
| 56 | 0.022 |
| 58 | 0.026 |
| 59 | 0.033 |
| 60 | 0.04 |
| 74 | 0.003 |
| 75 | 0.001 |
| 81 | 0.5 |
| 82 | 0.1 |
| 90 | 0.022 |
| 91 | 0.024 |

### Test Example 2: Antiproliferative activity in human colon cancer, WiDr cells.

Human colon cancer, WiDr cells suspended in MEM media containing 10% of fetal calf serum were seeded in 96-well microtiter plates at 2 x 10³cells/well, and precultured in an incubator with 5% of CO₂ at 37°C for 24 hours. Then, each compound diluted appropriately with the media was added into the well at 50 µl/well. At this time, a final concentration of each compound is up to 100 µmol/l or 1 µmol/l. The cells were cultured in the incubator with 5% of CO₂ at 37°C for additional 72 hours. At 5 hours before the culturing was terminated, MTT [3-(4,5-dimethylthiazol-2-yl)-2,5-dimethyltetrazolium bromide] dissolved in the medium at a final concentration of 1 mg/mL was added into the well at 50 µl/well. After the culturing, dimethyl sulfoxide was added to the well at 150 µl/well, and the plate was vigorously stirred using a plate mixer to dissolve crystals of MTT-formazan completely. Then, the difference between absorbance at 550 nM and that at 630 nM was measured by a microplate reader. The antiproliferative activity for cellular proliferation was expressed as the concentration at which 50% inhibition of proliferation (IC₅₀) was induced. The results are shown in Table 5.

**Table 5.**

| Antiproliferative activity in human colon cancer, WiDr cells (1) | |
|---|---|
| Compound No. | IC₅₀(72hours, µmol/L) |
| UCK14A₁ | >30 |
| 1 | 0.79 |
| 3 | 0.037 |
| 4 | 0.05 |
| 5 | 0.03 |
| 6 | 0.04 |
| 7 | 0.7 |
| 9 | 0.01 |
| 10 | 0.068 |
| 11 | 0.01 |
| 12 | <0.041 |
| 13 | 0.49 |
| 14 | 0.30 |
| 15 | 0.20 |
| 16 | 0.95 |
| 17 | 0.18 |
| 18 | 0.20 |
| 19 | 0.19 |
| 20 | 0.057 |
| 21 | 0.52 |
| 22 | <0.041 |
| 23 | 0.41 |
| 24 | 0.02 |

**Table 5.**

| Antiproliferative activity in human colon cancer, WiDr cells (2) | |
|---|---|
| Compound No. | IC₅₀(72 hours, µmol/L) |
| 25 | 0.059 |
| 26 | 0.16 |
| 28 | 0.093 |
| 30 | 0.064 |
| 31 | 0.113 |
| 32 | 0.433 |
| 33 | 0.076 |
| 34 | 1.2 |
| 35 | 0.093 |
| 36 | 0.062 |
| 37 | 5.7 |
| 39 | 0.486 |
| 40 | 0.45 |
| 41 | 2.4 |
| 43 | 3.9 |
| 45 | 0.3 |
| 46 | 0.15 |
| 50 | 1.8 |
| 56 | 1.9 |
| 68 | 1.13 |
| 90 | 40 |
| 91 | 22 |

### Test Example 3: Antitumor activity for human colon cancer cell line, WiDr transplanted in nude mice

Compound 4 was dissolved in ethanol and then prepared at the fixed concentrations by adding cremophor EL (supplied by SIGMA) and saline. At this time, final concentrations of ethanol, cremophor EL, and saline are 5%, 7.5% and 87.5%, respectively. 2 mm x 2 mm of the tumor piece of human colon cancer cell line, WiDr was subcutaneously transplanted in the ventral site of a BALB/c-nu/nu mouse. At the time when a tumor volume [a major axis (a), a minor axis (b): ab²/2] attained to 25-60 mm³, the test compound prepared was intraperitoneally administered for 5 consecutive days to five mice as one proup.

For evaluating antitumor activity of the compound, the tumor volume was measured everyday, and the ratio of the volume (V) on the day of each measurement to the volume (V0) on the day when the administration of the compound was started was obtained to calculate T/C(%). The results are shown in Table 6.

**Table 6.**

| Antitumor activity for WiDr solid tumor in mice | | |
|---|---|---|
| Compound No. | Dose (mg/kg/day) | T/C (7days,%) |
| 4 | 5.0 | 56 |
| 4 | 10.0 | 49 |

The compound obtained according to the present invention is useful as an antitumor agent, and can be used as such or in various dosing forms. For example, when Compound (I) or a pharmaceutically acceptable salt thereof is used as an injection, it may be used by dissolving it in a liquid used commonly in the art as a diluent, for example, saline, a glucose solution for injection, a lactose solution for injection, a mannitol solution for injection or the like, or may be used as a freeze dry injection or a powder injection mixed with sodium chloride or the like based on Pharmacopoeia of Japan. Also, an adjuvant such as polyethylene glycol, HCO-60 (surfactant; supplied by Nikko Chemical), or the like, or a carrier such as ethanol and/or liposome, cyclodextrin or the like may be added to these injections. These injections are usually provided for intravenous administration, however, intra-arterial, intraperitoneal and intrathoracic administrations are also possible.

Compound (I) or a pharmaceutically acceptable salt thereof can also be used as an oral agent by mixing it with an appropriate excipient, a disintegrator, a binder, a lubricant or the like and molding by a standard method to make tablets, granules, powders, syrups or the like. Also, Compound (I) or a pharmaceutically acceptable salt thereof can also be administered into the rectum by mixing it with a commonly used carrier and molding by a standard method to make a suppository. Further, Compound (I) or a pharmaceutically acceptable salt thereof can also be used as a transdermal agent by mixing it with a commonly used carrier by a standard method to make nasal drops, eye drops, topical cream or the like.

Compound (I) or a pharmaceutically acceptable salt thereof can be administered orally or parenterally as ointments, injections or the like. Its effective dose and administration schedule vary depending upon the mode of administration, the age, body weight, or conditions of the patients or the like. Generally, administrating 0.01 to 1000 mg/60 kg, preferably mg/60 kg per day once a week or once three weeks is preferred.

### Best Mode for Carrying Out the Invention

Physicochemical data of each compound shown in the following Examples and Reference Examples were measured using the following instruments.

| | | |
|---|---|---|
| MS | JEOL | HX/HX110A |
| ¹H NMR | Bruker | DMX500 (500MHz) |
| | JEOL | α 400 (400MHz) |
| | JEOL | Lambda300 (300MHz) |
| IR | JASCO Corporation | IR-810 |

In physical data of the compounds in the following Examples and Reference Examples, "FABMS" indicates mass spectra by a "FAB" method; "HRFABMS" indicates high resolution mass spectra by a "FAB" method; "calculated" means a theoretical value based on a molecular formula; and "found" means an actual measurement. "ODS" represents octadecylated silica gel.

Also, in the following Examples and Reference Examples, usual post-treatment indicates the following treatment after the reaction.

After the reaction of each step, water, an acid, a buffer or the like is added to a reaction mixture as needed, and the mixture is extracted with a water-insoluble solvent such as ethyl acetate, ether, chloroform, dichloroethane, or the like. The extract is washed with brine or the like followed by drying over anhydrous sodium sulfate, and the solvent is distilled off under reduced pressure.

### Example 1: Synthesis of Compound 1

UCK14A₂ (10 mg, 0.021 mmol) was dissolved in N,N-dimethylformamide (0.20 mL) followed by adding methyl iodide (0.0050 mL, 0.080 mmol) and potassium carbonate (5.0 mg, 0.036 mmol), and then the mixture was stirred at 25°C for one hour. After usual post-treatment, purifying by a chromatography on silica gel (eluted with chloroform/methanol =100/0 to 100/1) afforded Compound 1 (9.5 mg, yield; 92%).
¹H NMR (CDCl₃, 300MHz) δ ppm: 8.99(br d, J=7.8Hz, 1H), 6.57(br s, 1H), 5.36(br d, J=7.1Hz, 1H), 4.91(ddd, J=4.4, 4.6, 8.0Hz, 1H), 4.63(d, J=4.7Hz, 1H), 4.38(m, 1H), 3.73(s, 3H), 3.56(dd, J=4.6, 7.3Hz, 1H), 2.51(m, 1H), 2.44(m, 1H), 1.98(m, 1H), 1.66(m, 1H), 1.39-1.48(m, 12H), 1.34(m, 1H), 1.15(m, 1H), 1.08(d, J=6.8Hz, 3H), 0.96(t, J=7.3Hz, 3H), 0.68-0.83(m, 2H), 0.61(m, 1H)
FABMS m/z: 484(M+H)⁺ calculated for C₂₃H₃₇N₃O₈=483

### Example 2: Synthesis of Compound 2

Compound 1 (2.5 mg, 0.051 mmol) was dissolved in dichloromethane (0.20 mL) followed by adding trifluoroacetic acid (0.05 mL, 0.67 mmol), and then the mixture was stirred at 25°C for 2 hours. After concentrating a reaction mixture, purifying by an ODS column chromatography (eluted with water/methanol=0/100 to 100/0) afforded Compound 2 (2.0 mg, yield; 80%).
¹H NMR (CDCl₃, 300MHz) δ ppm: 9.67(d, J=9.3Hz, 1H), 6.74(br s, 1H), 4.89(m, 1H), 4.67(d, J=4.6Hz, 1H), 4.20(m, 1H), 3.73(s, 3H), 3.55(dd, J=4.7, 7.6Hz, 1H), 2.54(br s, 1H), 2.46(br d, J=14.9Hz, 1H), 1.99(m, 1H), 1.57-1.68(m, 5H), 1.33(m, 1H), 1.07(d, J=6.6Hz, 3H), 0.94 (t, J=7.3Hz, 3H), 0.79(m, 1H), 0.70(m, 1H), 0.63(m, 1H)
FABMS m/z: 384(M+H)⁺ calculated for C₁₈H₂₉N₃O₆=383

### Example 3: Synthesis of Compound 3

UCK14A₂ (9.6 mg, 0.021 mmol) was dissolved in N,N-dimethylformamide (0.24 mL) followed by adding benzyl bromide (0.011 mL, 0.092 mmol) and potassium carbonate (6.0 mg, 0.043 mmol), and then the mixture was stirred at 25°C for 1.5 hours. After usual post-treatment, purifying by a thin layer chromatography (developed with n-hexane/ethyl acetate =1/1) afforded Compound 3 (4.6 mg, yield; 40%).
¹H NMR (CDCl₃, 300MHz)δ ppm: 9.04(br d, J=9.5Hz, 1H), 7.33(m, 5H), 6.52 (br s, 1H), 5.36(br d, J=6.4Hz, 1H), 5.15(s, 2H), 4.96(m, 1H), 4.60(d, J=4.6Hz, 1H), 4.38(m, 1H), 3.74(dd, J=4.6, 7.4Hz, 1H), 2.49(m, 1H), 2.47(m, 1H), 1.97(m, 1H), 1.62(m, 1H), 1.43(s, 9H), 1.38(d, J=7.0Hz, 3H), 1.30(m, 1H), 1.13(m, 1H), 1.06(d, J=6.8Hz, 3H), 0.94(d, J=7.4Hz, 3H), 0.83(m, 1H), 0.73(m, 1H), 0.59(m, 1H)
FABMS m/z: 560(M+H)⁺ calculated for C₂₉H₄₁N₃O₈=559

### Example 4: Synthesis of Compound 4

Compound 3 (5.4 mg, 0.096 mmol) obtained in Example 3 was dissolved in dichloromethane (0.54mL) followed by adding trifluoroacetic acid (0.12 mL, 1.6 mmol), and then the mixture was stirred at 25°C for one hour. After concentrating a reaction mixture, purifying by an ODS column chromatography (eluted with water/acetonitrile=100/0 to 0/100) afforded Compound 4 (5.6 mg, yield; 100%).
¹H NMR (CDCl₃, 300MHz)δ ppm: 9.59(d, J=9.2Hz, 1H), 7.33(m, 5H), 6.71(br s, 1H), 5.14(s, 2H), 4.92(m, 1H), 4.63(d, J=4.6Hz, 1H), 4.13(m, 1H), 3.43(dd, J=4.6, 7.4Hz, 1H), 2.52(m, 1H), 2.46(br d, J=15.6Hz, 1H), 1.96(m, 1H), 1.61(m, 1H), 1.53(d, J=6.8Hz, 3H), 1.30(m, 1H), 1.11(m, 1H), 1.03(d, J=6.8Hz, 3H), 0.93(d, J=7.5Hz, 3H), 0.91(m, 1H), 0.73(m, 1H), 0.59(m, 1H)
FABMS m/z: 460(M+H)⁺ calculated for C₂₄H₃₃N₃O₆=459

### Example 5: Synthesis of Compound 5

In a manner similar to that in Example 3, Compound 5 (29 mg, yield; 69%) was obtained from UCK14A₂ (32 mg, 0.069 mmol), N,N-dimethylformamide (3.2 mL), 2-(bromomethyl)naphthalene (69 mg, 0.031 mmol) and potassium carbonate (20 mg, 12 mmol).
¹H NMR (CDCl₃, 300MHz) δ ppm: 9.12(d, J=9.0Hz, 1H), 7.77-7.88(m, 4H), 7.50(m, 2H), 7.42(dd, J=1.7, 8.4Hz, 1H), 6.63(br s, 1H), 5.39(brd, J=8.1Hz, 1H), 5.34(d, J=13.0Hz, 1H), 5.29(d, J=13.0Hz, 1H), 4.99(ddd, J=4.2, 4.4, 8.6Hz, 1H), 4.58(d, J=4.6Hz, 1H), 4.39(m, 1H), 3.39(dd, J=4.6, 7.4Hz, 1H), 2.43-2.56(m, 2H), 1.73-1.97(m, 2H), 1.54(m, 1H), 1.43(s, 9H), 1.38(d, J=7.0Hz, 3H), 1.26(m, 1H), 1.00(d, J=6.6Hz, 3H), 0.87(t, J=7.4Hz, 3H), 0.60-0.87(m, 2H), 0.5(m, 1H)
FABMS m/z: 610(M+H)⁺ calculated for C₃₃H₄₃N₃O₈=609
HRFABMS calculated for C₃₃H₄₄N₃O₈ (M+H)⁺ 610.3128 found 610.3129

### Example 6: Synthesis of Compound 6

In a manner similar to that in Example 4, Compound 6 (10 mg, yield; 99%) was obtained from Compound 5 (12 mg, 0.020 mmol) obtained in Example 5, dichloromethane (1.2 mL) and trifluoroacetic acid (0.24 mL, 3.1 mmol).
¹H NMR (CDCl₃, 300MHz) δ ppm: 9.59(brd, J=9.2Hz, 1H), 7.70-7.91(m, 4H), 7.43-7.56(m, 2H), 7.38(dd, J=1.4, 8.4Hz, 1H), 6.77(br s, 1H), 5.31(d, J=12.5Hz, 1H), 5.26(d, J=14.7Hz, 1H), 4.94 (m, 1H), 4.59(d, J=4.2Hz, 1H), 4.15(m, 1H), 3.34(dd, J=4.4, 7.3Hz, 1H), 2.38-2.60(m, 2H), 1.82(m, 1H), 1.40-1.65(m, 2H), 1.27(d, J=9.0Hz, 3H), 1.18(m, 1H), 0.95(d, J=6.6Hz, 3H), 0.88(m, 1H), 0.84(t, J=7.3Hz, 3H), 0.50-0.74(m, 2H)
FABMS m/z: 510(M+H)⁺ calculated for C₂₈H₃₅N₃O₆=509
HRFABMS calculated for C₂₈H₃₆N₃O₆ (M+H)⁺ 510.2605 found 510.2624

### Example 7: Synthesis of Compound 7

UCK14A₂ (49 mg, 0.10 mmol) was dissolved in acetonitrile (4.9 mL) followed by adding 9-(chloromethyl)anthracene (0.12 g, 0.52 mmol) and potassium carbonate (29 mg, 0.21 mmol), and then the mixture was stirred at 25°C for 7.5 hours. After usual post-treatment, the crude product (24 mg) was obtained by purifying with a thin layer chromatography (developed with n-hexane/ethyl acetate=1/1), and further purified by a preparative high performance liquid chromatography (HPLC) (ODS column, eluted with acetonitrile/water=80/20) to afford Compound 7 (16 mg, yield; 23%).
IR(KBr): 3244, 2970, 2930, 1836, 1708, 1656, 1529, 1492, 1451, 1383, 1366, 1246, 1167, 1098, 958, 913, 886, 734 cm⁻¹
¹H NMR (CDCl₃, 300MHz)δ ppm: 9.02(br d, J=9.2Hz, 1H), 8.50(s, 1H), 8.28(d, J=8.8Hz, 2H), 8.02(d, J=8.3Hz, 2H), 7.46-7.59(m, 4H), 6.40(br s, 1H), 6.23(d, J=12.4Hz, 1H), 6.13(d, J=12.7Hz, 1H), 5.31(brd, J=7.9Hz, 1H), 4.93(ddd, J=4.1, 4.7, 8.8Hz, 1H), 4.45(d, J=4.6Hz, 1H), 4.39(m, 1H), 2.86(dd, J=5.1, 5.9Hz, 1H), 2.32-2.43(m, 2H), 1.89(m, 1H), 1.67(m, 1H), 1.40(s, 9H), 1.22-1.45(m, 2H), 1.13(d, J=6.8Hz, 3H), 0.93(d, J=6.8Hz, 3H), 0.81(t, J=7.4Hz, 3H), 0.57(m, 1H), 0.41-0.49(m, 2H)
FABMS m/z: 660(M+H)⁺ calculated for C₃₇H₄₅N₃O₈=659
HRFABMS calculated for C₃₇H₄₆N₃O₈ (M+H)⁺ 660.3285 found 660.3281

### Example 8: Synthesis of Compound 8

UCK14A₂ (49 mg, 0.11 mmol) was dissolved in N,N-dimethyl-formamide (4.9 mL) followed by adding 3-fluorobenzyl bromide (0.065mL, 0.53 mmol) and potassium carbonate (29 mg, 0.21 mmol), and then the mixture was stirred at 25°C for one hour. After usual post-treatment, purifying by a chromatography on silica gel (eluted with n-hexane/ethyl acetate=1/1) afforded Compound 8 (48 mg, yield; 78%).
¹H NMR (CDCl₃, 300MHz)δ ppm: 9.17(d, J=9.2Hz, 1H), 7.32(m, 1H), 6.98-7.16 (m, 3H), 6.75(br s, 1H), 5.39(br d, J=7.7Hz, 1H), 5.15(s, 2H), 4.98(ddd, J=4.6, 4.6, 9.2Hz, 1H), 4.62(d, J=4.6Hz, 1H), 4.39(m, 1H), 3.50(dd, J=4.6, 7.5Hz, 1H), 2.50(m, 2H), 1.86-2.07(m, 2H), 1.63(m, 1H), 1.43(s, 9H), 1.39(d, J=7.0Hz, 3H), 1.28(m, 1H), 1.05(d, J=6.8Hz, 3H), 0.91(m, 3H), 0.78(m, 1H), 0.68(m, 1H), 0.60(m, 1H)
FABMS m/z: 578(M+H)⁺ calculated for C₂₉H₄₀N₃O₈F=577
HRFABMS calculated for C₂₉H₄₁N₃O₈F (M+H)⁺ 578.2878 found 578.2886

### Example 9: Synthesis of Compound 9

UCK14A₂ (50 mg, 0.11 mmol) was dissolved in N,N-dimethylformamide (5.0 mL) followed by adding allyl bromide (0.041 mL, 0.47 mmol) and potassium carbonate (31 mg, 0.22 mmol), and then the mixture was stirred at 25°C for 0.5 hour. After usual post-treatment, the crude product (41 mg) was obtained by purifying with a chromatography on silica gel (eluted with n-hexane/ethyl acetate=3/2), and further purified by a preparative HPLC (ODS column, eluted with acetonitrile/water =55/45) to afford Compound 9 (35 mg, yield; 63%).
¹H NMR (CDCl₃, 300MHz)δ ppm: 9.10(br d, J=8.8Hz, 1H), 6.70(br s, 1H), 5.89(ddd, J=5.7, 10.7, 16.2Hz, 1H), 5.40(br d, J=7.9Hz, 1H), 5.32(dd, J=1.4, 17.2Hz, 1H), 5.23(dd, J=1.3, 10.4Hz, 1H), 4.94(ddd, J=4.2, 4.6, 8.8Hz, 1H), 4.63(m, 3H), 4.40(m, 1H), 3.54(dd, J=4.6, 7.5Hz, 1H), 2.43-2.56(m, 2H), 1.98(m, 1H), 1.65(m, 1H), 1.44(s, 9H), 1.24-1.39(m, 5H), 1.08(d, J=6.1Hz, 3H), 0.95(t, J=7.5Hz, 3H), 0.68-0.85(m, 2H), 0.61(m, 1H)
FABMS m/z: 510(M+H)⁺ calculated for C₂₅H₃₉N₃O₈=509
HRFABMS calculated for C₂₅H₄₀N₃O₈ (M+H)⁺ 510.2815 found 510.2833

### Example 10: Synthesis of Compound 10

In a manner similar to that in Example 4, Compound 10(18 mg, yield; 100%) was obtained from Compound 9 (18 mg, 0.035 mmol) obtained in Example 9, dichloromethane (1.8 mL) and trifluoroacetic acid (0.35 mL, 4.6 mmol).
¹H NMR (CDCl₃, 300MHz)δ ppm: 9.67(br d, J=9.3Hz, 1H), 6.90(br s, 1H), 5.88(ddd, J=5.5, 10.5, 17.1Hz, 1H), 5.32(dd, J=1.3, 17.1Hz, 1H), 5.23(dd, J=1.3, 10.5Hz, 1H), 4.89(m, 1H), 4.68(d, J=4.6Hz, 1H), 4.62(d, J=4.8Hz, 2H), 4.18(m, 1H), 3.52(dd, J=4.6, 7.7Hz, 1H), 2.55(m, 1H), 2.47(m, 1H), 1.99(m, 1H), 1.65(m, 1H), 1.58(d, J=6.9Hz, 3H), 1.23-1.49(m, 2H), 1.07(d, J=6.7Hz, 3H), 0.95(t, J=7.5Hz, 3H), 0.89(m, 1H), 0.80(m, 1H), 0.62(m, 1H) FABMS m/z: 410(M+H)⁺ calculated for C₂₀H₃₁N₃O₆=409
HRFABMS calculated for C₂₀H₃₂N₃O₆ (M+H)⁺ 410.2291 found 410.2304

### Example 11: Synthesis of Compound 11

UCK14A₂ (50 mg, 0.11 mmol) was dissolved in dichloromethane (5.0 mL) followed by adding n-butyl alcohol (0.015 mL, 0.16 mmol), 1,3-dicyclohexylcarbodiimide (22 mg, 0.11 mmol), 1-hydroxybenzotriazole monohydrate (25 mg, 0.21 mmol) and 4-dimethylaminopyridine (2.6 mg, 0.021 mmol), and then the mixture was stirred at 25°C for 1.5 hours. After usual post-treatment, the crude product (31 mg) was obtained by purifying with a chromatography on silica gel (eluted with n-hexane/ethyl acetate=3/2), and further purified by a preparative HPLC (ODS column, eluted with acetonitrile/water=60/40) to afford Compound 11 (9.3 mg, yield; 9.8%).
¹H NMR (CDCl₃, 300MHz)δ ppm: 9.02(br d, J=8.2Hz, 1H), 6.61(br s, 1H), 5.40(br d, J=7.5Hz, 1H), 4.63(d, J=4.4Hz, 1H), 4.50(m, 1H), 4.40(m, 1H), 4.12(d, J=6.2Hz, 2H), 3.55 (m, 1H), 2.41-2.54 (m, 3H), 2.00 (m, 1H), 1.55-1.75 (m, 2H), 1.44(s, 9H), 1.42(d, J=7.4Hz, 3H), 1.23-1.40(m, 5H), 1.09(d, J=6.6Hz, 1H), 0.84-1.00(m, 8H), 0.61(m, 1H), 0.57(m, 1H)
FABMS m/z: 526(M+H)⁺ calculated for C₂₆H₄₃N₃O₈=525
HRFABMS calculated for C₂₆H₄₄N₃O₈ (M+H)⁺ 526.3128 found 526.3118

### Example 12: Synthesis of Compound 12

In a manner similar to that in Example 4, Compound 12 (27 mg, yield; 100%) was obtained from Compound 11 (25 mg, 0.048 mmol) obtained in Example 11, dichloromethane (2.5 mL) and trifluoroacetic acid (0.51 mL, 6.6 mmol).
¹H NMR (CDCl₃, 300MHz) δ ppm: 9.60(br d, J=9.5Hz, 1H), 6.86(br s, 1H), 4.86(m, 1H), 4.69(d, J=4.4Hz, 1H), 4.18(m, 1H), 4.12(t, J=6.6Hz, 2H), 3.53(dd, J=4.6, 7.7Hz, 1H), 2.55(m, 1H), 2.41(m, 1H), 1.99(m, 1H), 1.55-1.71(m, 5H), 1.24-1.44(m, 4H), 1.14(m, 1H), 0.95(t, J=7.5Hz, 3H), 0.92(t, J=7.5Hz, 3H), 0.67-0.84(m, 5H), 0.62(m, 1H)
FABMS m/z: 426(M+H)⁺ calculated for C₂₁H₃₅N₃O₆=425
HRFABMS calculated for C₂₁H₃₆N₃O₆ (M+H)⁺ 426.2605 found 426.2624

### Example 13: Synthesis of Compound 13

UCK14A₂ (30 mg, 0.064 mmol) was dissolved in dichloromethane (0.90 mL) and cyclohexane (1.8 mL) followed by adding tert-butyl 2,2,2-trichloroacetimidate (0.11 mL, 0.064 mmol) and boron trifluoride diethyl etherate (0.0079 mL, 0.064 mmol), and then the mixture was stirred at 25°C for 0.5 hour. After usual post-treatment, the crude product (20 mg) was obtained by purifying with a chromatography on silica gel (eluted with n-hexane/ethyl acetate=3/2), and further purified by a preparative HPLC (ODS column, eluted with acetonitrile/water =60/40) to afford Compound 13 ( 18 mg, yield; 54%).
¹H NMR (CDCl₃, 300MHz) δ ppm: 8.88(br d, J=8.8Hz, 1H), 6.63(br s, 1H), 5.42(br d, J=7.5Hz, 1H), 4.78(m, 1H), 4.63(d, J=4.6Hz, 1H), 4.35(m, 1H), 3.52(dd, J=4.6, 7.5Hz, 1H), 2,38-2.53(m, 2H), 2.00(m, 1H), 1.61-1.75(m, 2H), 1.45(s, 9H), 1.39-1.44(m, 12H), 1.33(m, 1H), 1.08(d, J=6.8Hz, 3H), 0.96(t, J=7.3Hz, 3H), 0.88(m, 1H), 0.77(m, 1H), 0.60(m, 1H)
FABMS m/z: 526(M+H)⁺ calculated for C₂₆H₄₃N₃O₈=525
HRFABMS calculated for C₂₆H₄₄N₃O₈ (M+H)⁺ 526.3128 found 526.3098

### Example 14: Synthesis of Compound 14

In a manner similar to that in Example 11, Compound 14 (15 mg, yield; 26%) was obtained from UCK14A₂ (47 mg, 0.099 mmol), dichloromethane (4.7 mL), cyclohexylmethanol (0.024 mL, 0.20 mmol), 1,3-dicyclohexylcarbodiimide (41 mg, 0.20 mmol), 1-hydroxybenzotriazole monohydrate (47 mg, 0.40 mmol) and 4-dimethylaminopyridine (4.8 mg, 0.040 mmol).
¹H NMR (CDCl₃, 300MHz) δ ppm: 9.02(d, J=9.0Hz, 1H), 6.63(br s, 1H), 5.40(br d, J=7.7Hz, 1H), 4.90(m, 1H), 4.62(d, J=4.6Hz, 1H), 4.38(m, 1H), 3.93(d, J=6.2Hz, 2H), 3.54(dd, J=4.6, 7.7Hz, 1H), 2.41-2.54(m, 2H), 1.98(m, 1H), 1.55-1.77(m, 7H), 1.39-1.45(m, 12H), 1.15-1.38(m, 7H), 1.08(d, J=6.6Hz, 3H), 0.96(t, J=7.3Hz, 3H), 0.88(m, 1H), 0.77(m, 1H), 0.61(m, 1H)
FABMS m/z: 566(M+H)⁺ calculated for C₂₉H₄₇N₃O₈=565
HRFABMS calculated for C₂₉H₄₈N₃O₈ (M+H)⁺ 566.3442 found 566.3453

### Example 15: Synthesis of Compound 15

In a manner similar to that in Example 11, Compound 15 (15 mg, yield; 25%) was obtained from UCK14A₂ (49mg, 0.11 mmol), dichloromethane (4.9mL), 2-(trimethylsilyl)ethanol (0.015mL, 0.11 mmol), 1,3-dicyclohexylcarbodiimide (22 mg, 0.11 mmol), 1-hydroxybenzotriazole monohydrate (25 mg, 0.21 mmol) and 4-dimethylaminopyridine (2.6 mg, 0.021 mmol).
¹H NMR (CDCl₃, 300MHz)δ ppm: 8.98(br d, J=9.0Hz, 1H), 6.62(br s, 1H), 5.39(br d, J=8.1Hz, 1H), 4.85(ddd, J=4.3, 4.5, 8.8Hz, 1H), 4.62(d, J=4.6Hz, 1H), 4.38(m, 1H), 4.12-4.37(m, 2H), 3.55(dd, J=4.6, 7.5Hz, 1H), 2.38-2.54(m, 2H), 1.99(m, 1H), 1.58-1.75(m, 2H), 1.38-1.47(m, 12H), 1.33(m, 1H), 1.08(d, J=6.8Hz, 3H), 0.98(t, J=8.7Hz, 2H), 0.95(t, J=7.5Hz, 3H), 0.71-0.86(m, 2H), 0.60(m, 1H), 0.04(s, 9H)
FABMS m/z: 570(M+H)⁺ calculated for C₂₇H₄₇N₃O₈Si=569
HRFABMS calculated for C₂₇H₄₈N₃O₈Si (M+H)⁺ 570.3211 found 570.3216

### Example 16: Synthesis of Compound 16

In a manner similar to that in Example 9, Compound 16 (24 mg, yield; 51%) was obtained from UCK14A₂ (43 mg, 0.091 mmol), N,N-dimethylformamide (4.3 mL), 2-iodoethanol (0.035 mL, 0.46 mmol) and potassium carbonate (25 mg, 0.18 mmol).
¹H NMR (CDCl₃, 300MHz) δ ppm: 9.02(br d, J=8.6Hz, 1H), 6.80(br s, 1H), 5.33(br d, J=7.8Hz, 1H), 4.96(ddd, J=4.0, 4.6, 8.6Hz, 1H), 4.63(d, J=4.4Hz, 1H), 4.40(ddd, J=2.9, 6.6, 11.4Hz, 1H), 4.32(m, 1H), 4.12(ddd, J=2.9, 5.5, 11.7Hz, 1H), 3.68-3.86(m, 2H), 3.62 (dd, J=4.4, 7.5Hz, 1H), 2.57(m, 1H), 2.48 (m, 1H), 1.99(m, 1H), 1.69(m, 1H), 1.39-1.48(m, 12H), 1.23-1.37(m, 2H), 1.09(d, J=6.8Hz, 3H), 0.96 (t, J=7.3Hz, 3H), 0.88(m, 1H), 0.71(m, 1H), 0.61(m, 1H)
FABMS m/z: 514(M+H)⁺ calculated for C₂₄H₃₉N₃O₉=513
HRFABMS calculated for C₂₄H₄₀N₃O₉ (M+H)⁺ 514.2764 found 514.2767

### Example 17: Synthesis of Compound 17

In a manner similar to that in Example 9, Compound 17 (15 mg, yield; 27%) was obtained from UCK14A₂ (51 mg, 0.11 mmol), N,N-dimethylformamide (5.1 mL), bromomethyl methyl ether (0.044 mL, 0.54 mmol and potassium carbonate (30 mg, 0.22 mmol). ¹H NMR (CDCl₃, 300MHz) δ ppm: 9.12(br d, J=8.8Hz, 1H), 6.65(br s, 1H), 5.38(br d, J=7.5Hz, 1H), 5.30(d, J=5.9Hz, 1H), 5.24(d, J=5.9Hz, 1H), 4.95(ddd, J=4.2, 4.6, 8.8Hz, 1H), 4.63(d, J=4.6Hz, 1H), 4.40(m, 1H), 3.55(dd, J=4.6, 7.5Hz, 1H), 3.37(s, 3H), 2.45-2.58(m, 2H), 1.99(m, 1H), 1.58-1.77(m, 2H), 1.40-1.48(m, 12H), 1.32(m, 1H), 1.07(d, J=6.7Hz, 3H), 0.95(t, J=7.3Hz, 3H), 0.74-0.85(m, 2H), 0.62(m, 1H)
FABMS m/z: 514(M+H)⁺ calculated for C₂₄H₃₉N₃O₉=513
HRFABMS calculated for C₂₄H₄₀N₃O₉ (M+H)⁺ 514.2765 found 514.2759

### Example 18: Synthesis of Compound 18

In a manner similar to that in Example 9, Compound 18 (28 mg, yield; 49%) was obtained from UCK14A₂ (46 mg, 0.098 mmol), N,N-dimethylformamide (4.6mL), 2-bromoacetophenone (98 mg, 0.49 mmol) and potassium carbonate (27 mg, 0.20 mmol). ¹H NMR (CDCl₃, 300MHz) δ ppm: 9.46(br d, J=9.3Hz, 1H), 7.87(m, 2H), 7.62(m, 1H), 7.50(m, 2H), 6.67(brs, 1H), 5.43(brd, J=8.4Hz, 1H), 5.38(s, 2H), 5.09(m, 1H), 4.62(d, J=4.5Hz, 1H), 4.47(m, 1H), 3.66(dd, J=4.6, 7.7Hz, 1H), 2.56-2.74(m, 2H), 1.89(m, 1H), 1.70(m, 1H), 1.55(m, 1H), 1.38-1.47(m, 12H), 1.19(m, 1H), 0.99(d, J=6.7Hz, 3H), 0.85-0.96(m, 2H), 0.79(d, J=7.3Hz, 3H), 0.65(m, 1H)
FABMS m/z: 588(M+H)⁺ calculated for C₃₀H₄₁N₃O₉=587

### Example 19: Synthesis of Compound 19

UCK14A₂ (52mg, 0.11 mmol) was dissolved in dichloromethane (5.2 mL) followed by adding benzylamine (0.012 mL, 0.11 mmol), 1,3-dicyclohexylcarbodiimide (23 mg, 0.11 mmol) and 1-hydroxybenzotriazole monohydrate(26 mg, 0.22 mmol), and then the mixture was stirred at 25°C for 0.5 hour. After usual post-treatment, the crude product (67 mg) was obtained by purifying with a chromatography on silica gel (eluted with n-hexane/ethyl acetate=1/3), and further purified by a preparative HPLC (ODS column, eluted with acetonitrile/water =50/50) to afford Compound 19 (31 mg, yield; 50%).
¹H NMR (CDCl₃, 300MHz) δ ppm: 9.14(br d, J=6.5Hz, 1H), 7.18-7.45(m, 5H), 6.90(m, 1H), 6.77(brs, 1H), 5.29(brd, J=6.7Hz, 1H), 4.83 (m, 1H), 4.56(d, J=4.5Hz, 1H), 4.50(dd, J=4.6, 14.9Hz, 1H), 4.36(dd, J=5.5, 15.0Hz, 1H), 4.31(m, 1H), 3.33(dd, J=4.6, 7.5Hz, 1H), 2.61(m, 1H), 2.41(m, 1H), 1.92(m, 1H), 1.80(m, 1H), 1.57(m, 1H), 1.42(s, 9H), 1.34(d, J=7.0Hz, 3H), 1.27(m, 1H), 1.01(d, J=6.8Hz, 3H), 0.90(d, J=7.3Hz, 3H), 0.87(m, 1H), 0.80(m, 1H), 0.60(m, 1H)
FABMS m/z: 559(M+H)⁺ calculated for C₂₉H₄₂N₄O₇=558
HRFABMS calculated for C₂₉H₄₃N₄O₇ (M+H)⁺ 559.3132 found 559.3149

### Example 20: Synthesis of Compound 20

In a manner similar to that in Example 4, Compound 20 (16 mg, yield; 85%)was obtained from Compound 19 (18 mg, 0.033 mmol) obtained in Example 19, dichloromethane (1.8 mL) and trifluoroacetic acid (0.36 mL, 4.7 mmol).
¹H NMR (CDCl₃, 300MHz) δ ppm: 9.51(br d, J=8.8Hz, 1H), 7.25-7.38(m, 6H), 7.02(br s, 1H), 4.74(m, 1H), 4.58(d, J=4.4Hz, 1H), 4.37-4.43(m, 2H), 4.17(m, 1H), 3.40(dd, J=4.6, 7.9Hz, 1H), 2.43(m, 1H), 2.28(m, 1H), 1.90(m, 1H), 1.44-1.63(m, 5H), 1.28(m, 1H), 0.98(d, J=6.8Hz, 3H), 0.90(m, 1H), 0.89(t, J=7.3Hz, 3H), 0.75(m, 1H), 0.56(m, 1H)
FABMS m/z: 459(M+H)⁺ calculated for C₂₄H₃₄N₄O₅=458

### Example 21: Synthesis of Compound 21

In a manner similar to that in Example 19, Compound 21 (38 mg, yield; 60%) was obtained from UCK14A₂ (49 mg, 0.11 mmol), dichloromethane (4.9 mL), 1-naphthalenemethylamine (0.015 mL, 0.11 mmol), 1,3-dicyclohexylcarbodiimide (22 mg, 0.11 mmol) and 1-hydroxybenzotriazole monohydrate (25 mg, 0.21 mmol).
IR (KBr): 3272, 3054, 2974, 2936, 1835, 1650, 1513, 1454, 1367, 1249, 1166, 1100, 1021, 914, 887, 778, 668 cm⁻¹
¹H NMR (CDCl₃, 300MHz) δ ppm: 9.07(br s, 1H), 7.97(m, 1H), 7.86(m, 1H), 7.80(m, 1H), 7.48(m, 2H), 7.40(m, 2H), 6.84(br s, 1H), 6.55(s, 1H), 5.25(br d, J=6.4Hz, 1H), 5.04(dd, J=6.5, 14.7Hz, 1H), 4.82(m, 1H), 4.74(dd, J=4.8, 14.5Hz, 1H), 4.36(d, J=4.6Hz, 1H), 4.26(m, 1H), 2.86(m, 1H), 2.63(m, 1H), 2.27(m, 1H), 1.88(m, 1H), 1.70(m, 1H), 1.40(s, 9H), 1.24-1.37(m, 2H), 1.20(d, J=7.0Hz, 3H), 0.90(d, J=6.6Hz, 3H), 0.78-0.85(m, 4H), 0.73(m, 1H), 0.55(m, 1H)
FABMS m/z: 609 (M+H)⁺ calculated for C₃₃H₄₄N₄O₇=608
HRFABMS calculated for C₃₃H₄₅N₄O₇ (M+H)⁺ 609.3288 found 609.3306

### Example 22: Synthesis of Compound 22

In a manner similar to that in Example 4, Compound 22 (25 mg, yield; 86%) was obtained from Compound 21 (28 mg, 0.046 mmol) obtained in Example 21, dichloromethane (2.8 mL) and trifluoroacetic acid (0.57 mL, 7.3 mmol).
¹H NMR (CDCl₃, 300MHz)δ ppm: 9.45(br d, J=8.8Hz, 1H), 7.76-7.90(m, 2H), 7.71(m, 1H), 7.37-7.47(m, 2H), 7.27-7.35(m, 2H), 7.23(m, 1H), 6.90(br s, 1H), 4.62-4.85(m, 3H), 4.41(d, J=4.2Hz, 1H), 4.13(m, 1H), 3.06(dd, J=4.4, 7.3Hz, 1H), 2.18-2.35(m, 2H), 1.77(m, 1H), 1.23-1.48(m, 5H), 1.11(m, 1H), 0.93(m, 1H), 0.88(d, J=6.8Hz, 3H), 0.80(t, J=7.3Hz, 3H), 0.66(m, 1H), 0.45(m, 1H)
FABMS m/z: 509(M+H)⁺ calculated for C₂₈H₃₆N₄O₅=508
HRFABMS calculated for C₂₈H₃₇N₄O₅ (M+H)⁺ 509.2764 found 509.2780

### Example 23: Synthesis of Compound 23

UCK14A₂ (30 mg, 0.063 mmol) was dissolved in dichloromethane (3.0 mL) followed by adding phenylhydrazine (0.0062 mL, 0.063 mmol), 1,3-dicyclohexylcarbodiimide (13 mg, 0.063 mmol) and 1-hydroxybenzotriazole monohydrate (15 mg, 0.13 mmol), and then the mixture was stirred at 25°C for 0.5 hour. After usual post-treatment, the crude product (25 mg) was obtained by purifying with a thin layer chromatography (developed with n-hexane/ethyl acetate=1/3), and further purified by a preparative HPLC (ODS column, eluted with acetonitrile/water=50/50) to afford Compound 23 (22 mg, yield; 62%).
¹H NMR (CDCl₃, 300MHz)δ ppm: 9.19(br d, J=7.5Hz, 1H), 8.52(br s, 1H), 7.14-7.23(m, 2H), 6.74-6.91(m, 4H), 6.16(br d, J=3.6Hz, 1H), 5.32(br d, J=7.1Hz, 1H), 4.90(m, 1H), 4.62(d, J=4.6Hz, 1H), 4.36(m, 1H), 3.55(dd, J=4.6, 7.9Hz, 1H), 2.44-2.58(m, 2H), 1.95(m, 1H), 1.84(m, 1H), 1.62(m, 1H), 1.40-1.46(m, 12H), 1.26(m, 1H), 1.03(d, J=6.6Hz, 3H), 0.90(t, J=7.3Hz, 3H), 0.89(m, 1H), 0.70(m, 1H), 0.59(m, 1H)
FABMS m/z: 560(M+H)⁺ calculated for C₂₈H₄₁N₅O₇=559
HRFABMS calculated for C₂₈H₄₂N₅O₇ (M+H)⁺ 560.3084 found 560.3094

### Example 24: Synthesis of Compound 24

In a manner similar to that in Example 19, Compound 24 (21 mg, yield; 89%) was obtained from UCK14A₂ (21 mg, 0.044 mmol), dichloromethane (2.1mL), n-butylamine (0.0044 mL, 0.044 mmol), 1,3-dicyclohexylcarbodiimide (9.1 mg, 0.044 mmol) and 1-hydroxybenzotriazole monohydrate (10 mg, 0.088 mmol).
¹H NMR (CDCl₃, 300MHz)δ ppm: 9.10(br d, J=7.1Hz, 1H), 6.81(br s, 1H), 6.48(m, 1H), 5.32(brd, J=6.2Hz, 1H), 4.75 (m, 1H), 4.62(d, J=4.4Hz, 1H), 4.33(m, 1H), 3.52(dd, J=4.4, 7.7Hz, 1H), 3.22(dd, J=7.0, 12.8Hz, 1H), 3.21(dd, J=6.6, 12.3Hz, 1H), 2.58(m, 1H), 2.42 (m, 1H), 2.00 (m, 1H), 1.39-1.55(m, 2H), 1.44(s, 9H), 1.41(d, J=7.0Hz, 3H), 1.23-1.39(m, 5H), 1.07(d, J=6.6Hz, 3H), 0.84-1.01(m, 7H), 0.80(m, 1H), 0.59(m, 1H)
FABMS m/z: 525(M+H)⁺ calculated for C₂₆H₄₄N₄O₇=524
HRFABMS calculated for C₂₆H₄₅N₄O₇ (M+H)⁺ 525.3288 found 525.3268

### Example 25: Synthesis of Compound 25

In a manner similar to that in Example 4, Compound 25 (23 mg, yield; 92%) was obtained from Compound 24 (24 mg, 0.046 mmol) obtained in Example 24, dichloromethane (2.4 mL) and trifluoroacetic acid (0.48 mL, 6.3 mmol).
¹H NMR (CD₃CN, 300MHz) δ ppm: 9.10(m, 1H), 7.50(s, 1H), 6.70(br s, 1H), 4.70(d, J=4.4Hz, 1H), 4.55(m, 1H), 4.10(m, 1H), 3.65(dd, J=4.4, 8.2Hz, 1H), 3.07-3.20(m, 3H), 2.49(m, 1H), 2.26(m, 1H), 1.85-1.98(m, 6H), 1.52(m, 2H), 1.23-1.46(m, 4H), 1.01(d, J=7.0Hz, 3H), 0.79-0.96(m, 6H), 0.54(m, 1H)
FABMS m/z: 425(M+H)⁺ calculated for C₂₁H₃₆N₄O₅=424
HRFABMS calculated for C₂₁H₃₇N₄O₅ (M+H)⁺ 425.2782 found 425.2764

### Example 26: Synthesis of Compound 26

UCK14A₂ (40 mg, 0.085 mmol) was dissolved in dichloromethane (4.0 mL) followed by adding morpholine (0.0074 mL, 0.085 mmol), 1,3-dicyclohexylcarbodiimide (18 mg, 0.085 mmol) and 1-hydroxybenzotriazole monohydrate (20 mg, 0.17 mmol), and then the mixture was stirred at 25°C for 2.5 hours. After usual post-treatment, purifying by a chromatography on silica gel (eluted with n-hexane/ethyl acetate=1/5 to 1/10) afforded Compound 26 (12 mg, yield; 26%).
¹H NMR (CDCl₃, 300MHz)δ ppm: 8.53(br d, J=7.1Hz, 1H), 6.65(br s, 1H), 5.33(br d, J=7.1Hz, 1H), 5.07(ddd, J=4.4, 4.6, 9.0Hz, 1H), 4.61(d, J=4.6Hz, 1H), 4.32 (m, 1H), 3.40-3.77 (m, 9H), 2.50(m, 1H), 1.90-2.09 (m, 2H), 1.61-1.82(m, 2H), 1.43(m, 9H), 1.38(d, J=7.0Hz, 3H), 1.31(m, 1H), 1.09(d, J=6.8Hz, 3H), 0.96(t, J=7.4Hz, 3H), 0.89(m, 1H), 0.80(m, 1H), 0.61(m, 1H)
FABMS m/z: 539(M+H)⁺ calculated for C₂₆H₄₂N₄O₈=538
HRFABMS calculated for C₂₆H₄₃N₄O₈ (M+H)⁺ 539.3081 found 539.3067

### Example 27: Synthesis of Compound 27

In a manner similar to that in Example 19, Compound 27 (20 mg, yield; 32%) was obtained from UCK14A₂ (46 mg, 0.098 mmol), dichloromethane (4.6 mL), L-alanine benzyl ester (21 mg, 0.12 mmol), 1,3-dicyclohexylcarbodiimide (24 mg, 0.12 mmol)
and 1-hydroxybenzotriazole monohydrate (27 mg, 0.23 mmol). ¹H NMR (CDCl₃, 300MHz) δ ppm: 9.00(br d, J=7.5Hz, 1H), 7.30-7.41(m, 5H), 7.20 (m, 1H), 6.67(brs, 1H), 5.30(brd, J=7.0Hz, 1H), 5.18(d, J=12.3Hz, 1H), 5.13(d, J=12.3Hz, 1H), 4.87(m, 1H), 4.61(d, J=4.6 Hz, 1H), 4.57(m, 1H), 4.34(m, 1H), 3.48(dd, J=4.6, 7.7Hz, 1H), 2.46-2.59(m, 2H), 1.98(m, 1H), 1.58-1.72(m, 2H), 1.40-1.48(m, 12H), 1.38(d, J=7.2Hz, 3H), 1.28(m, 1H), 1.05(d, J=6.8Hz, 3H), 0.98(m, 1H), 0.92(d, J=7.3Hz, 3H), 0.76(m, 1H), 0.56(m, 1H)
FABMS m/z: 631(M+H)⁺ calculated for C₃₂H₄₆N₄O₉=630
HRFABMS calculated for C₃₂H₄₇N₄O₉ (M+H)⁺ 631.3344 found 631.3358

### Example 28: Synthesis of Compound 28

Compound 4 (20 mg, 0.035 mmol) obtained in Example 4 was dissolved in tetrahydrofuran (1.0 mL) and water (1.0 mL) followed by adding benzyl chloroformate (20 mg, 0.14 mmol) and sodium hydrogencarbonate (12 mg, 0.14 mmol), and then the mixture was stirred at 0°C for one hour. After usual post-treatment, purifying by a chromatography on silica gel (eluted with n-hexane/ethyl acetate=1/1) afforded Compound 28 (20 mg, yield; 95%).
¹H NMR (CDCl₃, 300MHz) δ ppm: 9.13 (br d, J=9.4Hz, 1H), 7.23-7.42(m, 10H), 6.58(br s, 1H), 5.68(br d, J=7.7Hz, 1H), 5.16(s, 2H), 5.09(s, 2H), 4.97(m, 1H), 4.60(d, J=4.4Hz, 1H), 4.47(m, 1H), 3.45(dd, J=4.4, 7.5Hz, 1H), 2.42-2.55(m, 2H), 1.95(m, 1H), 1.54-1.72(m, 2H), 1.41(d, J=7.0Hz, 3H), 1.30(m, 1H), 1.04(d, J=6.8Hz, 3H), 0.94(t, J=7.3Hz, 3H), 0.89(m, 1H), 0.72(m, 1H), 0.57(m, 1H)
FABMS m/z: 594(M+H)⁺ calculated for C₃₂H₃₉N₃O₈=593

### Example 29: Synthesis of Compound 29

Compound 4 (40 mg, 0.070 mmol) obtained in Example 4 was dissolved in tetrahydrofuran (2.0 mL) and water (2.0 mL) followed by adding 4-bromobenzyl chloroformate (120 mg, 0.35 mmol) and sodium hydrogencarbonate (29mg, 0.35 mmol), and then the mixture was stirred at 0°C for one hour. After usual post-treatment, purifying by a chromatography on silica gel (eluted with n-hexane/ethyl acetate=1/1) afforded Compound 29 (39 mg, yield; 83%).
¹H NMR (CDCl₃, 300MHz) δ ppm: 9.13(br d, J=9.2Hz, 1H), 7.43-7.49 (m, 2H), 7.29-7.37(m, 5H), 7.17=7.24(m, 2H), 6.59(br s, 1H), 5.72(br d, J=7.7Hz, 1H), 5.15(s, 2H), 5.02(s, 2H), 4.96(m, 1H), 4.60(d, J=4.4Hz, 1H), 4.44(m, 1H), 3.45(dd, J=4.5, 7.5Hz, 1H), 2.42-2.55(m, 2H), 1.96(m, 1H), 1.70(m, 1H), 1.62(m, 1H), 1.40(d, J=7.0Hz, 3H), 1.30(m, 1H), 1.04(d, J=6.8Hz, 3H), 0.93(t, J=7.3Hz, 3H), 0.93(m, 1H), 0.53-0.68(m, 2H)
FABMS m/z: 672(M+H)⁺ calculated for C₃₂H₃₈N₃O₈⁷⁹Br=671

### Example 30: Synthesis of Compound 30

Compound 4 (20 mg, 0.035 mmol) obtained in Example 4 was dissolved in N,N-dimethylformamide (2.0 mL) followed by adding benzyl isocyanate (0.017 mL, 0.14 mmol) and triethylamine (9.7 mL, 0.070 mmol), and then the mixture was stirred at 0°C for 0.5 hour. After usual post-treatment, purifying by a chromatography on silica gel (eluted with n-hexane/ethyl acetate=1/3) afforded Compound 30 (19 mg, yield; 92%).
¹H NMR (CDCl₃, 300MHz) δ ppm: 9.12(br d, J=9.0Hz, 1H), 7.14-7.40(m, 10H), 6.65(brs, 1H), 5.98 (m, 1H), 5.55(m, 1H), 5.15(d, J=12.3Hz, 1H), 5.09(d, J=12.3Hz, 1H), 4.74(m, 1H), 4.61(m, 1H), 4.58(d, J=4.6Hz, 1H), 4.32(d, J=5.5Hz, 2H), 3.46(dd, J=4.4, 7.5Hz, 1H), 2.49(m, 1H), 2.29(m, 1H), 1.94(m, 1H), 1.61(m, 1H), 1.38(d, J=7.0Hz, 3H), 1.19-1.35(m, 2H), 1.03(d, J=6.8Hz, 3H), 0.93(t, J=7.4Hz, 3H), 0.90(m, 1H), 0.69(m, 1H), 0.50(m, 1H)
FABMS m/z: 593(M+H)⁺ calculated for C₃₂H₄₀N₄O₇=592

### Example 31: Synthesis of Compound 31

In a manner similar to that in Example 30, Compound 31 (16 mg, yield; 86%) was obtained from Compound 4 (20 mg, 0.035 mmol) obtained in Example 4, N,N-dimethylformamide (2.0 mL), isopropyl isocyanate (0.014 mL, 0.14 mmol) and triethylamine (0.0097 mL, 0.070 mmol).
¹H NMR (CDCl₃, 300MHz) δ ppm: 9.00(br d, J=7.9Hz, 1H), 7.33(s, 5H), 6.73(br s, 1H), 5.51(m, 1H), 5.15(s, 2H), 4.92(ddd, J=4.2, 4.6, 8.8Hz, 1H), 4.62(d, J=4.6Hz, 1H), 4.57(dq, J=7.2, 7.2Hz, 1H), 4.25(m, 1H), 3.81(dq, J=6.2, 12.8Hz, 1H), 3.51(dd, J=4.6, 7.0Hz, 1H), 2.55(m, 1H), 2.40(m, 1H), 1.98(m, 1H), 1.83(m, 1H), 1.64(m, 1H), 1.36(d, J=7.0Hz, 3H), 1.30(m, 1H), 1.02-1.15(m, 9H), 0.95(t, J=7.5Hz, 3H), 0.89(m, 1H), 0.74(m, 1H), 0.59(m, 1H)
FABMS m/z: 545(M+H)⁺ calculated for C₂₈H₄₀N₄O₇=544

### Example 32: Synthesis of Compound 32

In a manner similar to that in Example 30, Compound 32 (16 mg, yield; 75%) was obtained from Compound 4 (20 mg, 0.035 mmol) obtained in Example 4, N,N-dimethylformamide (2.0 mL), benzyl isothiocyanate (0.019 mL, 0.14 mmol) and triethylamine (0.0097 mL, 0.070 mmol).
¹H NMR (CDCl₃, 300MHz)δ ppm: 9.57(br d, J=9.2Hz, 1H), 7.63(br s, 1H), 7.29-7.38 (m, 10H), 6.58(br s, 1H), 6.55(br s, 1H), 5.13(s, 2H), 5.07(m, 1H), 4.74(m, 1H), 4.62(d, J=4.6Hz, 1H), 4.51(d, J=14.7Hz, 1H), 4.49(d, J=14.8Hz, 1H), 3.48(dd, J=4.6, 7.5Hz, 1H), 2.53(m, 1H), 2.29(m, 1H), 1.96(m, 1H), 1.55-1.75(m, 2H), 1.43(d, J=7.0Hz, 3H), 1.30(m, 1H), 1.05(d, J=6.8Hz, 3H), 0.94(t, J=7.3Hz, 3H), 0.55-0.78(m, 2H), 0.48(m, 1H)
FABMS m/z: 609(M+H)⁺ calculated for C₃₂H₄₀N₄O₆S=608

### Example 33: Synthesis of Compound 33

In a manner similar to that in Example 30, Compound 33 (20 mg, quantitative) was obtained from Compound 4 (20 mg, 0.034 mmol) obtained in Example 4, N,N-dimethylformamide (2.0 mL), benzoic anhydride (31 mg, 0.14 mmol) and triethylamine (0.0095 mL, 0.068 mmol).
¹H NMR (CDCl₃, 300MHz)δ ppm: 9.35(br d, J=9.3Hz, 1H), 7.30-7.52(m, 8H), 7.28-7.36(m, 2H), 7.18(br d, J=7.4Hz, 1H), 6.70(br s, 1H), 5.18(s, 1H), 5.00(m, 1H), 4.88(m, 1H), 4.63(d, J=4.4Hz, 1H), 3.48(dd, J=4.5, 7.5Hz, 1H), 2.47-2.58(m, 2H), 1.97(m, 1H), 1.63(m, 1H), 1.52(d, J=6.9Hz, 3H), 1.22-1.38(m, 2H), 1.06(d, J=6.8Hz, 3H), 0.95(t, J=7.5Hz, 3H), 0.89(m, 1H), 0.75(m, 1H), 0.59(m, 1H)
FABMS m/z: 564(M+H)⁺ calculated for C₃₁H₃₇N₃O₇=563

### Example 34: Synthesis of Compound 34

Compound 4 (20 mg, 0.035 mmol) obtained in Example 4 was dissolved in formic acid (2.0 mL) followed by adding acetic anhydride (0.67 mL, 7.1 mmol), and then the mixture was stirred at 40°C for 2 hours. After usual post-treatment, purifying by a chromatography on silica gel (eluted with n-hexane/ethyl acetate=1/5) afforded Compound 34 (15 mg, yield; 88%).
¹H NMR (CDCl₃, 300MHz) δ ppm: 9.22(br d, J=9.4Hz, 1H), 8.26(s, 1H), 7.34(s, 5H), 6.60-6.69(m, 2H), 5.16(s, 2H), 4.97(ddd, J=4.4, 4.6, 9.0Hz, 1H), 4.74(dq, J=7.0, 7.0Hz, 1H), 4.64(d, J=4.6Hz, 1H), 3.37(dd, J=4.4, 7.5Hz, 1H), 2.45-2.56(m, 2H), 1.98(m, 1H), 1.72(m, 1H), 1.63(m, 1H), 1.44(d, J=6.9Hz, 3H), 1.35(m, 1H), 1.07(d, J=6.8Hz, 3H), 0.96(t, J=7.4Hz, 3H), 0.88(m, 1H), 0.77(m, 1H), 0.60(m, 1H)
FABMS m/z: 488(M+H)⁺ calculated for C₂₅H₃₃N₃O₇=487

### Example 35: Synthesis of Compound 35

In a manner similar to that in Example 30, Compound 35 (18 mg, quantitative) was obtained from Compound 4 (20 mg, 0.035 mmol) obtained in Example 4, N,N-dimethylformamide (2.0 mL), acetic anhydride (0.013 mL, 0.14 mmol) and triethylamine (0.0098 mL, 0.070 mmol).
¹H NMR (CDCl₃, 300MHz) δ ppm: 9.14(br d, J=9.2Hz, 1H), 7.34(m, 5H), 6.68(br s, 1H), 6.47(br d, J=7.2Hz, 1H), 5.16(s, 2H), 4.97(m, 1H), 4.63(m, 1H), 4.62(d, J=4.4Hz, 1H), 3.48(dd, J=4.4, 7.3Hz, 1H), 2.45-2.54(m, 2H), 1.98(s, 3H), 1.96(m, 1H), 1.63(m, 1H), 1.40(d, J=7.0Hz, 3H), 1.23-1.48(m, 2H), 1.07(d, J=6.8Hz, 3H), 0.95(t, J=7.3Hz, 3H), 0.88(m, 1H), 0.74(m, 1H), 0.59(m, 1H)
FABMS m/z: 502(M+H)⁺ calculated for C₂₆H₃₅N₃O₇=501

### Example 36: Synthesis of Compound 36

Compound 4 (25 mg, 0.044 mmol) obtained in Example 4 was dissolved in N,N-dimethylformamide (2.5 mL) followed by adding 2-pyrazinecarboxylic acid (11 mg, 0.087 mmol), 1-ethyl-3-(3-dimethylaminopropyl)carbodiimide hydrochloride (17 mg, 0.087 mmol) and 1-hydroxybenzotriazole monohydrate (20 mg, 0.17 mmol), and then the mixture was stirred at 0°C for 0.5 hour. After usual post-treatment, purifying by a chromatography on silica gel (eluted with n-hexane/ethyl acetate=1/3) afforded Compound 36 (18 mg, yield; 71%).
¹H NMR (CDCl₃, 300MHz) δ ppm: 9.38(br d, J=9.5Hz, 1H), 9.35(d, J=1.2Hz, 1H), 8.73(d, J=2.6Hz, 1H), 8.55(dd, J=1.6, 2.5Hz, 1H), 8.51(br d, J=7.7Hz, 1H), 7.35(s, 5H), 6.64(br s, 1H), 5.80(s, 2H), 5.01(m, 1H), 4.88(m, 1H), 4.65(d, J=4.6Hz, 1H), 3.48(dd, J=4.6, 7.6Hz, 1H), 2.48-2.57(m, 2H), 1.99(m, 1H), 1.59-1.73(m, 2H), 1.55(d, J=7.0Hz, 3H), 1.34(m, 1H), 1.08(d, J=6.6Hz, 3H), 0.96(t, J=7.4Hz, 3H), 0.88(m, 1H), 0.76(m, 1H), 0.60(m, 1H)
FABMS m/z: 565(M+H)⁺ calculated for C₂₉H₃₅N₅O₇=564

### Example 37: Synthesis of Compound 37

In a manner similar to that in Example 36, Compound 37 (16 mg, yield; 56%) was obtained from Compound 4 (25 mg, 0.044 mmol) obtained in Example 4, N,N-dimethylformamide (2.5 mL), 3-tert-butyldimethylsilyloxypropionic acid (11 mg, 0.087 mmol), 1-ethyl-3-(3-dimethylaminopropyl)carbodiimide hydrochloride (17 mg, 0.087 mmol) and 1-hydroxybenzotriazole monohydrate (20 mg, 0.17 mmol).
¹H NMR (CDCl₃, 300MHz) δ ppm: 9.27(br d, J=9.5Hz, 1H), 7.47(br d, J=7.7Hz, 1H), 7.33(m, 5H), 6.61(brs, 1H), 5.15(s, 2H), 5.01(m, 1H), 4.66(m, 1H), 4.64(d, J=4.4Hz, 1H), 4.07(s, 2H), 3.56(dd, J=4.6, 7.5Hz, 1H), 2.45-2.57(m, 2H), 1.97(m, 1H), 1.55-1.75(m, 2H), 1.45(d, J=6.9Hz, 3H), 1.30(m, 1H), 1.07(d, J=6.8Hz, 3H), 0.91-0.98(m, 12H), 0.73(m, 1H), 0.65(m, 1H), 0.58(m, 1H), 0.04(s, 6H)
FABMS m/z: 632(M+H)⁺ calculated for C₃₂H₄₉N₃O₈Si=631

### Example 38: Synthesis of Compound 38

Compound 37 (24 mg, 0.038 mmol) obtained in Example 37 was dissolved in tetrahydrofuran (2.4 mL) followed by adding a 1 mol/L tetrabutylammonium fluororide/tetrahydrofuran solution (0.075 mL, 0.075 mmol) and acetic acid (4.3 mg, 0.075 mmol), and then the mixture was stirred at 25°C for 2 hours. After usual post-treatment, the crude product (15 mg) was obtained by purifying with a chromatography on silica gel (eluted with chloroform/ethanol=15/1), and further purified by a preparative HPLC (ODS column, eluted with acetonitrile/water=40/60) to afford Compound 38 (9.9mg, yield; 49%).
¹H NMR (CDCl₃, 300MHz) δ ppm: 9.02(brd, J=8.3Hz, 1H), 7.29-7.40(m, 5H), 7.13(br d, J=6.7Hz, 1H), 6.66(br s, 1H), 5.19(d, J=11.9Hz, 1H), 5.13(d, J=12.3Hz, 1H), 4.95(ddd, J=4.2, 4.2, 8.4Hz, 1H), 4.66(m, 1H), 4.62(d, J=4.4Hz, 1H), 4.01-4.14(m, 2H), 3.55(br s, 1H), 3.49(dd, J=4.4, 7.4Hz, 1H), 2.42-2.58(m, 2H), 1.98(m, 1H), 1.53-1.72(m, 2H), 1.44(d, J=7.0Hz, 3H), 1.31 (m, 1H), 1.07(d, J=6.8Hz, 3H), 0.96(t, J=7.3Hz, 3H), 0.75(m, 1H), 0.56-0.70(m, 1H)
FABMS m/z: 518(M+H)⁺ calculated for C₂₆H₃₅N₃O₈=517

### Example 39: Synthesis of Compound 39

In a manner similar to that in Example 30, Compound 39 (17 mg, yield; 83%) was obtained from Compound 4 (20 mg, 0.035 mmol) obtained in Example 4, N,N-dimethylformamide (2.0 mL), benzenesulfonyl chloride (0.018 mL, 0.14 mmol) and triethylamine (0.0096 mL, 0.069 mmol).
¹H NMR (CDCl₃, 300MHz)δ ppm: 8.96(br d, J=9.1Hz, 1H), 7.82-7.88(m, 2H), 7.38-7.55(m, 3H), 7.24-7.36(m, 5H), 6.59(brs, 1H), 5.80(br d, J=9.0Hz, 1H), 5.50-5.68(m, 2H), 4.70(m, 1H), 4.62(d, J=4.6Hz, 1H), 4.09 (m, 1H), 3.47(dd, J=4.4, 7.6Hz, 1H), 2.39 (m, 1H), 2.22 (m, 1H), 1.99(m, 1H), 1.56-1.72 (m, 2H), 1.34(d, J=6.9Hz, 3H), 1.28(m, 1H), 1.07(d, J=6.8Hz, 3H), 0.95(t, J=7.5Hz, 3H), 0.89(m, 1H), 0.45-0.58(m, 2H)
FABMS m/z: 600(M+H)⁺ calculated for C₃₀H₃₇N₃O₈S=599

### Example 40: Synthesis of Compound 40

In a manner similar to that in Example 30, Compound 40 (13 mg, yield; 95%) was obtained from Compound 4 (15 mg, 0.026 mmol) obtained in Example 4, N,N-dimethylformamide (1.5 mL), methanesulfonyl chloride (0.0081 mL, 0.10 mmol) and triethylamine (0.0073 mL, 0.052 mmol).
¹H NMR (CDCl₃, 300MHz)δ ppm: 9.18(d, J=9.3Hz, 1H), 7.34(m, 5H), 6.68(br s, 1H), 5.51(br d, J=12.3Hz, 1H), 5.17(m, 2H), 4.97(m, 1H), 4.63(d, J=4.6Hz, 1H), 4.26(m, 1H), 3.48(dd, J=4.6, 7.5Hz, 1H), 2.92(s, 3H), 2.44-2.60(m, 2H), 1.99(m, 1H), 1.57-1.72(m, 2H), 1.45(d, J=7.2Hz, 3H), 1.33(m, 1H), 1.09(d, J=6.8Hz, 3H), 0.96(t, J=7.6Hz, 3H), 0.78(m, 1H), 0.54-0.66(m, 2H)
FABMS m/z: 538(M+H)⁺ calculated for C₂₅H₃₅N₃O₈S=537

### Example 41: Synthesis of Compound 41

In a manner similar to that in Example 30, Compound 41 (7.1 mg, yield; 42%) was obtained from Compound 4 (15 mg, 0.026 mmol) obtained in Example 4, N,N-dimethylformamide (1.5 mL), diphenylphosphinic chloride (0.0985 mL, 0.052 mmol) and triethylamine (0.014 mL, 0.10 mmol).
¹H NMR (CDCl₃, 300MHz) δ ppm: 8.97(br d, J=8.9Hz, 1H), 7.80-7.96(m, 4H), 7.39-7.55(m, 11H), 6.71(br s, 1H), 5.16(s, 2H), 4.92(ddd, J=4.0, 4.6, 8.6Hz, 1H), 4.33 (d, J=4.4Hz, 1H), 3.86-4.04 (m, 2H), 3.34(dd, J=4.6, 7.3Hz, 1H), 2.37-2.48(m, 2H), 1.86(m, 1H), 1.51(m, 1H), 1.44(d, J=6.4Hz, 3H), 1.17-1.30(m, 2H), 0.94(d, J=6.8Hz, 3H), 0.89(t, J=7.4Hz, 3H), 0.66-0.74(m, 2H), 0.58(m, 1H)
FABMS m/z: 660 (M+H)⁺ calculated for C₃₆H₄₂N₃O₇=659

### Example 42: Synthesis of Compound 42

In a manner similar to that in Example 28, Compound 42 (22 mg, yield; 100%) was obtained from Compound 4 (20 mg, 0.035 mmol) obtained in Example 4, tetrahydrofuran (1.0 mL), water (1.0 mL), N-carbethoxyphthalimide (45 mg, 0.21 mmol) and sodium hydrogencarbonate (17 mg, 0.21 mmol).
IR(KBr): 3254, 2930, 1836, 1773, 1714, 1660, 1535, 1456, 1386, 1194, 1153, 1088, 1019, 913, 883, 722, 698, 669 cm⁻¹
¹H NMR (CDCl₃, 300MHz)δppm: 8.95(br d, J=9.4Hz, 1H), 7.82-7.89 (m, 2H), 7.70-7.77 (m, 2H), 7.28-7.39 (m, 5H), 6.37(brs, 1H), 5.16(m, 1H), 5.12(d, J=2.2Hz, 2H), 5.07(q, J=2.2Hz, 1H), 3.69(d, J=4.6Hz, 1H), 3.25(dd, J=4.6, 7.6Hz, 1H), 2.50 (m, 1H), 2.32 (m, 1H), 1.82 (m, 1H), 1.72(d, J=7.9Hz, 3H), 1.45(m, 1H), 1.14-1.34(m, 2H), 0.91(d, J=6.8Hz, 3H), 0.89(m, 1H), 0.88(t, J=7.6Hz, 3H), 0.50(m, 2H)
FABMS m/z: 590(M+H)⁺ calculated for C₃₂H₃₅N₃O₈=589

### Example 43: Synthesis of Compound 43

In a manner similar to that in Example 8, Compound 43 (7.2 mg, yield; 51%) was obtained from Compound 4 (13 mg, 0.022 mmol) obtained in Example 4, N,N-dimethylformamide (1.3 mL), benzyl bromide (0.010 mL, 0.088 mmol) and potassium carbonate (6.1 mg, 0.044 mmol).
¹H NMR (CDCl₃, 300MHz)δppm: 8.44(br d, J=8.1Hz, 1H), 7.20-7.44(m, 15H), 6.45(br s, 1H), 5.22(d, J=12.3Hz, 1H), 5.17(d, J=12.3Hz, 1H), 4.75(m, 2H), 4.42(d, J=4.4Hz, 1H), 3.78(d, J=14.0Hz, 2H), 3.55(d, J=14.1Hz, 2H), 3.45(dd, J=4.2, 7.3Hz, 1H), 2.36(m, 1H), 1.93(m, 1H), 1.74(m, 1H), 1.54-1.68(m, 2H), 1.32(d, J=7.1Hz, 3H), 1.27(m, 1H), 1.02(d, J=6.7Hz, 3H), 0.92(t, J=7.3Hz, 3H), 0.54-0.66(m, 3H)
FABMS m/z: 640(M+H)⁺ calculated for C₃₈H₄₅N₃O₆=639

### Example 44: Synthesis of Compound 44

Compound 4 (31 mg, 0.055 mmol) obtained in Example 4 was dissolved in dimethyl sulfoxide (0.31 mL) followed by adding a 0.04 mol/L phosphate buffer (pH=7.2)(2.8 mL), and then the mixture was stirred at 37°C for one day. After adding water to a reaction mixture, the crude product (15 mg) was obtained by purifying with an ODS column chromatography (eluted with water/acetonitrile =100/0 to 0/100), and further purified by a preparative HPLC [ODS column, eluted with acetonitrile/a 0.04 mol/L phosphate buffer (pH=6.5)=20/80] to afford Compound 44 (2.4 mg, yield; 12%).
¹H NMR (CD₃CN, 300MHz)δ ppm: 7.58 (m, 1H), 7.22(br s, 1H), 6.49(br s, 1H), 4.63(d, J=4.4Hz, 1H), 4.03(dt, J=4.6, 10.3Hz, 1H), 3.95(ddd, J=2.6, 7.0, 14.1Hz, 1H), 3.65(dd, J=4.4, 7.8Hz, 1H), 2.40(m, 1H), 1.90-2.08(m, 3H), 1.40(d, J=7.1Hz, 3H), 1.01(d, J=6.8Hz, 3H), 0.84-0.96(m, 5H), 0.80(m, 2H), 0.72(m, 1H)
FABMS m/z: 352(M+H)⁺ calculated for C₁₇H₂₅N₃O₅=351

### Example 45: Synthesis of Compound 45

In a manner similar to that in Example 9, Compound 45 (18 mg, yield; 74%) was obtained from Compound G (10 mg, 0.022 mmol) obtained in Reference Example 7, N,N-dimethylformamide (1.0 mL), benzyl bromide (0.012 mL, 0.098 mmol) and potassium carbonate (6.3 mg, 0.046 mmol).
¹H NMR (CDCl₃, 300MHz) δ ppm: 7.35(m, 5H), 6.71(br d, J=7.7Hz, 1H), 6.54(m, 1H), 5.18(dd, J=12.2, 18.7Hz, 2H), 5.10(m, 1H), 4.61(m, 1H), 4.57(d, J=4.6Hz, 1H), 4.17(m, 1H), 3.59(dd, J=4.6, 7.7Hz, 1H), 3.17-3.40(m, 2H), 1.84-2.06(m, 3H), 1.48-1.77(m, 2H), 1.43(s, 9H), 1.34(d, J=7.0Hz, 3H), 1.27(m, 2H), 1.08(d, J=6.6Hz, 3H), 0.95(d, J=7.5Hz, 3H)
FABMS m/z: 548(M+H)⁺ calculated for C₂₈H₄₁N₃O₈=547
HRFABMS calculated for C₂₈H₄₂N₃O₈ (M+H)⁺ 548.2972 found 548.2993

### Example 46: Synthesis of Compound 46

In a manner similar to that in Example 4, Compound 46 (9.0 mg, yield; 100%) was obtained from Compound 45 (9.2 mg, 0.017 mmol) obtained in Example 45, dichloromethane (0.82 mL) and trifluoroacetic acid (0.18 mL, 2.4 mmol).
¹H NMR (DMSO-d₆, 300MHz) δ ppm: 8.71(br d, J=5.7Hz, 1H), 8.47(m, 1H), 7.93(brs, 2H), 7.37(m, 5H), 5.12(s, 2H), 4.77(d, J=4.2Hz, 1H), 4.48(m, 1H), 3.73(m, 1H), 3.62(dd, J=4.2, 7.7Hz, 1H), 3.08-3.43(m, 2H), 1.91(m, 1H), 1.78(m, 1H), 1.65(m, 1H), 1.52(m, 2H), 1.30(d, J=7.2Hz, 3H), 1.24(m, 2H), 0.93(d, J=6.7Hz, 3H), 0.85(d, J=7.3Hz, 3H)
FABMS m/z: 448(M+H)⁺ calculated for C₂₃H₃₃N₃O₆=447
HRFABMS calculated for C₂₃H₃₄N₃O₆ (M+H)⁺ 448.2448 found 448.2438

### Example 47: Synthesis of Compound 47

Compound 71 (23 mg, 0.14 mmol) obtained in Example 71 was dissolved in dichloromethane (2.3 mL) followed by adding benzyl N-α-(tert-butyloxycarbonyl)-L-α,β-diaminopropionate (40 mg, 0.14 mmol), 1,3-dicyclohexylcarbodiimide (28 mg, 0.14 mmol) and 1-hydroxybenzotriazolemonohydrate (32 mg, 0.27 mmol), and then the mixture was stirred at 0°C for 0.5 hour. After usual post-treatment, purifying by a chromatography on silica gel (elutedwithn-hexane/ethyl acetate=3/1) afforded Compound 47(30 mg, yield; 50%).
¹H NMR (CDCl₃, 300MHz)δ ppm: 7.32-7.41(m, 5H), 6.81(m, 1H), 5.43(br d, J=6.2Hz, 1H), 5.19(s, 2H), 4.52(m, 1H), 4.48(d, J=4.4Hz, 1H), 3.72-3.87(m, 2H), 3.53(m, 1H), 1.92-1.70(m, 3H), 1.42(s, 9H), 0.99(d, J=6.5Hz, 3H), 0.95(d, J=6.0Hz, 3H)
FABMS m/z: 449(M+H)⁺ calculated for C₂₃H₃₂N₂O₇=448
HRFABMS calculated for C₂₃H₃₃N₂O₇ (M+H)⁺ 449.2288 found 449.2291

### Example 48: Synthesis of Compound 48

Compound 71 (16 mg, 0.095 mmol) obtained in Example 71 was dissolved in dichloromethane (1.6 mL) followed by adding N-a-(tert-butyloxycarbonyl)-L-lysine benzyl ester (32 mg, 0.095 mmol), 1,3-cyclohexylcarbodiimide (20 mg, 0.095 mmol) and 1-hydroxybenzotriazole monohydrate (22 mg, 0.19 mmol), and then the mixture was stirred at 0°C for 0.5 hour. After usual post-treatment, the crude product (24 mg) was obtained by purifying with a chromatography on silica gel (eluted with n-hexane/ethyl acetate=3/1), and further purified by a preparative HPLC (ODS column, eluted with acetonitrile/water =55/45) to afford Compound 48 (6.1 mg, yield; 34%).
¹H NMR (CDCl₃, 300MHz) δ ppm: 7.33-7.39(m, 5H), 6.34(m, 1H), 5.21(d, J=12.1Hz, 1H), 5.13(d, J=12.1Hz, 1H), 5.05(br d, J=7.6Hz, 1H), 4.52(d, J=4.4Hz, 1H), 4.33(m, 1H), 3.68(ddd, J=4.4, 7.4, 9.0Hz, 1H), 3.31(m, 1H), 3.23(m, 1H), 1.74-1.93(m, 4H), 1.43(s, 9H), 1.23-1.73(m, 5H), 1.00(d, J=6.3Hz, 3H), 0.97(d, J=6.2Hz, 3H)
FABMS m/z: 491(M+H)⁺ calculated for C₂₆H₃₈N₂O₇=490
HRFABMS calculated for C₂₆H₃₉N₂O₇ (M+H)⁺ 491.2757 found 491.2745

### Example 49: Synthesis of Compound 49

In a manner similar to that in Example 48, Compound 49 (24 mg, yield; 52%) was obtained from Compound 71 (14 mg, 0.080 mmol) obtained in Example 71, dichloromethane (1.4 mL), N-(tert-butyloxycarbonyl)-L-alanyl-L-lysine benzyl ester (33 mg, 0.080 mg), 1,3-dichlorohexylcarbodiimide (42 mg, 0.20 mmol) and 1-hydroxybenzotriazole monohydrate (19 mg, 0.17 mmol). ¹H NMR (CDCl₃, 300MHz)δ ppm: 7.32-7.38 (m, 5H), 6.77(br d, J=6.8Hz, 1H), 6.59(br s, 1H), 5.20(d, J=12.1Hz, 1H), 5.20(d, J=12.3Hz, 1H), 5.13(m, 1H), 4.60(d, J=4.1Hz, 1H), 4.58(m, 1H), 4.27(m, 1H), 3.70(ddd, J=4.3, 7.0, 8.8Hz, 1H), 3.35(m, 1H), 3.18(m, 1H), 1.46-1.94 (m, 9H), 1.43(s, 9H), 1.34 (d, J=6.9Hz, 3H), 0.99 (d, J=6.2Hz, 3H), 0.96(d, J=6.3Hz, 3H)
FABMS m/z: 562 (M+H)⁺ calculated for C₂₉H₄₃N₃O₈=561
HRFABMS calculated for C₂₉H₄₄N₃O₈ (M+H)⁺ 562.3128 found 562.3133

### Example 50: Synthesis of Compound 50

In a manner similar to that in Example 4, Compound 50 (8.5mg, yield; 70%) was obtained from Compound 49 (12 mg, 0.021 mmol) obtained in Example 49, dichloromethane (1.2 mL) and trifluoroacetic acid (0.24 mL, 3.1 mmol).
¹H NMR(CDCl₃, 300MHz)δ ppm: 7.90(br d, J=5.6Hz, 1H), 7.27-7.39(m, 5H), 7.12(m, 1H), 5.18(d, J=12.1Hz, 1H), 5.09(d, J=12.1Hz, 1H), 4.58(d, J=4.3Hz, 1H),4.50(m, 1H), 4.18(m, 1H), 3.70(ddd, J=4.2, 6.8, 8.6Hz, 1H), 3.18-3.39(m, 2H), 1.66-1.94(m, 6H), 1.41-1.59(m, 4H), 1.24-1.39(m, 2H), 0.96(d, J=6.3Hz, 3H), 0.93(d, J=6.1Hz, 3H)
FABMS m/z: 462(M+H)⁺ calculated for C₂₄H₃₅N₃O₆=461
HRFABMS calculated for C₂₄H₃₆N₃O₆ (M+H)⁺ 462.2604 found 462.2603

### Example 51: Synthesis of Compound 51

Compound 71 (36 mg, 0.21 mmol) obtained in Example 71 was dissolved in dichloromethane (3.6 mL) followed by adding N-α-(tert-butyloxycarbonyl)-α,ε-diaminopentane (51 mg, 0.25 mmol), 1,3-dicyclohexylcarbodiimide (52 mg, 0.25 mmol) and 1-hydroxybenzotriazole monohydrate (59mg, 0.50 mmol), and then the mixture was stirred at 0°C for 15 minutes. After usual post-treatment, purifying by a chromatography on silica gel (eluted with n-hexane/ethyl acetate=2/1) afforded Compound 51 (25 mg, yield; 34%).
FABMS m/z: 357(M+H)⁺ calculated for C₁₈H₃₂N₂O₅=356

### Example 52: Synthesis of Compound 52

Compound 51 (25 mg, 0.070 mmol) obtained in Example 51 was dissolved in dichloromethane (2.5 mL) followed by adding trifluoroacetic acid (0.50 mL, 6.6 mmol), and then the mixture was stirred at 25°C for one hour. After the solvent was distilled off under reduced pressure, the residue was purified by an ODS column chromatography (eluted with water/acetonitrile=100/0 to water/acetonitrile=0/100) to afford Compound 52 (32 mg, yield; 100%).
¹H NMR (CDCl₃, 300MHz) δ ppm: 7.99(br, 2H), 7.05(t, J=5.7Hz, 1H), 4.58(d, J=4.2Hz, 1H), 3.71(m, 1H), 3.19-3.39(m, 2H), 2.95(t, J=7.4Hz, 2H), 1.49-1.98(m, 6H), 1.22-1.48(m, 2H), 1.10(m, 1H), 0.97(d, J=6.0Hz, 3H), 0.94(d, J=6.0Hz, 3H)

### Example 53: Synthesis of Compound 53

In a manner similar to that in Example 48, Compound 53 (26 mg, yield; 46%) was obtained from Compound 71 (20 mg, 0.12 mmol) obtained in Example 71, dichloromethane (2.0 mL), N-α-[N-(tert-butyloxycarbonyl)-L-alanyl]-α,ε-diaminopentan e (32 mg, 0.12 mmol), 1,3-dicyclohexylcarbodiimide (24mg, 0.12 mmol) and 1-hydroxybenzotriazole monohydrate (27 mg, 0.23 mmol).
¹H NMR (CDCl₃, 300MHz)δ ppm: 6.62(m, 1H), 6.41(br s, 1H), 5.12(m, 1H), 4.58(d, J=4.8Hz, 1H), 4.13(m, 1H), 3.71(ddd, J=4.4, 7.0, 8.8Hz, 1H), 3.38(dt, J=7.0, 13.6Hz, 1H), 3.18-3.32(m, 3H), 1.77-1.94(m, 4H), 1.22-1.57(m, 5H), 1.44(s, 9H), 1.34(d, J=7.0Hz, 3H), 0.99(d, J=6.2Hz, 3H), 0.96(d, J=6.3Hz, 3H)
FABMS m/z: 428(M+H)⁺ calculated for C₂₁H₃₇N₃O₆=427
HRFABMS calculated for C₂₁H₃₈N₃O₆ (M+H)⁺ 428.2761 found 428.2760

### Example 54: Synthesis of Compound 54

Compound 52 (18 mg, 0.071 mmol) obtained in Example 52 was dissolved in N,N-dimethylformamide (1.8 mL) followed by adding N-(tert-butyloxycarbonyl)-cis-3-benzyloxy-L-proline (23 mg, 0.071 mmol), 1-ethyl-3-(3-dimethylaminopropyl)-carbodiimide hydrochloride (41 mg, 0.22 mmol), 1-hydroxybenzotriazole monohydrate (41 mg, 0.35 mmol), triethylamine (0.0098 mL, 0.071 mmol) and 4-dimethylaminopyridine (26 mg, 0.21 mmol), and then the mixture was stirred at 25°C for one hour. After usual post-treatment, the crude product (20 mg) was obtained by purifying with a chromatography on silica gel (eluted with n-hexane/ethyl acetate=1/3), and further purified by a preparative HPLC (ODS column, eluted with acetonitrile/water=75/25) to afford Compound 54 (22 mg, yield; 57%).
¹H NMR (CDCl₃, 300MHz) δ ppm: 7.27-7.40(m, 5H), 6.43(br s, 1H), 5.92(m, 1H), 4.50-4.63(m, 2H), 4.57(d, J=4.3Hz, 1H), 4.36(m, 1H), 4.31(d, J=6.1Hz, 1H), 3.69(ddd, J=4.2, 6.6, 7.6Hz, 1H), 3.50-3.64(m, 2H), 3.06-3.89(m, 4H), 2.08(m, 1H), 1.85-2.01(m, 3H), 1.20-1.54(m, 6H), 1.43(s, 9H), 0.99(d, J=6.2Hz, 3H), 0.96(d, J=6.2Hz, 3H), 0.89(m, 1H)
FABMS m/z: 560(M+H)⁺ calculated for C₃₀H₄₅N₃O₇=559
HRFABMS calculated for C₃₀H₄₆N₃O₇ (M+H)⁺ 560.3335 found 560.3354

### Example 55: Synthesis of Compound 55

In a manner similar to that in Example 47, Compound 55 (19 mg, yield; 40%) was obtained from Compound 71 (21 mg, 0.12 mmol) obtained in Example 71, dichloromethane (2.1 mL), benzyl 6-aminocaproate (42 mg, 0.12 mmol), 1,3-dicyclohexylcarbodiimide (26 mg, 0.12 mmol) and 1-hydroxybenzotriazole monohydrate (29 mg, 0.25 mmol).
¹H NMR (CDCl₃, 300MHz) δ ppm: 7.27-7.38(m, 5H), 6.40(m, 1H), 5.11(s, 2H), 4.52(d, J=4.4Hz, 1H), 3.69(ddd, J=4.4, 6.9, 8.4Hz, 1H), 3.20-3.41(m, 2H), 2.37(t, J=7.3Hz, 2H), 1.48-1.94(m, 6H), 1.29-1.42(m, 2H), 0.99(d, J=6.2Hz, 3H), 0.96(d, J=6.2Hz, 3H), 0.93(m, 1H)
FABMS m/z: 376(M+H)⁺ calculated for C₂₁H₂₉NO₅=375
HRFABMS calculated for C₂₁H₃₀NO₅ (M+H)⁺ 376.2124 found 376.2127

### Example 56: Synthesis of Compound 56

In a manner similar to that in Example 48, Compound 56 (33 mg, yield; 53%) was obtained from Compound 71 (19 mg, 0.11 mmol) obtained in Example 71, dichloromethane (1.9 mL), N-(tert-butyloxycarbonyl)-L-alanyl-L-lysinol benzoate (45 mg, 0.11 mmol), 1,3-dicyclohexylcarbodiimide (23 mg, 0.11 mmol) and 1-hydroxybenzotriazole monohydrate (26 mg, 0.22 mmol).
¹H NMR (CDCl₃, 300MHz)δ ppm: 8.01-8.08(m, 2H), 7.58(m, 1H), 7.41-7.49(m, 2H), 6.61(m, 1H), 6.53(m, 1H), 5.14(brd, J=6.8Hz, 1H), 4.63(d, J=4.2Hz, 1H), 4.22-4.42(m, 3H), 4.12(m, 1H), 3.71(ddd, J=4.4, 8.6, 8.6Hz, 1H), 3.45(m, 1H), 3.22(m, 1H), 1.76-1.94(m, 4H), 1.48-1.71(m, 4H), 1.41(s, 9H), 1.30(d, J=6.9Hz, 3H), 1.27(m, 1H), 0.99(d, J=6.4Hz, 3H), 0.96(d, J=6.2Hz, 3H)
FABMS m/z: 562(M+H)⁺ calculated for C₂₉H₄₃N₃O₈=561
HRFABMS calculated for C₂₉H₄₄N₃O₈ (M+H)⁺ 562.3129 found 562.3140

### Example 57: Synthesis of Compound 57

Compound 71 (82 mg, 0.48 mmol) obtained in Example 71 was dissolved in tetrahydrofuran (8.2 mL) followed by adding N-hydroxysuccineimide (0.11 g, 0.95 mmol) and 1,3-dicyclohexylcarbodiimide (0.11 g, 0.52 mmol), and then the mixture was stirred at 25°C for one hour. Further, N-α-(tert-butyloxycarbonyl)-L-ornithine (0.33 g, 1.43 mmol) was added to the reaction mixture and the mixture was stirred at 25°C for 17 hours. After usual post-treatment, purifying by a chromatography on silica gel (eluted with ethyl acetate) afforded Compound 57 (0.14 g, yield; 77%).
FABMS m/z: 387 (M+H)⁺ calculated for C₁₈H₃₀N₂O₇=386

### Example 58: Synthesis of Compound 58

In a manner similar to that in Example 9, Compound 58 (8.5 mg, yield; 18%) was obtained from Compound 57 (38mg, 0.098 mmol) obtained in Example 57, N,N-dimethylformamide (3.8 mL), benzyl bromide (0.052 mL, 0.44 mmol) and potassium carbonate (28 mg, 0.21 mmol).
¹H NMR (CDCl₃, 300MHz) δ ppm: 7.33-7.40(m, 5H), 6.44(m, 1H), 5.21(d, J=12.1Hz, 1H), 5.14(d, J=12.1Hz, 1H), 5.10(m, 1H), 4.50(d, J=4.4Hz, 1H), 4.35(m, 1H), 3.68(ddd, J=4.4, 7.1, 8.9Hz, 1H), 3.19-3.41(m, 2H), 1.75-1.92(m, 4H), 1.47-1.73(m, 3H), 1.43(s, 9H), 0.99(d, J=6.0Hz, 3H), 0.96(d, J=6.1Hz, 3H)
FABMS m/z: 477(M+H)⁺ calculated for C₂₅H₃₆N₂O₇=476
HRFABMS calculated for C₂₅H₃₇N₂O₇ (M+H)⁺ 477.2601 found 477.2601

### Example 59: Synthesis of Compound 59

Compound 58 (92 mg, 0.19 mmol) obtained in Example 58 was dissolved in dichloromethane (9.2 mL) followed by adding trifluoroacetic acid (1.8 mL, 24 mmol), and then the mixture was stirred at 25°C for one hour. After the solvent was distilled off under reduced pressure, the residue was dissolved in N,N-dimethylformamide (6.7 mL) followed by adding N-(tert-butyloxycarbonyl)-L-alanine (73 mg, 0.39 mmol), 1-ethyl-3-(3-dimethylaminopropyl)carbodiimide hydrochloride (74 mg, 0.39 mmol) and 1-hydroxybenzotriazole monohydrate (45 mg, 0.39 mmol), and then the mixture was stirred at 0°C for 0.5 hour. After usual post-treatment, the crude product (86 mg) was obtained by purifying with a chromatography on silica gel (eluted with n-hexane/ethyl acetate=1/1), and further purified by a preparative HPLC (ODS column, eluted with acetonitrile/water=50/50) to afford Compound 59 (61 mg, yield; 57%).
¹H NMR (CDCl₃, 300MHz) δ ppm: 7.43(br d, J=6.8Hz, 1H), 7.32(m, 5H), 6.65(m, 1H), 5.20(d, J=12.1Hz, 1H), 5.14(d, J=12.1Hz, 1H), 5.08(brs, 1H), 4.63(ddd, J=5.3, 7.5, 7.7Hz, 1H), 4.52(d, J=4.4Hz, 1H), 4.16(m, 1H), 3.70(ddd, J=4.4, 7.0, 9.0Hz, 1H), 3.34(m, 1H), 3.25(m, 1H), 1.74-1.97(m, 4H), 1.68(m, 1H), 1.47-1.61(m, 2H), 1.43(s, 9H), 1.33(d, J=7.1Hz, 3H), 0.99(d, J=6.3Hz, 3H), 0.95(d, J=6.3Hz, 3H)
FABMS m/z: 548(M+H)⁺ calculated for C₂₈H₄₁N₃O₈=547
HRFABMS calculated for C₂₈H₄₂N₃O₈ (M+H)⁺ 548.2972 found 548.2989

### Example 60: Synthesis of Compound 60

In a manner similar to that in Example 4, Compound 60 (22 mg, yield; 95%) was obtained from Compound 59 (23 mg, 0.042 mmol) obtained in Example 59, dichloromethane (2.3 mL) and trifluoroacetic acid (0.46 mL, 5.9 mmol).
¹H NMR (CDCl₃, 300MHz)δ ppm: 8.02(br s, 1H), 7.27-7.37(m, 5H), 7.24(br s, 1H), 5.17(d, J=12.3Hz, 1H), 5.08(d, J=12.1Hz, 1H), 4.52(d, J=4.0Hz, 1H), 4.51(m, 1H), 4.17(m, 1H), 3.71(m, 1H), 3.25(m, 2H), 1.36-1.98(m, 7H), 1.23-1.30(m, 3H), 0.94(d, J=6.1Hz, 3H), 0.92(d, J=7.0Hz, 3H)
FABMS m/z: 448(M+H)⁺ calculated for C₂₃H₃₃N₃O₆=447
HRFABMS calculated for C₂₃H₃₄N₃O₆ (M+H)⁺ 448.2447 found 448.2444

### Example 61: Synthesis of Compound 61

In a manner similar to that in Example 48, Compound 61 (40 mg, yield; 43%) was obtained from Compound 72 (33 mg, 0.19 mmol) obtained in Example 72, dichloromethane (3.3 mL), N-α-(tert-butyloxycarbonyl)-L-lysine benzyl ester (64 mg, 0.19 mmol), 1,3-cyclohexylcarbodiimide (39 mg, 0.19 mmol) and 1-hydroxybenzotriazole monohydrate (45 mg, 0.38 mmol).
¹H NMR (CDCl₃, 300MHz)δ ppm: 7.32-7.39(m, 5H), 6.34(m, 1H), 5.21(d, J=12.1Hz, 1H), 5.14(d, J=12.2Hz, 1H), 5.05(br d, J=7.7Hz, 1H), 4.52(d, J=4.4Hz, 1H), 4.33 (m, 1H), 3.69(ddd, J=4.4, 7.3, 9.0Hz, 1H), 3.30(m, 1H), 3.21(m, 1H), 1.74-1.92(m, 4H), 1.23-1.72(m, 5H), 1.43(s, 9H), 0.99(d, J=6.5Hz, 3H), 0.96(d, J=6.2Hz, 3H)
FABMS m/z: 491(M+H)⁺ calculated for C₂₆H₃₈N₂O₇=490
HRFABMS calculated for C₂₆H₃₉N₂O₇ (M+H)⁺ 491.2757 found 491.2747

### Example 62: Synthesis of Compound 62

In a manner similar to that in Example 4, Compound 62 (35 mg, yield; 84%) was obtained from Compound 61 (40 mg, 0.072 mmol) obtained in Example 61, dichloromethane (4.0 mL) and trifluoroacetic acid (0.80 mL, 10 mmol).
¹H NMR (CDCl₃, 300MHz)δ ppm: 7.29-7.38(m, 5H), 6.99(m, 1H), 5.22(d, J=12.3Hz, 1H), 5.15(d, J=12.1Hz, 1H), 4.51(d, J=4.2Hz, 1H), 4.02(m, 1H), 3.67(m, 1H), 3.17-3.34(m, 2H), 1.96(m, 1H), 1.65-1.89(m, 2H), 1.23-1.59(m, 6H), 0.95(d, J=4.9Hz, 3H), 0.93(d, J=6.3Hz, 3H)
FABMS m/z: 391 (M+H)⁺ calculated for C₂₁H₃₀N₂O₅=390
HRFABMS calculated for C₂₁H₃₁N₂O₅ (M+H)⁺ 391.2233 found 391.2245

### Example 63: Synthesis of Compound 63

In a manner similar to that in Example 48, Compound 63 (24 mg, yield; 44%) was obtained from Compound 72 (34 mg, 0.20 mmol) obtained in Example 72, dichloromethane (3.4 mL), N-(tert-butyloxycarbonyl)-L-alanyl-L-lysine benzyl ester (81 mg, 0.20 mmol), 1,3-dicyclohexylcarbodiimide (41 mg, 0.20 mmol) and 1-hydroxybenzotriazole monohydrate (47 mg, 0.40 mmol).
¹H NMR (CDCl₃, 300MHz) δ ppm: 7.32-7.38(m, 5H), 6.78(brd, J=7.6Hz, 1H), 6.59(m, 1H), 5.20(d, J=12.1Hz, 1H), 5.14(d, J=12.3Hz, 1H), 5.12(m, 1H), 4.62(ddd, J=5.0, 7.9, 7.9Hz, 1H), 4.54(d, J=4.2Hz, 1H), 4.37(m, 1H), 3.71(ddd, J=4.4, 7.2, 9.0Hz, 1H), 3.14-3.35(m, 2H), 1.63-1.94(m, 5H), 1.47-1.62(m, 2H), 1.44(s, 9H), 1.34(d, J=7.1Hz, 3H), 1.25-1.33(m, 2H), 0.99(d, J=6.4Hz, 3H), 0.97(d J=6.2Hz, 3H)
FABMS m/z: 562(M+H)⁺ calculated for C₂₉H₄₃N₃O₈=561
HRFABMS calculated for C₂₉H₄₄N₃O₈ (M+H)⁺ 562.3129 found 562.3155

### Example 64: Synthesis of Compound 64

In a manner similar to that in Example 4, Compound 64 (20 mg, yield; 100%) was obtained from Compound 63 (20 mg, 0.035 mmol) obtained in Example 63, dichloromethane (2.0 mL) and trifluoroacetic acid (0.40 mL, 5.0 mmol).
¹H NMR (CDCl₃, 300MHz) δ ppm: 7.82(br s, 1H), 7.28-7.37(m, 5H), 7.11(br s, 1H), 5.18(d, J=12.3Hz, 1H), 5.09(d, J=11.9Hz, 1H), 4.57(d, J=4.0Hz, 1H), 4.50(m, 1H), 4.18(m, 1H), 3.71(m, 1H), 3.11-3.32(m, 2H), 1.63-1.89(m, 5H), 1.40-1.56(m, 5H), 1.25-1.40(m, 2H), 0.96(d, J=5.3Hz, 3H), 0.92(d, J=6.4Hz, 3H)
FABMS m/z: 462(M+H)⁺ calculated for C₂₄H₃₅N₃O₆=461
HRFABMS calculated for C₂₄H₃₆N₃O₆ (M+H)⁺ 462.2604 found 462.2630

### Example 65: Synthesis of Compound 65

In a manner similar to that in Example 47, Compound 65 (44 mg, yield; 68%) was obtained from Compound 72 (31 mg, 0.18 mmol) obtained in Example 72, dichloromethane (3.1 mL), N-α-(tert-butyloxycarbonyl)-α,ε-diaminopentane (37 mg, 0.18 mmol), 1,3-dichlorohexylcarbodiimide (37 mg, 0.18 mmol) and 1-hydroxybenzotriazole monohydrate (42 mg, 0.36 mmol).
¹H NMR (CDCl₃, 300MHz) δ ppm: 6.46(br s, 1H), 4.57(m, 1H), 4.54(d, J=4.4Hz, 1H), 3.70(ddd, J=4.4, 7.3, 9.0Hz, 1H), 3.36(m, 1H), 3.28(m, 1H), 3.12(dd, J=6.4, 12.8Hz, 2H), 1.76-1.97(m, 3H), 1.31-1.53(m, 6H), 1.44(s, 9H), 1.00(d, J=6.2Hz, 3H), 0.98(d, J=6.2Hz, 3H)
FABMS m/z: 357(M+H)⁺ calculated for C₁₈H₃₂N₂O₅=356
HRFABMS calculated for C₁₈H₃₃N₂O₅ (M+H)⁺ 357.2390 found 357.2395

### Example 66: Synthesis of Compound 66

In a manner similar to that in Example 4, Compound 66 (15 mg, yield; 71%) was obtained from Compound 65 (20 mg, 0.056 mmol) obtained in Example 65, dichloromethane (2.0 mL) and trifluoroacetic acid (0.40 mL, 5.0 mmol).
¹H NMR (CDCl₃, 300MHz)δ ppm: 8.46(br s, 2H), 7.07(m, 1H), 4.58(d, J=4.2Hz, 1H), 3.71(ddd, J=4.2, 6.6, 9.0H2, 1H), 3.18-3.39(m, 2H), 2.90-3.01(m, 2H), 1.02-1.98(m, 9H), 0.98(d, J=6.1Hz, 3H), 0.94(d, J=6.0Hz, 3H)
FABMS m/z: 257(M+H)⁺ calculated for C₁₃H₂₄N₂O₃=256
HRFABMS calculated for C₁₃H₂₅N₂O₃ (M+H)⁺ 257.1865 found 257.1880

### Example 67: Synthesis of Compound 67

UCK14A₂ (2.0 mg, 0.0042 mmol) was dissolved in methanol (1.0 mL) followed by adding triethylamine (2.2 mL, 0.0022 mmol), and then the mixture was stirred at 25°C for 2 days. The solvent was distilled off under normal pressure. A 1 mol/L solution of hydrogen chloride in water was added to the residue, which was extracted with chloroform. The solvent was distilled off under reduced pressure to afford Compound 67 (0.8 mg, yield; 38%).
¹H NMR (CDCl₃, 300MHz)δ ppm: 9.36(br d, J=7.0Hz, 1H), 7.17(br s, 1H), 5.47(br d, J=6.9Hz, 1H), 4.82(m, 1H), 4.43(m, 1H), 4.23(d, J=2.6Hz, 1H), 3.65(s, 3H), 2.98(dd, J=2.2, 9.2Hz, 1H), 2.38-2.53(m, 2H), 1.95(m, 1H), 1.43(s, 9H), 1.41(m, 3H), 1.38(m, 1H), 1.20(m, 1H), 1.07(m, 1H), 1.03(d, J=7.0Hz, 3H), 0.92(t, J=7.7Hz, 3H), 0.75-0.87(m, 2H), 0.58(m, 1H)
FABMS m/z: 502(M+H)⁺ calculated for C₂₃H₃₉N₃O₉=501

### Example 68: Synthesis of Compound 68

Compound 67 (10 mg, 0.020 mmol) obtained in Example 67 was dissolved in N,N-dimethylformamide (3.8 mL) followed by adding benzyl bromide (0.0070 mL, 0.059 mmol) and potassium carbonate (5.0 mg, 0.036 mmol), and then the mixture was stirred at 25°C for 2 hours. After usual post-treatment, the crude product (12 mg) was obtained by purifying with a chromatography on silica gel (eluted with chloroform/methanol=100/0 to 50/1). The obtained crude product was dissolved in dichloromethane (0.40 mL) followed by adding trifluoroacetic acid (0.020 mL, 0.26 mmol), and then the mixture was stirred at 25°C for 4 hours. After concentrating the reaction mixture, the residue was purified by an ODS column chromatography (eluted with water/acetonitrile=100/0 to 0/100) to afford Compound 68 (4.0 mg, yield; 33%)
¹H NMR (CDCl₃, 300MHz) δ ppm: 10.04(br, 1H), 8.26(br, 2H), 7.33(s, 6H), 5.13(m, 2H), 4.93(br, 1H), 4.33(br, 2H), 3.55(s, 3H), 2.82(br, 1H), 2.68(br, 1H), 2.43(br, 1H), 2.05(br, 2H), 1.95(br, 1H), 1.67(br, 3H), 1.40(m, 1H), 1.15(br, 1H), 1.02(br, 3H), 0.88(br, 3H), 0.45-0.76(m, 3H)
FABMS m/z: 492(M+H)⁺ calculated for C₂₅H₃₇N₃O₇=491

### Example 69: Synthesis of Compound 69

Compound 3 (30 mg, 0.054 mmol) obtained in Example 3 was dissolved in dimethyl sulfoxide (0.90 mL) and a 0.04 mol/L sodium hydrogenphosphate - potassium dihydrogenphosphate buffer (pH=7.2, 2.8 mL) followed by adding mercaptoethanol (0.11 mL, 0.162 mmol), and then the mixture was stirred at 0°C for 0.5 hour. After adding water to the reaction mixture, the crude product (33 mg) was obtained by purifying with an ODS column chromatography (eluted with acetonitrile/water=100/0 to 0/100), and further purified by a preparative HPLC (ODS column, eluted with acetonitrile/a 0.04 mol/L phosphate buffer (pH=6.5)=55/45) to afford Compound 69 (13 mg, yield; 39%).
¹H NMR (CDCl₃, 500MHz) δ ppm: 9.52(d, J=9.2Hz, 1H), 7.31-7.44(m, 5H), 7.07(s, 1H), 5.49(s, 1H), 5.21(d, J=12.4Hz, 1H), 5.13(d, J=12.5Hz, 1H), 4.99(m, 1H), 4.42(m, 1H), 4.28(dd, J=1.8, 9.2Hz, 1H), 3.76(m, 1H), 3.59(m, 1H), 3.18(ddd, J=4.1, 4.1, 13.7Hz, 1H), 3.13(dd, J=1.7, 9.9Hz, 1H), 3.06(dd, J=5.9, 5.9Hz, 1H), 2.88(ddd, J=3.4, 7.7, 12.4Hz, 1H), 2.48(br s, J=14.6Hz, 1H), 2.39(br s, 1H), 2.00(m, 1H), 1.49(m, 1H), 1.44(s, 9H), 1.16-1.32(m, 5H), 1.06(d, J=6.7Hz, 3H), 0.92(t, J=7.3Hz, 3H), 0.61-0.74(m, 2H), 0.52(m, 1H)
FABMS m/z: 638(M+H)⁺ calculated for C₃₁H₄₇N₃O₉S=637

### Example 70: Synthesis of Compound 70

In a manner similar to that in Example 69, Compound 70(5.4 mg, yield; 32%) was obtained from Compound 4 (18 mg, 0.031 mmol) obtained in Example 4, dimethyl sulfoxide (0.18 mL), a 0.04 mol/L sodium hydrogenphosphate - potassium dihydrogenphosphate buffer (pH=7.2)(0.81 mL) and mercaptoethanol (0.066 mL, 0.939 mmol).
¹H NMR (CDCl₃, 300MHz)δ ppm: 9.04(br s, 1H), 7.28-7.41(m, 5H), 7.06(br s, 1H), 5.20(d, J=12.5Hz, 1H), 5.14(d, J=12.6Hz, 1H), 4.92(m, 1H), 4.26(m, 1H), 3.59-3.78(m, 3H), 3.04-3.24(m, 2H), 2.89(m, 1H), 2.38(br s, 1H), 2.28(br d, J=14.4Hz, 1H), 2.00(m, 1H), 1.12-1.58(m, 3H), 1.40(d, J=6.8Hz, 3H), 1.02(d, J=6.0H2, 3H), 0.99(d, J=7.4Hz, 3H), 0.63-0.82(m, 2H), 0.52(m, 1H)
FABMS m/z: 538(M+H)⁺ calculated for C₂₆H₃₉N₃O₇S=537
HRFABMS calculated for C₂₆H₄₀N₃O₇S (M+H)⁺ 538.2587 found 538.2580

### Example 71: Synthesis of Compound 71

Compound C (11 mg, 0.046 mmol) obtained in Reference Example 3 was dissolved in ethanol (1.1 mL) followed by adding 10% palladium on carbon (1.1 mg), and then the mixture was stirred at 25°C for 19 hours under hydrogen atmosphere. After passing the reaction mixture through Celite R545, the solvent was distilled off under reduced pressure. The residue was purified by a chromatography on silica gel (eluted with chloroform/ethanol=2/1) to afford Compound 71 (7.5 mg, yield; 94%).
¹H NMR (CDCl₃, 300MHz)δ ppm: 8.25(br s, 1H), 4.64(d, J=3.7Hz, 1H), 3.86 (m, 1H), 1.72-1.91(m, 3H), 0.98(d, J=6.4Hz, 3H), 0.96(d, J=6.8Hz, 3H)
FABMS m/z: 171(M-H)⁻ calculated for C₈H₁₂O₄=172

### Example 72: Synthesis of Compound 72

In a manner similar to that in Example 71, Compound 72 (0.084 g, yield; 100%) was obtained from Compound F (0.11 g, 0.42 mmol) obtained in Reference Example 6, ethanol (11 mL) and 10% palladium on carbon (0.011 g).
¹H NMR (CDCl₃, 300MHz) δ ppm: 9.10(br s, 1H), 4.61(m, 1H), 3.81(m, 1H), 1.67-1.87(m, 3H), 0.97(d, J=6.2Hz, 3H), 0.94(d, J=6.5Hz, 3H)
FABMS m/z: 171(M-H)⁻ calculated for C₈H₁₂O₄=172

### Example 73: Synthesis of Compound 73

In a manner similar to that in Example 47, Compound 73 (18 mg, yield; 25%) was obtained from (R)-4-carboxy-β-propiolactone (15 mg, 0.13 mmol), dichloromethane (1.5 mL), N-a-[N-(tert-butyloxycarbonyl)-L-alanyl]-L-lysine (1-napht halenemethyl)amide (59 mg, 0.13 mmol), 1,3-dicyclohexylcarbodiimide (53 mg, 0.26 mmol) and 1-hydroxybenzotriazole monohydrate (60 mg, 0.51 mmol).
¹H NMR (CDCl₃, 300MHz)δ ppm: 7.96(d, J=7.5Hz, 1H), 7.37-7.56(m, 4H), 7.36(m, 1H), 7.29(m, 1H), 6.72-6.80(m, 2H), 6.63(br s, 1H), 5.00(br d, J=6.6Hz, 1H), 4.28-4.40(m, 3H), 4.38(m, 1H), 4.06(dq, J=6.6, 6.6Hz, 1H), 3.81(dd, J=7.0, 16.9Hz, 1H), 3.52(dd, J=4.6, 16.9Hz, 1H), 3.33 (m, 1H), 3.19(m, 1H), 1.74-1.99(m, 2H), 1.47-1.76(m, 4H), 1.35(s, 9H), 1.23(d, J=7.1Hz, 3H)
FABMS m/z: 555(M+H)⁺ calculated for C₂₉H₃₈N₄O₇=554

### Example 74: Synthesis of Compounds 74 and 75

Compound C (0.20 g, 0.78 mmol) obtained in Reference Example 3 was dissolved in ethanol (13 mL) followed by adding 10% palladium on carbon (0.020 g) and then the mixture was stirred at 25°C for 1.5 hours under hydrogen atmosphere. After passing the reaction mixture through Celite R545, the solvent was distilled off under reduced pressure. The residue was purified by a chromatography on silica gel (eluted with chloroform/ethanol=2/1) to afford a carboxylic acid (0.14 g). A part (10 mg, 0.060 mmol) of the carboxylic acid was dissolved in dichloromethane (1.0 mL) followed by adding N-α-[N-(tert-butyloxycarbonyl)-L-alanyl]-L-lysine (1-napht halenemethyl)amide (28 mg, 0.060 mmol), 1,3-dicyclohexylcarbodiimide (13 mg, 0.060 mmol) and 1-hydroxybenzotriazole monohydrate (14 mg, 0.12 mmol), and then the mixture was stirred at 0°C for 30 minutes. After usual post-treatment, the crude product was obtained by purifying with a thin layer column chromatography (developed with n-hexane/ethyl acetate=1/2), and further purified by a preparative HPLC (ODS column, eluted with acetonitrile/water=55/45) to afford Compound 74 (6.3mg, yield; 17%) and Compound 75 (13 mg, yield; 36%).

### Compound 74

¹H NMR (CDCl₃, 300MHz) δ ppm: 7.97(m, 1H), 7.87(m, 1H), 7.79(dd, J=3.5, 5.9Hz, 1H), 7.38-7.57 (m, 4H), 6.68-6.77(m, 2H), 6.53(m, 1H), 5.32(m, 1H), 4.98(d, J=6.8Hz, 1H), 4.85-4.91(m, 2H), 4.61(d, J=4.4Hz, 1H), 4.48(dd, J=4.6, 8.8Hz, 1H), 4.38(dt, J=5.1, 8.4Hz, 1H), 4.06(dq, J=6.8, 6.8Hz, 1H), 3.37(m, 1H), 3.20(m, 1H), 1.85-2.08(m, 2H), 1.79(s, 3H), 1.48-1.75(m, 4H), 1.72(s, 3H), 1.35(s, 9H), 1.23(d, J=7.0Hz, 3H)
FABMS m/z: 609(M+H)⁺ calculated for C₃₃H₄₄N₄O₇=608

### Compound 75

¹H NMR (CDCl₃, 300MHz) δ ppm: 7.96(m, 1H), 7.85(m, 1H), 7.78(dd, J=3.8, 5.7Hz, 1H), 7.37-7.55(m, 4H), 6.73-6.83(m, 2H), 6.58(m, 1H), 5.01(d, J=7.0Hz, 1H), 4.79-4.94(m, 2H), 4.58(d, J=4.1Hz, 1H), 4.37(dt, J=5.0, 8.3Hz, 1H), 4.08(dq, J=7.0, 7.0Hz, 1H), 3.68(ddd, J=4.2, 7.0, 8.6Hz, 1H), 3.37(m, 1H), 3.17(m, 1H), 1.62-1.98(m, 6H), 1.46-1.60(m, 3H), 1.35(s, 9H), 1.21(d, J=7.2Hz, 3H), 0.97(d, J=6.2Hz, 3H), 0.94(d, J=6.1Hz, 3H)
FABMS m/z: 611(M+H)⁺ calculated for C₃₃H₄₆N₄O₇=610

### Example 75: Synthesis of Compound 76

Compound J (11 mg, 0.041 mmol) obtained in Reference Example 10 was dissolved in 1,4-dioxane (0.55 mL) and water (0.55 mL) followed by adding, a 12 mol/L solution of hydrogen chloride in water (0.0069 mL, 0.081 mmol) and the mixture was stirred at 60°C for one hour. The solvent was distilled off under reduced pressure. The residue was dissolved in dichloromethane (0.96 mL) followed by adding N-α-[N-(tert-butyloxycarbonyl)-L-alanyl]-L-lysine (1-napht halenemethyl)amide (18 mg, 0.040 mmol), 1,3-dicyclohexylcarbodiimide (16 mg, 0.079 mmol) and 1-hydroxybenzotriazole monohydrate(19 mg, 0.16mmol), and then the mixture was stirred at 25°C for one hour. After usual post-treatment, purifying by a thin layer column chromatography (developed with chloroform/methanol=20/1) afforded Compound 76 (23 mg, yield; 87%).
¹H NMR (CDCl₃, 300MHz)δ ppm: 7.97(m, 1H), 7.85(m, 1H), 7.38(dd, J=2.9, 6.5Hz, 1H), 7.37-7.56(m, 4H), 6.95(m, 1H), 6.79-7.00(m, 3H), 5.19(br d, J=6.6Hz, 1H), 4.78-4.95(m, 2H), 4.37(m, 1H), 4.02-4.16(m, 2H), 3.32(m, 1H), 3.11(m, 1H), 2.83(dd, J=7.6, 15.1Hz, 1H), 2.82(dd, J=7.5, 14.9Hz, 1H), 1.59-1.98(m, 3H), 1.42-1.57(m, 2H), 1.14-1.40(m, 4H), 1.34(s, 9H), 1.17-1.34(m, 6H), 0.94(d, J=6.3Hz, 3H), 0.91(d, J=6.2Hz, 3H)
FABMS m/z: 673(M+H)⁺ calculated for C₃₅H₅₂N₄O₇S=672

### Example 76: Synthesis of Compounds 77 and 78

Compound L (57 mg, 0.17 mmol) obtained in Reference Example 12 was dissolved in ethanol (2.8 mL) followed by adding 10% palladium on carbon (5.7 mg) and then the mixture was stirred at 25°C for 2 hours under hydrogen atmosphere. After passing the reaction mixture through Celite R545, the solvent was distilled off under reduced pressure. A part (18 mg, 0.071 mmol) of the residue was dissolved in dichloromethane (1.8 mL) followed by adding N-α-[N-(tert-butyloxycarbonyl)-L-alanyl]-L-lysine (1-napht halenemethyl)amide (32 mg, 0.071 mmol), 1,3-dicyclohexylcarbodiimide (15 mg, 0.071 mmol) and 1-hydroxybenzotriazole monohydrate (17 mg, 0.14 mmol), and then the mixture was stirred at 0°C for 30 minutes. After usual post-treatment, the crude product (32 mg) was obtained by purifying with a thin layer column chromatography (developed with n-hexane/ethyl acetate=1/4), and further purified by a preparative HPLC (ODS column, eluted with acetonitrile/water=55/45) to afford Compound 77 (8.9 mg, yield; 28%) and Compound 78 (12 mg, yield; 36%).

### Compound 77

¹H NMR (CDCl₃, 300MHz) δ ppm: 7.98(m, 1H), 7.85(m, 1H), 7.78(dd, J=2.4, 7.2Hz, 1H), 7.38-7.56(m, 4H), 6.90(br s, 1H), 6.78(br d, J=6.9Hz, 1H), 6.55(m, 1H), 5.21(d, J=9.7Hz, 1H), 5.16(d, J=6.1Hz, 1H), 4.95(dd, J=5.7, 14.5Hz, 1H), 4.82(dd, J=5.3, 15.0Hz, 1H), 4.64(dd, J=6.8, 8.2Hz, 2H), 4.36(dt, J=5.2, 8.1Hz, 1H), 4.28(d, J=6.3Hz, 1H), 4.08(dq, J=7.0, 7.0Hz, 1H), 3.78(dd, J=6.2, 9.9Hz, 1H), 3.65(s, 3H), 3.36(s, 3H), 3.10-3.32(m, 2H), 1.95(m, 1H), 1.73(s, 3H), 1.63-1.72(m, 3H), 1.65(s, 3H), 1.40-1.55(m, 2H), 1.32(s, 9H), 1.27(d, J=6.8Hz, 3H)
FABMS m/z: 685(M+H)⁺ calculated for C₃₆H₅₂N₄O₉=684

### Compound 78

¹H NMR (CDCl₃, 300MHz) δ ppm: 7.98(m, 1H), 7.85(m, 1H), 7.78(dd, J=2.6, 6.8Hz, 1H), 7.38-7.56(m, 4H), 6.89(br s, 1H), 6.81(br d, J=7.4Hz, 1H), 6.62(m, 1H), 5.11(d, J=6.4Hz, 1H), 4.94(dd, J=5.5, 14.1Hz, 1H), 4.82(dd, J=5.0, 14.7Hz, 1H), 4.63(s, 2H), 4.37(dt, J=4.8, 4.8Hz, 1H), 4.20(d, J=5.9Hz, 1H), 4.08(dq, J=6.7, 6.7Hz, 1H), 3.66(s, 3H), 3.36(s, 3H), 3.11-3.34(m, 2H), 2.96(dt, J=4.9, 9.7Hz, 1H), 1.94(m, 1H), 1.66-1.77(m, 4H), 1.45-1.61(m, 2H), 1.21-1.39(m, 5H), 1.34(s, 9H), 0.89(d, J=6.6Hz, 3H), 0.87(d, J=6.4Hz, 3H)
FABMS m/z: 687(M+H)⁺ calculated for C₃₆H₅₄N₄O₉=686

### Example 77: Synthesis of Compound 79

Compound 78 (13 mg, 0.019 mmol) obtained in Example 76 was dissolved in 1,4-dioxane (0.65 mL) and water (0.65 mL) followed by adding a 4 mol/L aqueous solution of potassium hydroxide (0.014mL, 0.056 mmol) and then the mixture was stirred at 25°C for 13 hours. DOWEX50W was added to the reaction mixture for neutralization, and then the solvent was distilled off under reduced pressure. The residue was purified by a thin layer column chromatography (developed with chloroform/methanol=5/1) to afford Compound 79 (13 mg, yield; 100%).
¹H NMR (CDCl₃, 300MHz)δ ppm: 7.95(d, J=7.8Hz, 1H), 7.85(m, 1H), 7.78(dd, J=3.5, 5.9Hz, 1H), 7.37-7.56(m, 4H), 7.05(br s, 1H), 6.64(m, 2H), 5.30(s, 2H), 4.85(m, 1H), 4.73(d, J=6.9Hz, 1H), 4.66(d, J=7.0Hz, 1H), 4.36(m, 1H), 4.24 (m, 1H), 4.14(dq, J=8.3, 8.3Hz, 1H), 3.39(s, 3H), 2.85-3.03(m, 2H), 1.58-1.84(m, 4H), 1.41-1.53(m, 3H), 1.29-1.39(m, 2H), 1.38(s, 9H), 1.27(m, 3H), 0.94(d, J=5.7Hz, 3H), 0.92(d, J=6.1Hz, 3H)
FABMS m/z: 673(M+H)⁺ calculated for C₃₅H₅₂N₄O₉=672

### Example 78: Synthesis of Compound 80

In a manner similar to that in Example 47, Compound 80 (20 mg, yield; 61%) was obtained from compound 71 (11 mg, 0.063 mmol) obtained in Example 71, dichloromethane (1.1 mL), 4-amino-N-(tert-butyloxycarbonyl)-L-phenylalanine benzyl ester (23 mg, 0.063 mmol), 1,3-dicyclohexylcarbodiimide (26 mg, 0.13 mmol) and 1-hydroxybenzotriazole monohydrate (15 mg, 0.13 mmol).
¹H NMR (CDCl₃, 300MHz) δ ppm: 7.95(br s, 1H), 7.42(d, J=8.5Hz, 2H), 7.28-7.49(m, 5H), 7.01(d, J=8.3Hz, 2H), 5.19(d, J=12.5Hz, 1H), 5.08(d, J=12.3Hz, 1H), 4.97(br d, J=8.1Hz, 1H), 4.67(d, J=4.4Hz, 1H), 4.60(m, 1H), 3.83(ddd, J=4.4, 7.1, 8.7Hz, 1H), 3.43(m, 1H), 2.98-3.14(m, 2H), 1.80-1.98(m, 2H), 1.41(s, 9H), 1.27(m, 1H), 1.02(d, J=6.0Hz, 3H), 0.99(d, J=6.1Hz, 3H)
FABMS m/z: 525(M+H)⁺ calculated for C₂₉H₃₆N₂O₇=524

### Example 79: Synthesis of Compound 81

In a manner similar to that in Example 47, Compound 81 (13 mg, yield; 43%) was obtained from Compound 71 (9.1mg, 0.053 mmol) obtained in Example 71, dichloromethane (0.9 mL), Compound N (21 mg, 0.053 mmol) obtained in Reference Example 14, 1,3-dicyclohexylcarbodiimide (11 mg, 0.053 mmol) and 1-hydroxybenzotriazole monohydrate (12 mg, 0.11 mmol).
¹H NMR (CDCl₃, 300MHz) δ ppm: 8.59(br s, 1H), 8.19(s, 1H), 7.30-7.37(m, 5H), 6.82(dd, J=2.0, 8.5Hz, 1H), 6.76(d, J=8.3Hz, 1H), 5.21(d, J=12.5Hz, 1H), 5.15(d, J=12.1Hz, 1H), 5.01(br d, J=7.5Hz, 1H), 4.65(d, J=4.4Hz, 1H), 4.59(m, 1H), 3.87(s, 3H), 3.71(m, 1H), 2.92-3.13(m, 2H), 1.79-1.96(m, 2H), 1.40(s, 9H), 1.28(m, 1H), 1.02(d, J=6.1Hz, 3H), 0.99(d, J=6.1Hz, 3H)
FABMS m/z: 555(M+H)⁺ calculated for C₃₀H₃₈N₂O₈=554

### Example 80: Synthesis of Compound 82

Compound 71 (11 mg, 0.064 mmol) obtained in Example 71 was dissolved in dichloromethane (1.1 mL) followed by adding Compound P (30 mg, 0.064 mmol) obtained in Reference Example 16, 1,3-dicyclohexylcarbodiimide (13 mg, 0.064 mmol) and 1-hydroxybenzotriazole monohydrate (15 mg, 0.13 mmol), and then the mixture was stirred at 25°C for 0.5 hour. After usual post-treatment, purifying by a thin layer column chromatography (developed with n-hexane/ethyl acetate=3/1) afforded Compound 82 (16 mg, yield; 40%).
¹H NMR (CDCl₃, 300MHz) δ ppm: 8.73(br s, 1H), 8.18(s, 1H), 7.30-7.46(m, 10H), 6.84(d, J=8.4Hz, 1H), 6.79(dd, J=1.8, 8.3Hz, 1H), 5.21(d, J=12.2Hz, 1H), 5.15(d, J=12.2Hz, 1H), 5.12(s, 2H), 5.01(br d, J=8.5Hz, 1H), 4.62(d, J=4.4Hz, 1H), 4.59(m, 1H), 3.79(ddd, J=4.3, 6.5, 9.1Hz, 1H), 2.98-3.12(m, 2H), 1.78-1.94(m, 2H), 1.41(s, 9H), 1.27(m, 1H), 1.01(d, J=6.0Hz, 3H), 0.99(d, J=6.1Hz, 3H)
FABMS m/z: 631(M+H)⁺ calculated for C₃₆H₄₂N₂O₈=630

### Example 81: Synthesis of Compound 83

A 12 mol/L solution of hydrogen chloride in water (5.2 mL) was added to UCK14A₂ (0.10 g, 0.22 mmol), and the mixture was stirred at 120°C for 23 hours. The reaction mixture was concentrated, and then the residue was dissolved in water (5.2 mL). Amberlite" IRA-400 was added thereto for neutralization, and then purifying by a column chromatography using Amberlite™ IRC-50 (eluted with water/a 1 mol/L aqueous solution of hydrogen chloride=100/0 to 0/100) afforded Compound 83 (0.030 g, yield; 74%).
¹H NMR (D₂O, 300MHz)δ ppm: 4.05(brs, 1H), 2.54(brs, 1H), 2.12(br s, 1H), 1.72(br s, 1H), 1.32(br s, 1H), 1.05(br s, 1H), 0.83(br s, 1H)
FABMS m/z: 145(M+H)⁺ calculated for C₆H₁₂N₂O₂=144

### Example 82: Synthesis of Compound 84

Compound 83 (30 mg, 0.16 mmol) obtained in Example 81 was dissolved in water (3.0 mL) followed by adjusting the pH to 10 with a 1 mol/L aqueous solution of potassium hydroxide. Then ethyl trifluoroacetate (1.2 mL, 10 mmol) was added thereto and the mixture was stirred at 25°C for 2 hours. After usual post-treatment, the crude product was obtained by purifying with a chromatography on silica gel (eluted with chloroform/methanol/acetic acid=20/10/1). The resulting crude product was dissolved in tetrahydrofuran (1.9 mL) and water (1.9 mL) followed by adding sodium hydrogencarbonate (110 mg, 1.3 mmol) and di-tert-butyl dicarbonate (0.30 mL, 1.3 mmol), and then the mixture was stirred at 25°C for 11 hours. After usual post-treatment, purifying by a chromatography on silica gel (eluted with chloroform/methanol=5/1) afforded Compound 84 (22 mg, yield; 40%).
¹H NMR (CDCl₃, 300MHz) δ ppm: 6.48-6.98(m, 2H), 6.01(d, J=8.1Hz, 1H), 4.51(m, 1H), 2.50(m, 2H), 2.08(m, 1H), 1.46(s, 9H), 0.98(m, 1H), 0.89(m, 1H), 0.83(m, 1H)
FABMS m/z: 341(M+H)⁺ calculated for C₁₃H₁₉N₂O₅F₃=340

### Example 83: Synthesis of Compound 85

In a manner similar to that in Example 19, Compound 85 (24 mg, yield; 77%) was obtained from Compound 84 (22 mg, 0.065 mmol) obtained in Example 82, dichloromethane (1.1 mL), 1-naphthalenemethylamine (0.019 mL, 0.13 mmol), 1,3-dicyclohexylcarbodiimide (27 mg, 0.13 mmol) and 1-hydroxybenzotriazole monohydrate (31 mg, 0.26 mmol).
¹H NMR (CDCl₃, 300MHz)δ ppm: 7.98(m, 1H), 7.86(m, 1H), 7.79(dd, J=2.9, 6.4Hz, 1H), 7.38-7.55(m, 4H), 6.88(m, 2H), 6.43(d, J=8.8Hz, 1H), 4.93(dd, J=5.7, 14.7Hz, 1H), 4.86(dd, J=5.5, 14.9Hz, 1H), 4.39(m, 1H), 2.19-2.41(m, 2H), 1.69(m, 1H), 1.38(s, 9H), 0.96(m, 1H), 0.80(m, 1H), 0.63(dd, J=6.3, 13.1Hz, 1H)
FABMS m/z: 480(M+H)⁺ calculated for C₂₄H₂₈N₃O₄F₃=479

### Example 84: Synthesis of Compound 86

Compound 85 (22 mg, 0.047 mmol) obtained in Example 83 was dissolved in methanol (2.2 mL) followed by adding a 4 mol/L aqueous solution of sodium hydroxide (0.047 mL, 0.19 mmol), and then the mixture was stirred at 60°C for 6 hours. After usual post-treatment, purifying by a thin layer column chromatography (developed with chloroform/methanol=10/1) afforded Compound 86 (16 mg, yield; 40%).
¹H NMR (CDCl₃, 300MHz) δ ppm: 8.00(m, 1H), 7.87(m, 1H), 7.80(dd, J=2.1, 7.4Hz, 1H), 7.38-7.58(m, 4H), 7.51(m, 1H), 5.13(m, 1H), 4.91(d, J=5.5Hz, 2H), 4.15(m, 1H), 2.05(m, 1H), 1.62(dd, J=7.2, 7.2Hz, 2H), 1.37(s, 9H), 0.68(m, 1H), 0.49(m, 1H), 0.32(m, 1H)
FABMS m/z: 384(M+H)⁺ calculated for C₂₂H₂₉N₃O₃=383

### Example 85: Synthesis of Compound 87

Compound 85 (75 mg, 0.16 mmol) obtained in Example 83 was dissolved in dichloromethane (7.5 mL) followed by adding trifluoroacetic acid (0.75 mL) and then the mixture was stirred at 25°C for one hour. The reaction mixture was concentrated to afford Compound 87 (62 mg, yield; 100%).
¹H NMR (CDCl₃, 300MHz)δ ppm: 8.00(d, J=8.6Hz, 1H), 7.86(m, 1H), 7.79(dd, J=3.0, 6.6Hz, 1H), 7.38-7.56(m, 4H), 7.00(br s, 1H), 6.97(br s, 1H), 4.88(d, J=5.3Hz, 2H), 3.60(m, 1H), 2.37(m, 1H), 1.85-2.12(m, 2H), 0.89(m, 1H), 0.81(m, 1H), 0.70(m, 1H)
FABMS m/z: 380(M+H)⁺ calculated for C₁₉H₂₀N₃O₂F₃=379

### Example 86: Synthesis of Compound 88

Compound 87 (59 mg, 0.16 mmol) obtained in Example 85 was dissolved in N,N-dimethylformamide (5.9 mL) followed by adding N-(tert-butyloxycarbonyl)-L-alanine (59mg, 0.31 mmol), 1-ethyl-3-(3-dimethylaminopropyl)carbodiimidehydrochloride (60 mg, 0.31 mmol) and 1-hydroxybenzotriazole monohydrate (73 mg, 0.62 mmol), and then the mixture was stirred at 25°C for 0.5 hour. After usual post-treatment, purifying by a chromatography on silica gel (eluted with chloroform/methanol=30/1) afforded Compound 88 (81 mg, yield; 94%).
¹H NMR (CDCl₃, 300MHz) δ ppm: 8.38(br d, J=8.6Hz, 1H), 7.94(dd, J=2.4, 5.5Hz, 1H), 7.82(m, 1H), 7.75(m, 1H), 7.52(br s, 1H), 7.43-7.51(m, 2H), 7.34-7.39(m, 2H), 7.18(m, 1H), 5.17(m, 1H), 4.84(d, J=5.6Hz, 2H), 4.80(m, 1H), 4.11(m, 1H), 2.26-2.52(m, 2H), 1.36(s, 9H), 1.23(d, J=7.2Hz, 3H), 0.93-1.11(m, 2H), 0.79(m, 1H), 0.55(dd, J=6.2, 13.0Hz, 1H)
FABMS m/z: 551(M+H)⁺ calculated for C₂₇H₃₃N₄O₅F₃=550

### Example 87: Synthesis of Compound 89

In a manner similar to that in Example 84, Compound 89 (26mg, yield; 100%) was obtained from Compound 88 (26 mg, 0.047 mmol) obtained in Example 86, methanol (2.6 mL) and a 4 mol/L aqueous solution of sodium hydroxide (0.047 mL, 0.19 mmol).
¹H NMR (CDCl₃, 300MHz)δ ppm: 8.00(m, 1H), 7.85(m, 1H), 7.78(m, 1H), 7.37-7.56(m, 4H), 6.88(brd, J=8.2Hz, 1H), 6.86(br s, 1H), 5.09(d, J=6.6Hz, 1H), 4.89(d, J=5.3Hz, 2H), 4.50(dd, J=6.8, 14.7Hz, 1H), 4.09 (m, 1H), 2.01 (m, 1H), 1.53-1.66 (m, 2H), 1.34 (s, 9H), 1.26(d, J=7.3Hz, 3H), 0.61(m, 1H), 0.44(m, 1H), 0.30(m, 1H)
FABMS m/z: 455(M+H)⁺ calculated for C₂₅H₃₄N₄O₄=454

### Example 88: Synthesis of Compound 90

Compound 71 (4.6 mg, 0.027 mmol) obtained in Example 71 was dissolved in dichloromethane (0.92 mL) followed by adding Compound 86 (10 mg, 0.027 mmol) obtained in Example 84, 1,3-dicyclohexylcarbodiimide (11 mg, 0.054 mmol) and 1-hydroxybenzotriazole monohydrate (13 mg, 0.11 mmol), and then the mixture was stirred at 25°C for 30 minutes. After usual post-treatment, the crude product (11 mg) was obtained by purifying with a thin layer column chromatography (developed with toluene/ethyl acetate=1/1), and further purified by a preparative HPLC (ODS column, eluted with acetonitrile/water=60/40) to afford Compound 90 (4.2mg, yield; 29%).
¹H NMR (CDCl₃, 300MHz)δ ppm: 8.01(m, 1H), 7.87(m, 1H), 7.80(dd, J=2.8, 7.0Hz, 1H), 7.47-7.53(m, 2H), 7.37-7.44(m, 2H), 6.82(m, 1H), 6.37(m, 2H), 4.96(dd, J=5.7, 14.5Hz, 1H), 4.42(m, 1H), 4.38(dd, J=5.3, 14.5Hz, 1H), 4.21 (m, 1H), 3.18(m, 1H), 2.43(d, J=13.3Hz, 1H), 2.11(m, 1H), 1.52-1.72(m, 3H), 1.40(s, 9H), 1.11(m, 1H), 0.90(d, J=5.8Hz, 3H), 0.87(m, 1H), 0.82(d, J=6.0Hz, 3H), 0.66(m, 1H), 0.52(dd, J=6.4, 13.2Hz, 1H)
FABMS m/z: 538(M+H)⁺ calculated for C₃₀H₃₉N₃O₆=537
HRFABMS calculated for C₃₀H₄₀N₃O₆ (M+H)⁺ 538.2930 found 538.2903

### Example 89: Synthesis of Compound 91

In a manner similar to that in Example 88, Compound 91 (5.9 mg, yield; 21%) was obtained from Compound 71 (8.2 mg, 0.047 mmol) obtained in Example 71, dichloromethane (1.6 mL), Compound 89 (22 mg, 0.047 mmol) obtained in Example 87, 1,3-dicyclohexylcarbodiimide (29 mg, 0.14 mmol) and 1-hydroxybenzotriazole monohydrate (33 mg, 0.28 mmol).
¹H NMR (CDCl₃, 300MHz)δ ppm: 8.98(m, 1H), 7.98(dd, J=3.5, 6.2Hz, 1H), 7.87(dd, J=3.3, 6.1Hz, 1H), 7.80(dd, J=4.6, 4.6Hz, 1H), 7.49(dd, J=3.3, 6.4Hz, 2H), 7.39(d, J=5.5Hz, 2H), 6.85(br s, 1H), 6.51(br s, 1H), 5.22(m, 1H), 5.00(dd, J=6.0, 14.5Hz, 1H), 4.74-4.86(m, 2H), 4.35(d, J=4.2Hz, 1H), 4.25(m, 1H), 3.08(m, 1H), 2.62(m, 1H), 2.28(br s, 1H), 1.63(m, 3H), 1.40(s, 9H), 1.21(d, J=7.0Hz, 3H), 1.00(m, 1H), 0.89(d, J=6.3Hz, 3H), 0.87(m, 1H), 0.77(d, J=6.5Hz, 3H), 0.72(m, 1H), 0.56(m, 1H)
FABMS m/z: 609(M+H)⁺ calculated for C₃₃H₄₄N₄O₇=608
HRFABMS calculated for C₃₃H₄₅N₄O₇ (M+H)⁺ 609.3280 found 609.3295

### Example 90: Injections

Finely ground Compound 4 is dissolved in water for injections. The solution is filtered and the filtrate is sterilized with an autoclave to afford the injections.

| Inqredient per ampoule | |
|---|---|
| Compound 4 | 10 mg |
| Water for injections | an appropriate amount |
| Total volume | 1.0 ml |

### Example 91: Tablets

Finely ground Compound 3 is mixed with powdered potato starch, lactose, magnesium stearate, polyvinyl alcohol and tar dye, and the mixture is compressed to mold the tablets.

| Ingredient per tablet | |
|---|---|
| Compound 3 | 100 mg |
| Lactose | 60 mg |
| Potato starch | 50 mg |
| Polyvinyl alcohol | 2 mg |
| Magnesium stearate | 1 mg |
| Tar dye | an appropriate amount |

### Example 92: Powders

Finely ground Compound 21 is mixed with powdered lactose to afford the powders.

| Ingredient per dose | |
|---|---|
| Compound 21 | 100 mg |
| Lactose | 240 mg |

### Example 93: Suppository

Finely ground Compound 50 is mixed with melted cocoa butter, and the mixture is poured into molds and cooled to afford the suppository.

| Ingredient per one dose of suppository | |
|---|---|
| Compound 50 | 10 mg |
| Cocoa butter (basis) | an appropriate amount |
| Total volume | 2.0 g |

### Example 94: Capsules

Finely ground Compound 3 is mixed with powdered lactose and magnesium stearate. Gelatin capsules are filled with the mixture to afford the capsules.

| Ingredient per capsule | |
|---|---|
| Compound 3 | 100 mg |
| Lactose | 540 mg |
| Magnesium stearate | 1 mg |

### Example 95: Nasal drops

An antiseptic agent is dissolved in warmed purified water and the solution is gradually cooled. Sodium chloride and finely ground Compound 24 are added thereto. The pH of the mixture is adjusted to 5.5 to 6.5, and the mixture is diluted with purified water into a final volume of 100 ml to afford the nasal drops.

| Ingredient per 100 ml of drops | |
|---|---|
| Compound 24 | 1000 mg |
| Sodium chloride | 800 mg |
| Antiseptic agent | 500 mg |
| Purified water | an appropriate amount |
| Total volume | 100 ml |

### Example 96: Eye drops

The eye drops are obtained by a method similar to that

The eye drops are obtained by a method similar to that in the case of nasal drops.

| Ingredient per 100 ml of drops | |
|---|---|
| Compound 24 | 100 mg |
| Sodium chloride | 800 mg |
| Antiseptic agent | 500 mg |
| Purified water | an appropriate amount |
| Total | 100 ml |

### Example 97: Topical cream

An antiseptic agent is dissolved in warmed purified water and the solution is gradually cooled. Emulsified wax, mineral oil, and white petrolatum are added thereto followed by mixing thoroughly at 70 to 80°C. An aqueous solution containing Compound 50 is added thereto and the mixture is stirred. Purified water is added thereto with stirring up to a total weight of 100 g to afford the topical cream.

| Inqredient per 100 g of cream | |
|---|---|
| Compound 50 | 1000 mg |
| Emulsified wax | 15 g |
| Mineral oil | 5 g |
| White petrolatum | 5 g |
| Antiseptic agent | 200 mg |
| Purified water | an appropriate amount |
| Total weight | 100 g |

The Reference Examples of the present invention are shown below. The structures of the compounds in the Reference Examples are shown in Table 7.

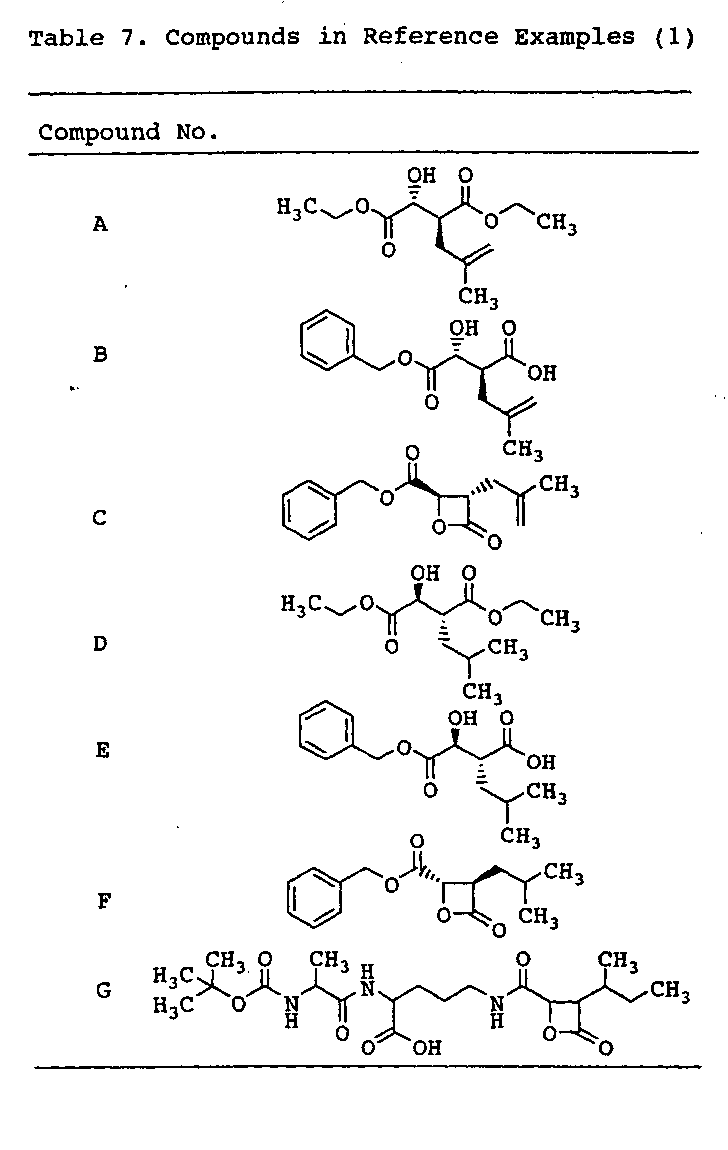

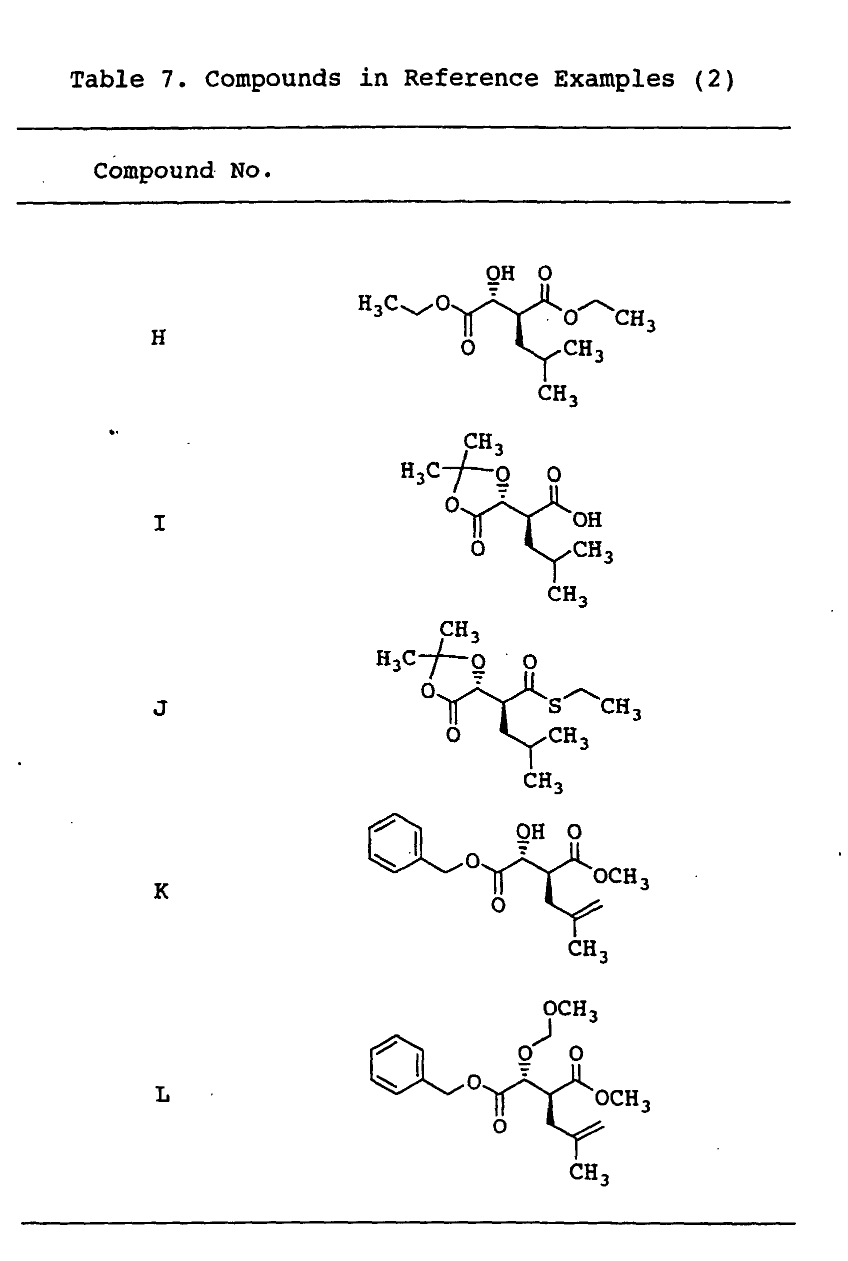

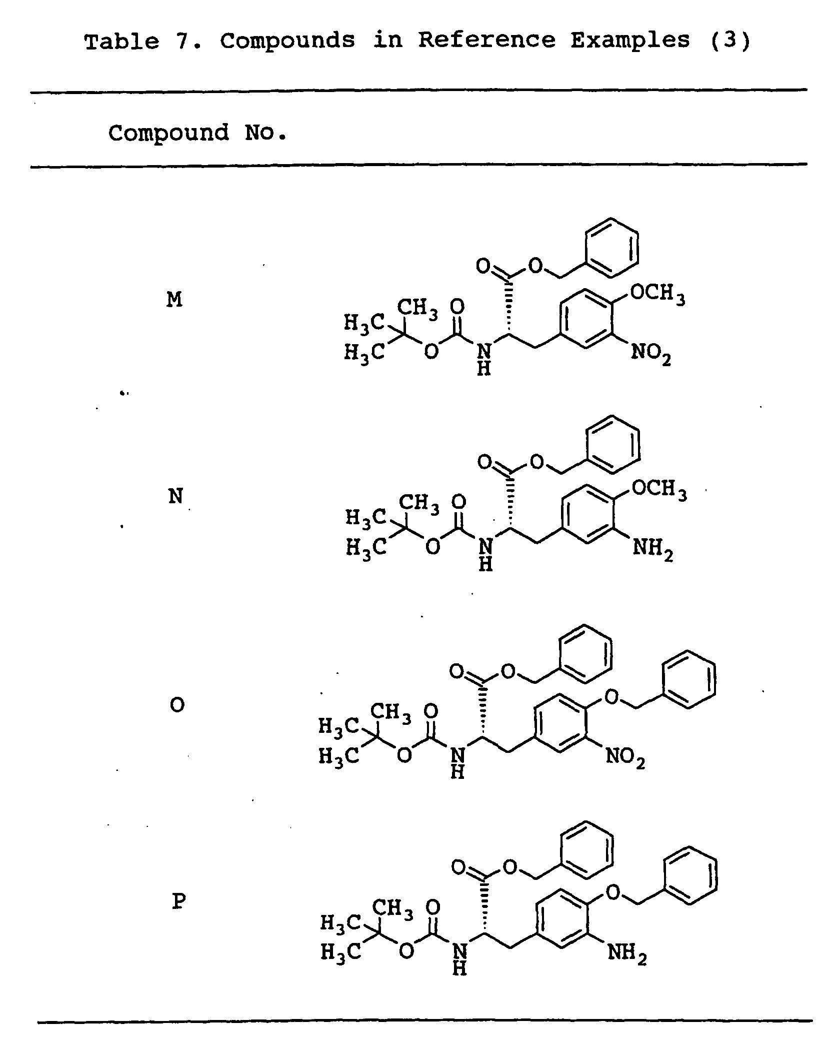

### Reference Example 1: Synthesis of Compound A

Diethyl (R)-(+)-malate (5.0 g, 0.026 mol) was dissolved in tetrahydrofuran (150 mL) followed by adding a 1 mol/L solution of lithium bis(trimethylsilyl)amide/tetrahydrofuran (53 mL, 0.053 mmol) and then the mixture was stirred at -78°C for 15 minutes. Further, 3-bromo-2-methylpropene (27 mL, 0.26 mol) was added thereto and the temperature was elevated up to 0°C over one hour. After usual post-treatment, purifying by a chromatography on silica gel (eluted with n-hexane/ethyl acetate=2/1) afforded Compound A (5.2 g, yield; 81%).
¹H NMR (CDCl₃, 300MHz)δ ppm: 4.84(d, J=11.7Hz, 2H), 4.21-4.33(m, 3H), 4.14 (q, J=7.1Hz, 2H), 3.18(d, J=7.2Hz, 1H), 3.11(ddd, J=3.0, 6.6, 9.2Hz, 1H), 2.58(dd, J=6.6, 14.5Hz, 1H), 2.44(dd, J=9.0, 14.3Hz, 1H), 1.78(s, 3H), 1.32 (t, J=7.1Hz, 3H), 1.24(t, J=7.1Hz, 3H)
FABMS m/z: 245(M+H)⁺ calculated for C₁₂H₂₀O₅=244

### Reference Example 2: Synthesis of Compound B

Compound A (0.50 g, 0.0020 mol) obtained in Reference Example 1 was dissolved in 1,4-dioxane (2.5 mL) and water (2.5 mL) followed by adding a 4.5 mol/L aqueous solution of potassium hydroxide (1.4 mL, 0.0061 mmol), and the mixture was stirred at 100°C for 6 hours. After DOWEX50W was added to the reaction mixture for neutralization, the solvent was distilled off under reduced pressure. Trifluoroacetic anhydride (1.9 mL) was added to the residue, followed by stirring at 25°C for 0.5 hour. After the solvent was distilled off under reduced pressure, the residue was dissolved in benzyl alcohol (5.4 mL) followed by adding triethylamine (1.4 mL, 0.010 mol) and 4-dimethylaminopyridine (0.25 g, 0.0020 mol), and then the mixture was stirred at 60°C for 0.5 hour. After usual post-treatment, purifying by a chromatography on silica gel (eluted with chloroform/methanol=20/1) afforded Compound B (0.56 g, yield; 100%).
¹H NMR (CDCl₃, 300MHz)δ ppm: 7.27-7.39(m, 5H), 5.18(br s, 2H), 4.82(brd, J=6.5Hz, 2H), 4.32(br s, 1H), 3.09(m, 1H), 2.43-2.62 (m, 2H), 1.72(br s, 3H)
FABMS m/z: 277(M-H)⁻ calculated for C₁₅H₁₈O₅=278

### Reference Example 3: Synthesis of Compound C

Compound B (0.53 g, 2.0 mmol) obtained in Reference Example 2 was dissolved in dichloromethane (110 mL) followed by adding bis(2-oxo-3-oxazolidinyl)phosphinic chloride (1.0 g, 4.0 mmol) and N,N-diisopropylethylamine (1.2 mL, 6.0 mmol), and then the mixture was stirred at 25°C for 2 hours. After usual post-treatment, purifying by a chromatography on silica gel (eluted with toluene/ethyl acetate=20/1) afforded Compound C (0.22 g, yield; 44%).
¹H NMR (CDCl₃, 300MHz ) δ ppm: 7.30-7.40(m, 5H), 5.18-5.32(m, 2H), 4.85(br s, 1H), 4.76(br s, 1H), 4.62(d, J=4.2Hz, 1H), 3.92(ddd, J=4.2, 5.9, 9.9Hz, 1H), 2.62(dd, J=5.9, 15.3Hz, 1H), 2.52(dd, J=9.7, 14.8Hz, 1H), 1.71(s, 3H)

### Reference Example 4: Synthesis of Compound D

Diethyl (S)-(-)-malate (10 g, 0.053 mol) was dissolved in tetrahydrofuran (30 mL) followed by adding a 1 mol/L solution of lithium bistrimethylsilylamide/tetrahydrofuran (110 mL, 0.11 mmol) and then the mixture was stirred at -78°C for 2.5 hours. Then 1-iodo-2-methylpropane (9.2 mL, 0.080 mol) was added thereto, and the mixture was stirred at 25°C for one hour. After usual post-treatment, purifying by a chromatography on silica gel (eluted with n-hexane/ethyl acetate=2/1) afforded Compound D (2.7 g, yield; 21%).
¹H NMR (CDCl₃, 300MHz)δ ppm: 4.31-4.12(m, 3H), 4.13(q, J=7.1Hz, 1H), 3.19 (br d, J=7.5Hz, 1H), 2.93(ddd, J=3.5, 7.6, 7.6Hz, 1H), 1.59-1.82(m, 2H), 1.52(dd, J=7.0, 13.4Hz, 1H), 1.31(t, J=7.1Hz, 3H), 1.25(t, J=6.9Hz, 3H), 0.95(d, J=6.7Hz, 3H), 0.94 (d, J=6.5Hz, 3H)
FABMS m/z: 247(M+H)⁺ calculated for C₁₂H₂₂O₅=246

### Reference Example 5: Synthesis of Compound E

In a manner similar to that in Reference Example 2, Compound E (1.2g, yield; 73%) was obtained from Compound D (2.7 g, 0.011 mol) obtained in Reference Example 4, 1,4-dioxane (130 mL), water (130 mL), a 4.5 mol/L aqueous solution of potassium hydroxide (7.3 mL, 0.033 mol), trifluoroacetic acid anhydride (62 mL), benzyl alcohol (88 mL), triethylamine (1.4 mL, 0.010 mol) and 4-dimethylaminopyridine (1.3 g, 0.011 mol).
¹H NMR (DMSO-d₆, 300MHz) δ ppm: 7.31-7.40(m, 5H), 5.16(d, J=12.2Hz, 1H), 5.11(d, J=12.3Hz, 1H), 4.13(d, J=7.3Hz, 1H), 2.67(m, 1H), 1.40-1.52(m, 2H), 1.03(dd, J=4.6, 9.2Hz, 1H), 0.80(d, J=6.4Hz, 3H), 0.79(d, J=6.4Hz, 3H)
FABMS m/z: 281(M+H)⁺ calculated for C₁₅H₂₀O₅=280

### Reference Example 6: Synthesis of Compound F

In amanner similar to that in Reference Example 3, Compound F (4.3 g, yield; 42%) was obtained from Compound E (11 mg, 0.039 mmol) obtained in Reference Example 5, dichloromethane (0.19 mL), bis(2-oxo-3-oxazolidinyl)phosphinic chloride (12 mg, 0.047 mmol) and triethylamine (0.016 mL, 0.12 mmol).
¹H NMR (CDCl₃, 300MHz)δ ppm: 7.34-7.40(m, 5H), 5.30(d, J=12.2Hz, 1H), 5.24(d, J=12.1Hz, 1H), 4.61(d, J=4.2Hz, 1H), 3.77(ddd, J=4.2, 6.3, 10.3Hz, 1H), 1.68-1.86(m, 3H), 0.93(d, J=6.4Hz, 3H), 0.88(d, J=6.2Hz, 3H)

### Reference Example 7: Synthesis of Compound G

UCK14C (0.060 g, 0.17 mmol) was dissolved in a 50% aqueous solution of tetrahydrofuran (1.5 mL) followed by adjusting the pH to 7.5 with a saturated aqueous solution of sodium hydrogencarbonate. Then, di-tert-butyl dicarbonate (0.040 g, 0.018 mmol) was added thereto and the mixture was stirred at room temperature for one hour. After usual post-treatment, purifying by a chromatography on silica gel (eluted with chloroform/methanol/acetic acid=200/1/0.1 to 200/4/0.4) afforded Compound G (0.052 g, yield; 68%).
¹H NMR (CDCl₃, 300MHz)δppm: 7.18(d, J=7.9Hz, 1H), 7.06(t, J=5.9Hz, 1H), 5.34(brs, 1H), 4.63(d, J=4.6Hz, 1H), 4.58(m, 1H), 4.23(br s, 1H), 3.62(dd, J=4.4, 7.7Hz, 1H), 3.21-3.48(m, 2H), 1.88-2.05(m, 2H), 1.54-1.82(m, 4H), 1.43(s, 9H), 1.35(d, J=7.0Hz, 3H), 1.29(m, 1H), 1.07(d, J=6.6Hz, 3H), 0.93(t, J=7.6Hz, 3H)
FABMS m/z: 458(M+H)⁺ calculated for C₂₁H₃₅N₃O₈=457

### Reference Example 8: Synthesis of Compound H

Compound A (0.50 g, 2.1mmol) obtained in Reference Example 1 was dissolved in ethanol (50 mL) followed by adding platinum oxide (0.050 g), and then the mixture was stirred at 25°C for 4 hours under hydrogen atmosphere. After passing the reaction mixture through Celite R545, the solvent was distilled off under reduced pressure to afford Compound H (0.45 g, yield; 90%).
¹H NMR (CDCl₃, 300MHz)δ ppm: 4.27 (q, J=7.2Hz, 1H), 4.26(q, J=7.2Hz, 1H), 4.14 (q, J=7.1Hz, 2H), 3.18(d, J=7.7Hz, 1H), 2.93(ddd, J=3.7, 7.0, 8.3Hz, 1H), 1.48-1.81 (m, 3H), 1.31 (t, J=7.1Hz, 3H), 1.24 (t, J=7.1Hz, 3H), 0.95(d, J=6.4Hz, 3H), 0.93(d, J=6.5Hz, 3H)

### Reference Example 9: Synthesis of Compound I

Compound H (0.45 g, 1.8 mmol) obtained in Reference Example 8 was dissolved in 1,4-dioxane (11 mL) and water (11 mL) followed by adding a 4.5 mol/L aqueous solution of potassium hydroxide (1.2 mL, 5.5 mmol), and then the mixture was stirred at 100°C for one hour. DOWEX50W was added to the reaction mixture for neutralization, and then the solvent was distilled off under reduced pressure. The residue (0.35 g, 1.8 mmol) was dissolved in dichloromethane (11 mL) followed by adding 2,2-dimethoxypropane (0.45 mL, 3.7 mmol) and camphorsulfonic acid (0.086 g, 0.37 mmol), and then the mixture was stirred at 40°C for 1.5 hours. After usual post-treatment, purifying by a chromatography on silica gel (eluted with n-hexane/ethyl acetate=1/1) afforded Compound I (0.33 g, yield; 77%).
¹H NMR (CDCl₃, 300MHz)δ ppm: 4.47(d, J=4.9H2, 1H), 3.02(m, 1H), 1.59-1.86(m, 3H), 1.61(s, 3H), 1.55(s, 3H), 0.96(d, J=6.2Hz, 3H), 0.95(d, J=6.0Hz, 3H)
FABMS m/z: 231(M+H)⁺ calculated for C₁₁H₁₈O₅=230

### Reference Example 10: Synthesis of Compound J

Compound I (45mg, 0.20 mmol) obtained in Reference Example 9 was dissolved in dichloromethane (4.5 mL) followed by adding ethanethiol (0.025 mL, 0.34 mmol), 1-ethyl-3-(3-dimethylaminopropyl)carbodiimide hydrochloride (64 mg, 0.34 mmol) and 4-dimethylaminopyridine (4.8 mg, 0.039 mmol), and then the mixture was stirred at 25°C for 1.5 hours. After usual post-treatment, purifying by a chromatography on silica gel (eluted with n-hexane/ethyl acetate=20/1) afforded Compound J (39 mg, yield; 73%).
¹H NMR (CDCl₃, 300MHz) δ ppm: 4.45(d, J=6.2Hz, 1H), 3.10(m, 1H), 2.83-3.01(m, 2H), 1.64-1.85(m, 3H), 1.61(s, 3H), 1.52(s, 3H), 1.26(t, J=7.3Hz, 3H), 0.97(d, J=6.8Hz, 3H), 0.63(d, J=6.6Hz, 3H)

### Reference Example 11: Synthesis of Compound K

Compound B (0.13 g, 0.46 mmol) obtained in Reference Example 2 was dissolved in methanol (6.4 mL) followed by adding a 2 mol/L solution of trimethylsilyldiazomethane/n-hexane (4.1 mL, 4.6 mmol), and then the mixture was stirred at 0°C for 10 minutes. After usual post-treatment, purifying by a chromatography on silica gel (eluted with n-hexane/ethyl acetate=3/1) afforded Compound K (62 mg, yield; 46%).
¹H NMR (CDCl₃, 300MHz)δ ppm: 7.33-7.39(m, 5H), 5.22(d, J=8.1Hz, 2H), 4.84(s, 1H), 4.80(s, 1H), 4.68(s, 1H), 4.30(d, J=3.1Hz, 1H), 3.54(s, 3H), 3.13(ddd, J=3.1, 6.6, 9.2Hz, 1H), 2.54(dd, J=6.8, 14.5Hz, 1H), 2.42(dd, J=5.5, 14.3Hz, 1H), 1.73(s, 3H)

### Reference Example 12: Synthesis of Compound L

Compound K (62mg, 0.21 mmol) obtained in Reference Example 11 was dissolved in tetrahydrofuran (3.1 mL) followed by adding sodium hydride (7.1 mg, 0.30 mmol) and bromomethyl methyl ether (0.052 mL, 0.63 mmol), and then the mixture was stirred at 0°C for 10 minutes. After usual post-treatment, purifying by a chromatography on silica gel (eluted with n-hexane/ethyl acetate=5/1) afforded Compound L (57 mg, yield; 80%).
¹H NMR(CDCl₃, 300MHz) δ ppm: 7.33-7.40(m, 5H), 5.20(s, 2H), 4.78(s, 1H), 4.71(s, 1H), 4.67(dd, J=7.0, 9.0Hz, 2H), 4.30(d, J=6.6Hz, 1H), 3.60(s, 3H), 3.32(s, 3H), 3.17(dt, J=6.5, 8.8Hz, 1H), 2.41(dd, J=8.9, 14.4Hz, 1H), 2.13(dd, J=6.3, 14.4Hz, 1H), 1.68(s, 3H)
FABMS m/z: 337(M+H)⁺ calculated for C₁₈H₂₄O₆=336

### Reference Example 13: Synthesis of Compound M

N-(tert-butyloxycarbonyl)-3-nitro-L-tyrosine benzyl ester (10 mg, 0.024 mmol) was dissolved inN,N-dimethylformamide (1.0mL) followed by adding methyl iodide (0.0075 mL, 0.12 mmol) and potassium carbonate (6.7 mg, 0.28 mmol), and then the mixture was stirred at 25°C for one hour. After usual post-treatment, purifying by a thin layer column chromatography (developed with n-hexane/ethyl acetate=2/1) afforded Compound M (11 mg, yield; 100%).
¹H NMR (CDCl₃, 300MHz) δ ppm: 7.55(d, J=2.2Hz, 1H), 7.30-7.40(m, 5H), 7.19(dd, J=2.3, 8.9Hz, 1H), 6.91(d, J=8.6Hz, 1H), 5.20(d, J=12.1Hz, 1H), 5.11(d, J=11.9Hz, 1H), 5.06(br d J=7.3Hz, 1H), 4.60(m, 1H), 3.91(s, 3H), 3.13(dd, J=6.6, 14.1Hz, 1H), 3.01(dd, J=6.1, 14.2Hz, 1H), 1.42(s, 9H)
FABMS m/z: 431(M+H)⁺ calculated for C₂₂H₂₆N₂O₇=430

### Reference Example 14: Synthesis of Compound N

Compound L (110 mg, 0.025 mmol) obtained in Reference Example 13 was dissolved in 2-methoxyethanol (1.6 mL) and water (1.6 mL) followed by adding sodium dithionite (13 mg, 0.74 mmol), and then the mixture was stirred at 80°C for 7 hours. After usual post-treatment, purifying by a thin layer column chromatography (developed with chloroform/methanol=20/1) afforded Compound N (21 mg, yield; 21%).
¹H NMR (CDCl₃, 400MHz) δ ppm: 7.30-7.38(m, 5H), 6.62(d, J=8.1Hz, 1H), 6.39(br d, J=8.1Hz, 1H), 6.32(brs, 1H), 5.20(d, J=12.3Hz, 1H), 5.08(d, J=12.2Hz, 1H), 4.94(br d, J=7.5Hz, 1H), 4.55(m, 1H), 3.80(s, 3H), 3.65(br s, 2H), 2.94(m, 2H), 1.42(s, 9H)

### Reference Example 15: Synthesis of Compound O

N-(tert-butyloxycarbonyl)-3-nitro-L-tyrosine (0.21 g, 0.63 mmol) was dissolved in N,N-dimethylformamide (1.0 mL) followed by adding benzyl bromide (0.34 mL, 2.8 mmol) and potassium carbonate (0.18 g, 1.3 mmol), and then the mixture was stirred at 25°C for 0.5 hour. After usual post-treatment, purifying by a chromatography on silica gel (eluted with n-hexane/ethyl acetate=3/1) afforded Compound O (0.21 mg, yield; 65%).
¹H NMR (CDCl₃, 300MHz)δ ppm: 7.56(br d, J=1.7Hz, 1H), 7.27-7.46 (m, 10H), 7.13(br d, J=7.4Hz, 1H), 6.92(d, J=8.7Hz, 1H), 5.05-5.22(m, 5H), 4.59(m, 1H), 3.11(dd, J=6.0, 13.9Hz, 1H), 2.99(dd, J=5.7, 14.0Hz, 1H), 1.41(s, 9H)
FABMS m/z: 507(M+H)⁺ calculated for C₂₈H₃₀N₂O₇=506

### Reference Example 16: Synthesis of Compound P

In a manner similar to that in Reference Example 14, Compound P (30 mg, yield; 18%) was obtained from Compound O (0.18 g, 0.35 mmol) obtained in Reference Example 15, 2-methoxyethanol (4.4 mL), water (4.4 mL) and sodium dithionite (0.18 g, 1.1 mmol).
¹H NMR (CDCl₃, 400MHz) δ ppm: 7.28-7.48(m, 10H), 6.69(d, J=8.3Hz, 1H), 6.37(br d, J=8.3Hz, 1H), 6.33(br s, 1H), 4.91-5.25(m, 5H), 4.56(m, 1H), 3.70(br s, 2H), 2.89-2.99(m, 2H), 1.40(s, 9H)
FABMS m/z: 476 M⁺ calculated for C₂₈H₃₂N₂O₅=476

### Industrial Applicability

The present invention provides proteasome inhibitors effective for the treatment of malignant tumors, such as leukemia, lung cancer, colon cancer, breast cancer or the like by growth suppression of various cancer cells, for the treatment of diseases associated with autoimmune diseases, inflammation, neurodegeneration or the like, such as rheumatoid chronic arthritis, asthma, Alzheimer disease or the like, or further for the reduction of rejection in organ transplantation.

## Claims

1. A proteasome inhibitor comprising, as an active ingredient, a carboxylic acid derivative represented by the formula (I) or a pharmaceutically acceptable salt thereof: 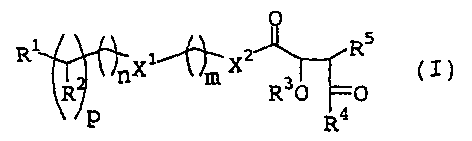 <wherein m and n are the same or different and represent an integer of 0 to 10; p represents 0 or 1; R¹ represents a hydrogen atom, substituted or unsubstituted alkyl, substituted or unsubstituted alicyclic alkyl, substituted or unsubstituted aralkyl, substituted or unsubstituted aryl or NR⁶R⁷ {wherein R⁶ represents a hydrogen atom, substituted or unsubstituted alkyl, or substituted or unsubstituted aralkyl, and R⁷ represents a hydrogen atom, substituted or unsubstituted alkyl, substituted or unsubstituted aralkyl, CW¹R⁸ (wherein R⁸ represents a hydrogen atom, substituted or unsubstituted alkyl, substituted or unsubstituted alkylamino, substituted or unsubstituted alkoxy, substituted or unsubstituted aryl, a substituted or unsubstituted heterocyclic group, substituted or unsubstituted aralkyl, substituted or unsubstituted aralkylamino, or substituted or unsubstituted aralkyloxy, and W¹ represents an oxygen atom or a sulfur atom), or the formula: 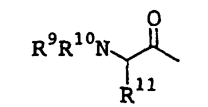 (wherein R⁹ represents a hydrogen atom, substituted or unsubstituted alkyl, or substituted or unsubstituted aralkyl; R¹⁰ represents a hydrogen atom, substituted or unsubstituted alkyl, substituted or unsubstituted aralkyl, CW²R^{8a} (wherein R^{8a} and W² have the same significances as the above R⁸ and W¹, respectively), substituted or unsubstituted alkylsulfonyl, substituted or unsubstituted arylsulfonyl, or PW³R¹²₂ (wherein R¹²'s are the same or different and represent substituted or unsubstituted alkyl, or substituted or unsubstituted aryl; and W³ has the same significance as the above W¹); or R⁹ and R¹⁰ together represent the formula: 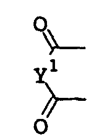 (wherein Y¹ represents substituted or unsubstituted alkylene or substituted or unsubstituted arylene); and R¹¹ represents a hydrogen atom, substituted or unsubstituted alkyl, or substituted or unsubstituted aralkyl)}; R² represents a hydrogen atom, COR¹³ (wherein R¹³ represents hydroxy, substituted or unsubstituted alkoxy, substituted or unsubstituted alkenyloxy, substituted or unsubstituted aralkyloxy, substituted or unsubstituted alicyclic alkylalkoxy, substituted or unsubstituted aroylalkoxy, or NR¹⁴R¹⁵ (wherein R¹⁴ represents a hydrogen atom, substituted or unsubstituted alkyl, or substituted or unsubstituted aryl; and R¹⁵ represents substituted or unsubstituted alkyl, substituted or unsubstituted aralkyl, substituted or unsubstituted alkoxycarbonylalkyl, amino, substituted or unsubstituted alkylamino, or substituted or unsubstituted arylamino; or R¹⁴ and R¹⁵ together with the adjacent N form a substituted or unsubstituted heterocyclic group)) or CH₂OR^{3a} (wherein R^{3a} represents a hydrogen atom, substituted or unsubstituted alkyl, substituted or unsubstituted aralkyl, substituted or unsubstituted alkanoyl, substituted or unsubstituted aroyl, or SiR¹⁶₃ (wherein R¹⁶'s are the same or different and represent substituted or unsubstituted alkyl, or substituted or unsubstituted aryl)); or R¹ and R² together represent the formula: 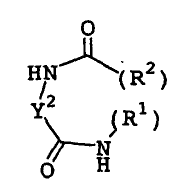 (wherein Y² represents substituted or unsubstituted alkylene); X¹ represents a bond, substituted or unsubstituted alkylene, substituted or unsubstituted alicyclic alkylene, substituted or unsubstituted alkenylene, or substituted or unsubstituted arylene; X² represents an oxygen atom, a sulfur atom or NR¹⁷ (wherein R¹⁷ represents a hydrogen atom, substituted or unsubstituted alkyl, or substituted or unsubstituted aralkyl); R³ has the same significance as the above R^{3a}; R⁴ represents hydroxy, mercapto, substituted or unsubstituted alkoxy, or substituted or unsubstituted alkylthio; or R³ and R⁴ together represent a bond; and R⁵ represents a hydrogen atom, substituted or unsubstituted alkyl, substituted or unsubstituted alkenyl, or substituted or unsubstituted aralkyl>.

2. The proteasome inhibitor according to claim 1, wherein R³ and R⁴ together represent a bond.

3. The carboxylic acid derivative or the pharmaceutically acceptable salt thereof according to claim 1, wherein R⁴ is hydroxy, or substituted or unsubstituted alkoxy; p is 1; R¹ is a hydrogen atom or NR⁶R⁷ (wherein each of R⁶ and R⁷ has the same significance as defined above), or R¹ and R² together are the formula: 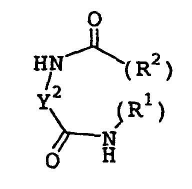 (wherein Y² has the same significance as defined above); X¹ is substituted or unsubstituted alicyclic alkylene, or substituted or unsubstituted arylene; and X² is NR¹⁷ (wherein R¹⁷ has the same significance as defined above).

4. The carboxylic acid derivative or the pharmaceutically acceptable salt thereof according to claim 1, wherein R⁴ is mercapto, or substituted or unsubstituted alkylthio, or R³ and R⁴ together are a bond; X² is NR¹⁷ (wherein R¹⁷ has the same significance as defined above)[when m is 0; n and p are 1; R² is carboxy; R³ and R⁴ together are a bond; R⁵ is sec-butyl; and X¹ is cyclopropylene or ethylene, R¹ is neither NHC(=O)-C(CH₃)NH₂ nor NHC(=O)-C(CH₃)NHC(=O)O-C(CH₃)₃].

5. The carboxylic acid derivative or the pharmaceutically acceptable salt thereof according to claim 3, wherein R¹ is a hydrogen atom or NR⁶R⁷ (wherein each of R⁶ and R⁷ has the same significance as defined above).

6. The carboxylic acid derivative or the pharmaceutically acceptable salt thereof according to claim 5, wherein R¹ is NR⁶R⁷ (wherein each of R⁶ and R⁷ has the same significance as defined above); X¹ is cyclopropylene or alkylene; and X² is NH.

7. The carboxylic acid derivative or the pharmaceutically acceptable salt thereof according to claim 4, wherein R⁴ is mercapto, or substituted or unsubstituted alkylthio; R¹ is NR⁶R⁷ (wherein each of R⁶ and R⁷ has the same significance as defined above); and X¹ is cyclopropylene or alkylene.

8. The carboxylic acid derivative or the pharmaceutically acceptable salt thereof according to claim 4, wherein R³ and R⁴ together are a bond.

9. The carboxylic acid derivative or the pharmaceutically acceptable salt thereof according to claim 8, wherein m is 0; n and p are 1; R¹ is NR⁶R⁷ (wherein each of R⁶ and R⁷ has the same significance as defined above); R² is COR^{13a} (wherein R^{13a} is alkylamino, aralkyloxy or aralkylamino); R⁵ is alkyl; X¹ is cyclopropylene, alkylene, or substituted or unsubstituted phenylene; and X² is NH.

10. A process for producing the carboxylic acid derivative according to claim 1, wherein R³ and R⁴ together represent a bond and X² is NR¹⁷, **characterized in that** a carboxylic acid represented by the formula (II): 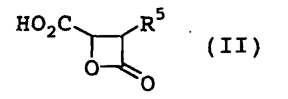 (wherein R⁵ has the same significance as defined above) is reacted with an amine represented by the formula (III): 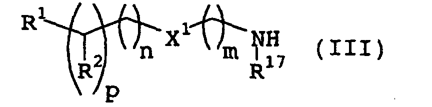 (wherein each of m, n, p, R¹, R², R¹⁷ and X¹ has the same significance as defined above).

11. The carboxylic acid according to claim 10, wherein R⁵ is substituted or unsubstituted alkyl, substituted or unsubstituted alkenyl, or substituted or unsubstituted aralkyl, or a salt thereof.

12. The amine according to claim 10, wherein m is 0; n and p are 1; R¹ is NR⁶R⁷ (wherein each of R⁶ and R⁷ has the same significance as defined above); R² is COR¹³ (wherein R¹³ has the same significance as defined above) or CH₂OR^{3a} (wherein R^{3a} has the same significance as defined above), or R¹ and R² together are the formula: 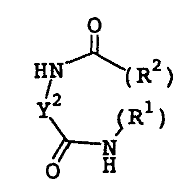 (wherein Y² has the same significance as defined above); and X¹ is cyclopropylene, or a salt thereof.

13. The amine or the salt thereof according to claim 12, wherein R¹ is amino and R¹⁷ is a hydrogen atom.

14. The amine or the salt thereof according to claim 13, wherein R² is carboxy.

15. A pharmaceutical composition comprising the amine or the salt thereof according to any one of claims 12 to 14 as an active ingredient.

16. A compound wherein the amine according to any one of claims 12 to 14 is protected with a protecting group, or a salt thereof.

17. A pharmaceutical composition comprising the carboxylic acid derivative or the pharmaceutically acceptable salt thereof according to any one of claims 3 to 9 as an active ingredient.

18. A proteasome inhibitor comprising the carboxylic acid derivative or the pharmaceutically acceptable salt thereof according to any one of claims 3 to 9 as an active ingredient.

19. An antitumor agent comprising the carboxylic acid derivative or the pharmaceutically acceptable salt thereof according to any one of claims 3 to 9 as an active ingredient.

20. A pharmaceutical composition comprising the carboxylic acid derivative or the pharmaceutically acceptable salt thereof according to any one of claims 3 to 9 as an active ingredient, used for the treatment of the diseases curable by proteasome inhibition.

21. A use of the carboxylic acid derivative or the pharmaceutically acceptable salt thereof according to any one of claims 3 to 9 for the manufacture of a proteasome inhibitor.

22. A use of the carboxylic acid derivative or the pharmaceutically acceptable salt thereof according to any one of claims 3 to 9 for the manufacture of an antitumor agent.

23. Amethod to inhibit proteasome comprising a process in which an effective amount of the carboxylic acid derivative or the pharmaceutically acceptable salt thereof according to any one of claims 3 to 9 is administered to a mammal including human.

24. A method of treatment or prevention of a tumor comprising a process in which an effective amount of the carboxylic acid derivative or the pharmaceutically acceptable salt thereof according to any one of claims 3 to 9 is administered to a mammal including human.
